(19) Europäisches Patentamt
European Patent Office
Office européen des brevets

(11) Veröffentlichungsnummer: **0 365 915 B1**

(12) **EUROPÄISCHE PATENTSCHRIFT**

(45) Veröffentlichungstag der Patentschrift: **20.04.94**

(51) Int. Cl.[5]: **C07D 417/12**, A61K 31/505

(21) Anmeldenummer: **89118839.3**

(22) Anmeldetag: **11.10.89**

# (54) Substituierte 2-Aminothiazole.

(30) Priorität: **24.10.88 DE 3836167**
**25.11.88 DE 3839758**

(43) Veröffentlichungstag der Anmeldung:
**02.05.90 Patentblatt 90/18**

(45) Bekanntmachung des Hinweises auf die Patenterteilung:
**20.04.94 Patentblatt 94/16**

(84) Benannte Vertragsstaaten:
**AT BE CH DE ES FR GB GR IT LI LU NL SE**

(56) Entgegenhaltungen:
**EP-A- 0 095 640**
**DE-A- 1 695 910**

(73) Patentinhaber: **BAYER AG**

**D-51368 Leverkusen(DE)**

(72) Erfinder: **Ippen, Joachim, Dr.**
**Völklinger Strasse 12**
**D-5090 Leverkusen 1(DE)**
Erfinder: **Baasner, Bernd, Dr.**
**Hamberger Strasse 27d**
**D-5090 Leverkusen 3(DE)**
Erfinder: **Marhold, Albrecht, Dr.**
**Carl-Duisberg-Strasse 329**
**D-5090 Leverkusen 1(DE)**
Erfinder: **Kysela, Ernst, Dr.**
**Virchowstrasse 14**
**D-5060 Bergisch-Gladbach 1(DE)**
Erfinder: **Schaller, Klaus, Dr.**
**Am Sonnenschein 38**
**D-5600 Wuppertal 1(DE)**
Erfinder: **von Bittera, Miklos**
**Max-Scheler-Strasse 7**
**D-5090 Leverkusen 1(DE)**

EP 0 365 915 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte 2-Aminothiazole, ein Verfahren zu ihrer Herstellung und ihre Verwendung bei der Bekämpfung von Krankheiten, insbesondere Mykosen.

Es ist bekannt, daß bestimmte substituierte Aminothiazole oder deren Säureadditionssalze, wie beispielsweise die Verbindung 4-(2,4-Dichlorphenyl)-2-[2-(1,4,5,6-tetrahydropyrimidinyl)-amino]-thiazol Hydrochlorid oder die Verbindung 4-(4-Chlor-3-methylphenyl)-2-[2-(1,4,5,6-tetrahydropyrimidinyl)-amino]-thiazol Hydrochlorid oder die Verbindung 4-(4-Chlor-2-methoxyphenyl)-2-[2-(1,4,5,6-tetrahydropyrimidinyl)-amino]-thiazol gute antimykotische Eigenschaften besitzen (vgl. z.B. DE-OS 32 20 118).

Die Wirksamkeit dieser vorbekannten Verbindungen ist jedoch nicht in allen Indikationen völlig zufriedenstellend.

Es wurden neue 2-Aminothiazole der allgemeinen Formel (I),

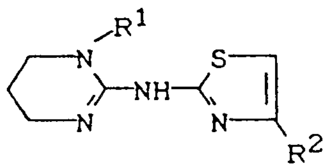

(I)

in welcher

$R^1$ für Wasserstoff oder für geradkettiges oder verzweigtes Alkyl mit 1 bis 4 Kohlenstoffatomen steht und

$R^2$ für einen Rest der Formel

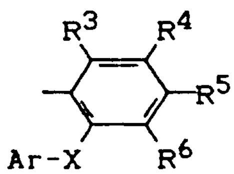 ; 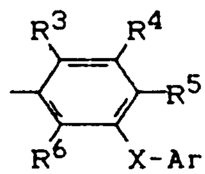 oder 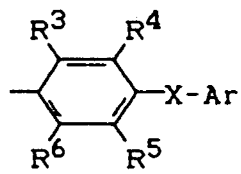

steht,

wobei

$R^3$, $R^4$, $R^5$ und $R^6$ unabhängig voneinander jeweils für Wasserstoff, Fluor, Chlor, Brom, Iod, Nitro, für jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 4 Kohlenstoffatomen in den jeweiligen Alkylteilen oder für jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen -insbesondere Fluor, Chlor, Brom oder Iod- stehen,

X für Sauerstoff, Schwefel, Sulfinyl oder Sulfonyl steht und

Ar für jeweils gegebenenfalls einfach oder mehrfach, gleich oder verschieden substituiertes Phenyl, α-Naphthyl, β-Naphthyl, Tetrahydronaphthyl oder Indanyl steht, wobei als Substituenten jeweils infrage kommen: Fluor, Chlor, Brom, Iod, jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 8 Kohlenstoffatomen in den jeweiligen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, Cycloalkyl mit 3 bis 7 Kohlenstoffatomen, Phenylalkyl oder Phenoxyalkyl mit jeweils 1 bis 4 Kohlenstoffatomen im geradkettigen oder verzweigten Alkylteil sowie Phenyl oder Phenoxy,

sowie deren physiologisch verträgliche Säureadditionssalze gefunden.

Die Verbindungen der Formel (I) stehen im Gleichgewicht mit den tautomeren Verbindungen der Formeln (Ia) und (Ib),

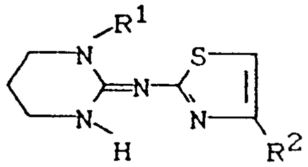

(Ia)

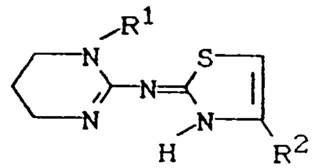

(Ib)

(wobei $R^1$ und $R^2$ jeweils die oben angegebene Bedeutung haben,) welche ebenfalls erfindungsgemäß beansprucht werden.

Weiterhin wurde gefunden, daß man die neuen substituierten 2-Aminothiazole der allgemeinen Formel (I),

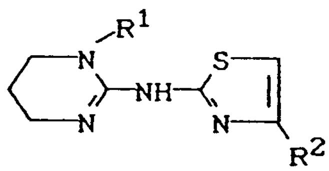

(I)

sowie deren physiologisch verträgliche Säureadditionssalze erhält, wenn man Thioharnstoff-Derivate der Formel (II),

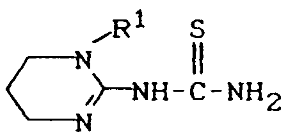

(II)

in welcher

$R^1$ die oben angegebene Bedeutung hat,

mit Acetophenon-Derivaten der Formel (III),

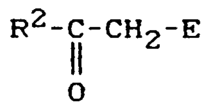

(III)

in welcher

$R^2$ die oben angegebene Bedeutung hat und

E für Hydroxy oder Halogen steht,

gegebenenfalls in Gegenwart eines Verdünnungsmittels und gegebenenfalls in Gegenwart eines Reaktionshilfsmittels umsetzt und gegebenenfalls anschließend eine Säure addiert.

Schließlich wurde gefunden, daß die neuen substituierten 2-Aminothiazole der allgemeinen Formel (I) gute antimikrobielle, insbesondere gute antimykotische Eigenschaften besitzen.

Überraschenderweise zeigen die erfindungsgemäßen substituierten 2-Aminothiazole der allgemeinen Formel (I) in bestimmten Indikationen eine erheblich bessere antimykotische Wirksamkeit als die aus dem Stand der Technik bekannten substituierten Aminothiazole sowie deren Säureadditionssalze, wie beispielsweise die Verbindung 4-(2,4-Dichlorphenyl)-2-[2-(1,4,5,6-tetrahydropyrimidinyl)-amino]-thiazol Hydrochlorid oder die Verbindung 4-(4-Chlor-2-methylphenyl)-2-[2-(1,4,5,6-tetrahydropyrimidinyl)-amino]-thiazol Hydrochlorid oder die Verbindung 4-(4-Chlor-2-methoxyphenyl)-2-[2-(1,4,5,6-tetrahydropyrimidinyl)-amino]-thiazol, welche chemisch und wirkungsmäßig naheliegende Verbindungen sind.

Die erfindungsgemäßen substituierten 2-Aminothiazole sind durch die Formel (I) allgemein definiert. Bevorzugt sind Verbindungen der Formel (I), bei welchen

$R^1$ für Wasserstoff, Methyl oder Ethyl steht,

$R^2$ für einen Rest der Formel

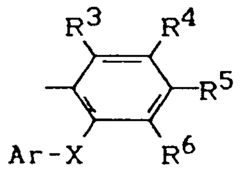 ; 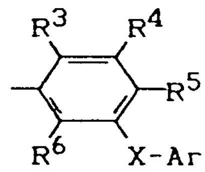 oder 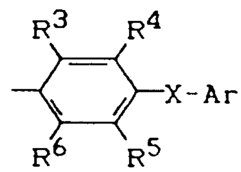

steht,
wobei

R$^3$, R$^4$, R$^5$ und R$^6$ unabhängig voneinander jeweils für Wasserstoff, Fluor, Chlor, Brom, Nitro, Methyl, Ethyl, Methoxy, Ethoxy, Methoxycarbonyl, Ethoxycarbonyl, Dimethylamino, Diethylamino, Methylthio, Ethylthio, Methylsulfinyl, Ethylsulfinyl, Methylsulfonyl oder Ethylsulfonyl oder für Halogenmethyl, Halogenethyl, Halogenmethoxy, Halogenethoxy, Halogenmethylthio, Halogenethylthio, Halogenmethylsulfinyl, Halogenethylsulfinyl, Halogenmethylsulfonyl oder Halogenethylsulfonyl mit jeweils 1 bis 5 gleichen oder verschiedenen Halogenatomen, insbesondere Fluor, Chlor oder Brom stehen,

X für Sauerstoff, Schwefel, Sulfinyl oder Sulfonyl steht und

Ar für jeweils gegebenenfalls ein- bis fünffach gleich oder verschieden substituiertes Phenyl, α-Naphthyl, β-Naphthyl, Tetrahydronaphthyl oder Indanyl steht, wobei als Substituenten jeweils infrage kommen: Fluor, Chlor, Brom, Nitro, jeweils geradkettiges oder verzweigtes Alkyl, Alkoxy, Alkoxycarbonyl oder Dialkylamino mit jeweils 1 bis 6 Kohlenstoffatomen in den jeweiligen Alkylteilen, jeweils geradkettiges oder verzweigtes Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, insbesondere Fluor, Chlor oder Brom, Cyclohexyl mit 3 bis 6 Kohlenstoffatomen, Phenylalkyl oder Phenoxyalkyl mit jeweils 1 bis 3 Kohlenstoffatomen im geradkettigen oder verzweigten Alkylteil sowie Phenyl oder Phenoxy.

Besonders bevorzugt sind Verbindungen der Formel (I), bei welchen

R$^1$ für Wasserstoff oder Methyl steht und

R$^2$ für einen Rest

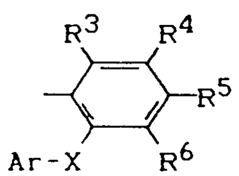 ; 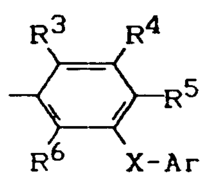 oder 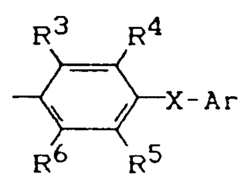

steht,
wobei

R$^3$, R$^4$, R$^5$ und R$^6$ unabhängig voneinander jeweils für Wasserstoff, Fluor, Chlor, Nitro, Methyl, Methoxy, Ethoxycarbonyl, Methoxycarbonyl, Dimethylamino, Methylthio, Trifluormethyl, Trifluormethoxy, Trifluormethylthio, Trifluormethylsulfinyl oder Trifluormethylsulfonyl stehen,

X für Sauerstoff oder Schwefel stehen und

Ar für jeweils gegebenenfalls ein- bis vierfach, gleich oder verschieden substituiertes Phenyl, α-Naphthyl, β-Naphthyl, Tetrahydronaphthyl oder Indanyl steht, wobei als Substituenten jeweils infrage kommen: Fluor, Chlor, Brom, Nitro, Methyl, Ethyl, n- oder i-Propyl, n-, i-, s- oder t-Butyl, Methoxy, Ethoxy, n- oder i-Propoxy, Methoxycarbonyl, Ethoxycarbonyl, Dimethylamino, Diethylamino, Methylthio, Ethylthio, Methylsulfinyl, Methylsulfonyl, Trifluormethyl, Trifluormethoxy, Trifluormethylthio, Trifluormethylsulfinyl, Trifluormethylsulfonyl, Cyclopentyl, Cyclohexyl, Benzyl, Phenylethyl, Phenylpropyl, Phenoxymethyl, Phenyl oder Phenoxy.

Besonders bevorzugte erfindungsgemäße Verbindungen sind auch Additionsprodukte aus Säuren und denjenigen substituierten 2-Aminothiazolen der Formel (I), in denen die Substituenten $R^1$ und $R^2$ die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Oxalsäure, Maleinsäure, Malonsäure, Bernsteinsäure, Fumarsäure, Glutarsäure, Hydroxyglutarsäure, Adipinsäure, Oleinsäure, Weinsäure, Äpfelsäure, Zitronensäure, Benzoesäure, Salicylsäure, Sorbinsäure und Milchsäure, Sulfonsäuren, wie z.B. Methansulfonsäure, p-Chlorbenzolsulfonsäure, p-Toluolsulfonsäure und 1,5-Naphthalindisulfonsäure, Schwefelsäurehalbester wie Schwefelsäuremonomethylester oder Schwefelsäuremonoethylester sowie Saccharin oder Thiosaccharin.

Insbesondere bevorzugt sind Verbindungen der Formel (I), bei welchen

$R^1$ für Wasserstoff steht und

$R^2$ für einen Rest der Formel

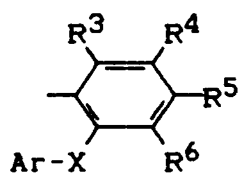

steht, wobei

$R^3$, $R^4$, $R^5$ und $R^6$ unabhängig voneinander jeweils für Wasserstoff, Fluor, Chlor, Methyl oder Nitro stehen,

X für Sauerstoff steht und

Ar für gegebenenfalls ein- bis dreifach, gleich oder verschieden substituiertes Phenyl steht, wobei als Substituenten insbesondere infrage kommen: Fluor, Chlor, Nitro, Methyl, Ethyl, n- oder i-Propyl, n-, i-, s- oder t-Butyl, Methoxy, Methylthio, Trifluormethyl, Trifluormethoxy, Trifluormethylthio, Trifluormethylsulfinyl, Trifluormethylsulfonyl, Cyclopentyl, Cyclohexyl, Phenyl oder Phenoxy.

Insbesondere bevorzugt sind daneben auch Verbindungen der Formel (I), bei welchen

$R^1$ für Wasserstoff steht und

$R^2$ für einen Rest der Formel

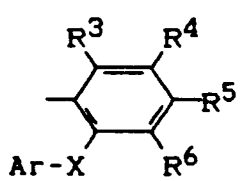

steht, wobei

$R^3$, $R^4$, $R^5$ und $R^6$ unabhängig voneinander jeweils für Wasserstoff, Fluor, Chlor, Methyl oder Nitro stehen,

X für Sauerstoff steht und

Ar für mindestens einfach durch Trifluormethylthio substituiertes Phenyl steht, wobei ein bis drei gleiche oder verschieden weitere Substituenten vorhanden sein können und wobei als weitere Substituenten infrage kommen: Fluor, Chlor, Methyl, Ethyl, n- oder i-Propyl, n-, i-, s- oder t-Butyl, Methoxy, Trifluormethyl, Trifluormethoxy und Trifluormethylthio.

Verwendet man beispielsweise N-(1,4,5,6-Tetrahydro-2-pyrimidinyl)-thioharnstoff und 2-Bromacetyl-4'-trifluormethylthio-diphenylether als Ausgangsstoffe, so läßt sich der Reaktionsablauf des erfindungsgemäßen Verfahrens durch das folgende Formelschema darstellen:

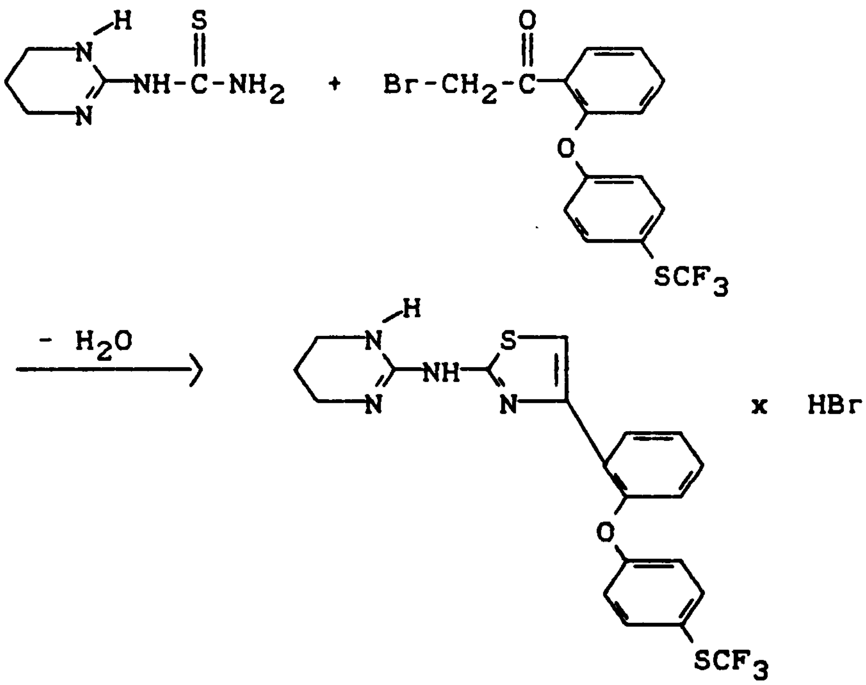


Die zur Durchführung des erfindungsgemäßen Verfahrens als Ausgangsstoffe benötigten Thioharnstoff-Derivate sind durch die Formel (II) allgemein definiert. In dieser Formel (II) steht $R^1$ vorzugsweise für diejenigen Reste, die bereits im Zusammenhang mit der Beschreibung der erfindungsgemäßen Stoffe der Formel (I) als bevorzugt für diesen Substituenten genannt werden.

Die Thioharnstoff-Derivate der Formel (II) sind bekannt (vgl. z.B. Arzneim.-Forsch. 35, 573-577 [1985] oder DE-OS 32 20 118 bzw. EP 95 640) oder erhältlich in Analogie zu bekannten Verfahren (vgl. z.B. Organic Syntheses, Coll. Vol. IV, 502), beispielsweise wenn man Tetrahydropyrimidinylcyanamide der Formel (XI),

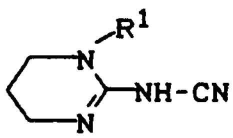 (XI)

in welcher

$R^1$ die oben angegebene Bedeutung hat,

mit Schwefelwasserstoff gegebenenfalls in Gegenwart eines Verdünnungsmittels wie beispielsweise Wasser und gegebenenfalls in Gegenwart eines Reaktionshilfsmittels wie beispielsweise Natriumhydroxid bei Temperaturen zwischen 20 °C und 120 °C umsetzt.

Tetrahydropyrimidinylcyanamide der Formel (XI) sind bekannt (vgl. z.B. DE-OS 22 05 745; DE-OS 22 05 744; J. Org. Chem. 38, 155-156 [1973]).

Die zur Durchführung des erfindungsgemäßen Verfahrens weiterhin als Ausgangsstoffe benötigten Acetophenon-Derivate sind durch die Formel (III) allgemein definiert. In dieser Formel (III) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden.

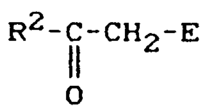 (III)

E steht vorzugsweise für Hydroxy, Chlor oder Brom,

Acetophenon-Derivate der Formel (III) sind teilweise bekannt (vgl. z.B. Chem. Pharm. Bull. 32, 3066-3074 [1984]; DE-OS 35 29 646; JP 62/19 566; J. Chem. Soc. Perkin I, 1983, 1483-1488; J. Med. Chem. 26, 1353-1360 [1983]; J. Chem. Soc. Perkin I, 1978, 446-451; Isr. J. Chem. 1974, 12, 977-979).

Acetophenon-Derivate der Formel (IIIa),

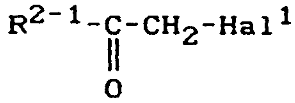

(IIIa)

in welcher

Hal[1] für Chlor oder Brom steht und

$R^{2-1}$ für einen Rest der Formel

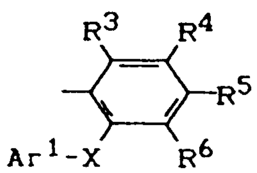

;

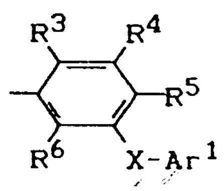

oder

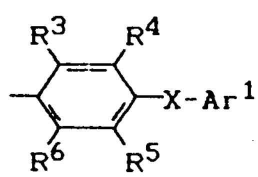

steht,

wobei

$R^3$, $R^4$, $R^5$, $R^6$ und X jeweils die oben angegebene Bedeutung haben und

$Ar^1$ für einen gegebenenfalls substituierten Arylrest steht, wobei mindestens ein Substituent für Fluor, Halogenalkyl, Halogenalkoxy, Halogenalkylthio, Halogenalkylsulfinyl oder Halogenalkylsulfonyl steht und wobei für $Ar^1$ die Bedeutung 2-Chlor-4-trifluormethylphenyl ausgenommen ist,

sind teilweise neu.

Man erhält bekannte und nicht bekannte Acetophenon-Derivate der Formel (III) in Analogie zu bekannten Verfahren, beispielsweise wenn man Acetophenone der Formel (IV),

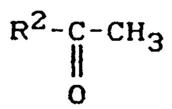

(IV)

in welcher

$R^2$ die oben angegebene Bedeutung hat,

mit Halogenierungsmitteln wie beispielsweise Sulfurylchlorid oder Brom gegebenenfalls in Gegenwart eines Verdünnungsmittels wie beispielsweise Dichlormethan oder Eisessig und gegebenenfalls in Gegenwart eines Reaktionshilfsmittels wie beispielsweise Salzsäure bei Temperaturen zwischen - 20 °C und + 80 °C umsetzt oder wenn man Diaryl(thio)ether-Derivate der Formel (V),

$R^2$-H (V)

in welcher

$R^2$ die oben angegebene Bedeutung hat,

mit Chloracetylchlorid oder Bromacetylbromid gegebenenfalls in Gegenwart eines Verdünnungsmittels wie beispielsweise Dichlormethan und gegebenenfalls in Gegenwart eines Reaktionshilfsmittels wie beispielsweise Aluminiumtrichlorid bei Temperaturen zwischen - 20 °C und + 120 °C umsetzt (vgl. z.B. J. Org. Chemistry 40, 2304-2307 [1975]).

Acetophenone der Formel (IV) sind teilweise bekannt (vgl. z.B. Chem. Pharm. Bull. 23, 2223-2231 [1975]; Collect. Czech. Chem. Commun. 51, 2598-2616 [1986]; J. Org. Chem. 43, 1763-1768 [1978]; JP 56/2925; Chem. Ber. 120, 1151-1173 [1987]; Anal. Chim. Acta 54, 321-336 [1971]).

Acetophenone der Formel (IVa),

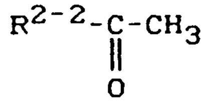 (IVa)

in welcher

$R^{2-2}$ für einen Rest der Formel

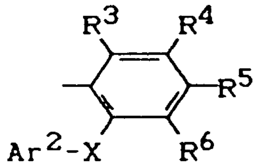 ; 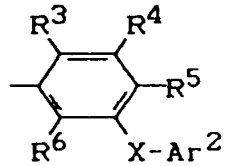 oder 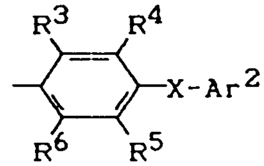

steht,

wobei

$R^3$, $R^4$, $R^5$, $R^6$ und X jeweils die oben angegebene Bedeutung haben und

$Ar^2$ für einen gegebenenfalls substituierten Arylrest steht, wobei mindestens ein Substituent für Halogenalkoxy, Halogenalkylthio, Halogenalkylsulfinyl oder Halogenalkylsulfonyl steht,

sind teilweise neu.

Man erhält bekannte und nicht bekannte Acetophenone der Formel (IV) in Analogie zu bekannten Verfahren, beispielsweise wenn man Halogenacetophenone der Formel (VI),

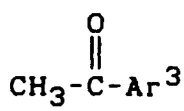 (VI)

in welcher

$Ar^3$ für einen Rest der Formel

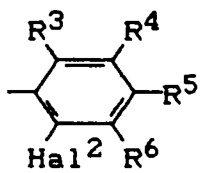 ; 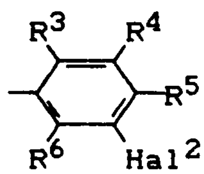 oder 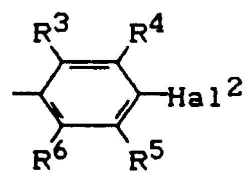

steht,

wobei

$Hal^2$ jeweils für Chlor oder Brom steht und

$R^3$, $R^4$, $R^5$ und $R^6$ jeweils die oben angegebene Bedeutung haben,

mit Phenolen der Formel (VII),

Ar-OH (VII)

in welcher

Ar die oben angegebene Bedeutung hat,

gegebenenfalls in Gegenwart eines Verdünnungsmittels wie beispielsweise Dioxan, gegebenenfalls in Gegenwart eines Reaktionshilfsmittels wie beispielsweise Kaliumhydroxid und gegebenenfalls in Gegenwart eines Katalysators wie beispielsweise Kupfer oder Palladium bei Temperaturen zwischen 50 °C und 150 °C umsetzt oder wenn man Diaryl(thio)ether-Derivate der Formel (V),

$R^2$-H (V)

in welcher

$R^2$ die oben angegebene Bedeutung hat,

mit Acetylierungsmitteln wie beispielsweise Acetylchlorid oder Acetanhydrid gegebenenfalls in Gegenwart eines Verdünnungsmittels wie beispielsweise Dichlormethan und gegebenenfalls in Gegenwart eines Reaktionshilfsmittels wie beispielsweise Aluminiumtrichlorid bei Temperaturen zwischen - 20 °C und + 120 °C umsetzt (vgl. z.B. J. org. Chem. 40, 2304-2307 [1975]) oder wenn man Anilin-Derivate der Formel (VIII),

$R^2$-$NH_2$ (VIII)

in welcher

$R^2$ die oben angegebene Bedeutung hat,

zunächst mit Natriumnitrit in Gegenwart einer Säure wie beispielsweise Salzsäure in üblicher Art und Weise bei Temperaturen zwischen - 20 °C und + 20 °C diazotiert und anschließend mit Acetaldoxim in Gegenwart eines Reaktionshilfsmittels wie beispielsweise Natriumacetat sowie gegebenenfalls in Gegenwart eines Katalysators wie beispielsweise Kupfer-II-sulfat bei Temperaturen zwischen 0 und 30 °C umsetzt und anschließend die so erhältlichen Acetophenonoxime der Formel (IX),

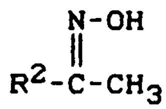 (IX)

in welcher

$R^2$ die oben angegebene Bedeutung hat,

mit konzentrierter Salzsäure bei Temperaturen zwischen 80 °C und 150 °C verseift (vgl. z.B. J. Chem. Soc. 1954, 1297; J. Med. Chem. 26, 1353 [1983]) oder wenn man Benzonitril-Derivate der Formel (X),

$R^2$-CN (X)

in welcher

$R^2$ die oben angegebene Bedeutung hat,

mit Methylmagnesiumbromid in Gegenwart eines Verdünnungsmittels wie beispielsweise Diethylether und gegebenenfalls in Gegenwart eines Katalysators wie beispielsweise Kupfer(I)chlorid bei Temperaturen zwischen - 20 °C und + 50 °C umsetzt und anschließend gegebenenfalls die mit Hilfe der oben beschriebenen Verfahren erhältlichen Acetophenone der Formel (IVb),

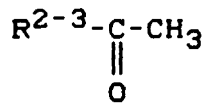 (IVb)

in welcher

$R^{2-3}$ für einen Rest der Formel

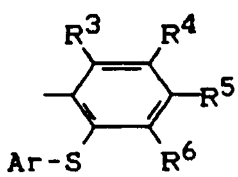 ; 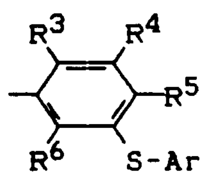 oder 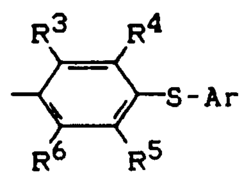

steht,

wobei

$R^3$, $R^4$, $R^5$, $R^6$ und Ar jeweils die oben angegebene Bedeutung haben,

mit üblichen Oxidationsmitteln wie beispielsweise 3-Chlorperbenzoesäure gegebenenfalls in Gegenwart eines Verdünnungsmittels wie beispielsweise Dichlormethan bei Temperaturen zwischen 0 °C und 50 °C

am Schwefel oxidiert (vgl. auch die Herstellungsbeispiele).

Diarylthioether-Derivate der Formel (V) sind bekannt oder erhältlich in Analogie zu bekannten Verfahren (vgl. z.B. Synth. Commun. 17, 685-692 [1987]).

Halogenacetophenone der Formel (VI) sind bekannt oder erhältlich in Analogie zu bekannten Verfahren (vgl. z.B. J. org. Chem. 46, 2169-2171 [1981]; J. chem. Soc. Perkin Trans. I, 1974, 1769-1771).

Phenole der Formel (VII) sind größtenteils allgemein bekannte Verbindungen der organischen Chemie (vgl. z.B. DE 12 57 784). Teilweise sind sie auch Gegenstand einer eigenen parallelen Patentanmeldung.

Fluor enthaltende Phenole der Formel (VIIa)

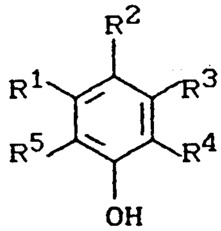

(VIIa)

in der

einer der Reste $R^1$ und $R^2$ für $OCF_3$, $OCF_2CFClH$, $OCF_2CF_2H$, $SCF_2CF_2H$ oder $SCF_2CFClH$ und der andere der Reste $R^1$ und $R^2$ für Wasserstoff oder $C_1$- bis $C_4$-Alkyl,

$R^3$ für Wasserstoff, Chlor oder $OCF_3$,

$R^4$ für Wasserstoff, $C_1$- bis $C_4$-Alkyl, $NO_2$, Chlor, $CONH_2$ oder COOH und

$R^5$ für Wasserstoff, Chlor oder $NO_2$ stehen,

wobei einer der Reste $R^1$ und $R^2$ auch für $SCF_3$ stehen kann, wenn $R^4$ für $NO_2$, $CONH_2$ oder COOH steht, sind neue Verbindungen.

Soweit es sich bei den genannten Substituenten um $C_1$-bis $C_4$-Alkyl handelt ist Methyl und Ethyl bevorzugt, insbesondere Methyl.

Bevorzugt sind solche Verbindungen der Formel (VIIa) bei denen

$R^1$ für Wasserstoff,
$R^2$ für $OCF_3$ oder $OCF_2CFClH$,
$R^3$ für Wasserstoff oder Chlor,
$R^4$ für Methyl, $NO_2$, Chlor oder $CONH_2$ und
$R^5$ für Wasserstoff, Chlor oder $NO_2$ stehen.

Beispiele für derartige bevorzugte Verbindungen sind solche, bei denen gilt (nicht genannte Reste = Wasserstoff):

$R^2$ = $OCF_3$, $R^4$ = Methyl,
$R^2$ = $OCF_2CFClH$, $R^4$ = Methyl,
$R^2$ = $OCF_3$, $R^4$ = $NO_2$,
$R^2$ = $OCF_3$, $R^3$ = $R^4$ = $R^5$ = Chlor,
$R^2$ = $OCF_3$, $R^4$ = $CONH_2$ und
$R^2$ = $OCF_3$, $R^4$ = $R^5$ = $NO_2$.

Besonders bevorzugt sind solche Verbindungen der Formel (VIIa), bei denen

$R^1$ für Wasserstoff,
$R^2$ für $OCF_3$ oder $OCF_2CFClH$,
$R^3$ für Wasserstoff oder Chlor,
$R^4$ für Methyl, $NO_2$ oder Chlor und
$R^5$ für Wasserstoff oder Chlor stehen.

Beispiele für derartige besonders bevorzugte Verbindungen sind solche, bei denen gilt (nicht genannte Reste = Wasserstoff):

$R^2$ = $OCF_3$, $R^4$ = Methyl,
$R^2$ = $OCF_2CFClH$, $R^4$ = Methyl,
$R^2$ = $OCF_3$, $R^4$ = $NO_2$ und
$R^2$ = $OCF_3$, $R^3$ = $R^4$ = $R^5$ = Chlor.

Eine ganz besonders bevorzugte Verbindung der Formel (VIIa) ist 2-Methyl-4-trifluormethoxy-benzol.

Die Fluor enthaltenden Phenole können auf verschiedene Weise hergestellt werden.

Beispielsweise kann man gemäß einer ersten Methode so vorgehen, daß man bei einem Phenol der Formel (VIIb)

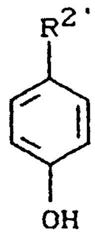

(VIIb)

in der

$R^{2'}$ für $OCF_3$, $OCF_2CFClH$, $OCF_2CF_2H$, $SCF_2CFClH$ oder $SCF_2CF_2H$ steht

in die 2-Position und gegebenenfalls zusätzlich in die 3- und/oder 6-Position Substituenten einführt. Beim Einführen einer $NO_2$-, COOH- oder $CONH_2$-Gruppe kann im Ausgangsphenol der Formel (VIIb) $R^{2'}$ auch für $SCF_3$ stehen.

Beispielsweise kann man so Chloratome durch Umsetzung mit einem Chlorierungsmittel einführen. Ein geeignetes Chlorierungsmittel ist z.B. elementares Chlor. Geeignete Temperaturen sind hierfür solche von beispielsweise 0 bis 50 °C. Man arbeitet zweckmäßigerweise in Gegenwart eines Katalysators, z.B. Eisen, und eines inerten Lösungsmittels, z.B. eines chlorierten Kohlenwasserstoffs.

Nitrogruppen kann man beispielsweise durch Umsetzung mit Salpetersäure einführen. Je nach Reaktionstemperatur und Konzentration der Salpetersäure lassen sich z.B. eine (in 2-Stellung) oder zwei Nitrogruppen (in 2- und 6-Stellung) einführen. Eine $NO_2$-Gruppe kann man beispielsweise mit 20 bis 33 gew.-%iger Salpetersäure bei 10 bis 30 °C, zwei $NO_2$-Gruppen beispielsweise mit 37 bis 60 gew.-%iger $HNO_3$ bei 50 bis 80 °C einführen.

Carboxylgruppen kann man beispielsweise durch Umsetzen mit Kohlendioxid unter erhöhtem Druck, bei erhöhter Temperatur und in Gegenwart einer Base einführen. Eine geeignete Base ist z.B. Kaliumcarbonat, geeignete Drucke beispielsweise solche von 10 bis 100 bar, geeignete Temperaturen beispielsweise solche von 150 bis 250 °C.

Säureamidgruppen kann man beispielsweise einführen, indem man gegebenenfalls wie oben beschrieben eingeführte Carboxylgruppen beispielsweise zunächst in Gegenwart eines Lösungsmittels mit $SOCl_2$ und anschließend mit Ammoniak umsetzt.

Beispielsweise kann man gemäß einer zweiten Methode so vorgehen, daß man ein Anilin der Formel (VIIIa)

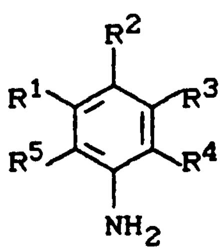

(VIIIa)

in der die Reste $R^1$ bis $R^5$ die bei Formel (VIIa) angegebene Bedeutung haben, diazotiert und verkocht. Die Diazotierung kann man beispielsweise mit Natriumnitrit in Gegenwart von Salzsäure vornehmen, die Verkochung beispielsweise bei azeotroper Entfernung von Wasser in Gegenwart von Schwefelsäure bei Temperaturen um 100 bis 130 °C.

Beispielsweise kann man gemäß einer dritten Methode so vorgehen, daß man eine Verbindung der Formel (VIIc)

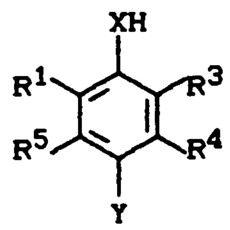

(VIIc)

in der

| | |
|---|---|
| X | für Sauerstoff oder Schwefel, |
| Y | für OH, NHAcetyl oder $NO_2$ und |
| $R^1$ | für Wasserstoff oder $C_1$- bis $C_4$-Alkyl stehen und |
| $R^3$ bis $R^5$ | die bei Formel (VIIa) angegebene Bedeutung haben |

mit Tetrafluorethylen umsetzt und im Falle Y = NHAcetyl oder $NO_2$ diese Gruppen in an sich bekannter Weise in eine OH-Gruppe überführt. Falls X für Sauerstoff steht, steht Y vorzugsweise für NHAcetyl oder $NO_2$, falls X für Schwefel steht, steht Y vorzugsweise für OH.

Diese Methode eignet sich besonders zur Herstellung von Fluor enthaltenden Phenolen der Formel (VIIa), bei denen $R^2$ für $OCF_2CF_2H$ oder $SCF_2CF_2H$ steht. Die Umsetzung mit Tetrafluorethylen erfolgt dabei vorzugsweise in Gegenwart einer Base und eines dipolaren, aprotischen Lösungsmittels beispielsweise bei Temperaturen im Bereich 50 bis 150 °C.

Es ist ausgesprochen überraschend, daß die Verbindungen der Formel (VIIa) in so guten Ausbeuten und Selektivitäten zugänglich sind, denn bei den vielen denkbaren Substitutions-, Eliminierungs- und Additionsmöglichkeiten an den einzusetzenden Edukten und den erhaltenen Produkten war in großem Umfang mit dem Ablauf von unerwünschten Nebenreaktionen zu rechnen. Trotzdem können die Verbindungen der Formel (VIIa) in Ausbeuten häufig von über 70 % der Theorie erhalten werden.

4-Trifluormethylmercapto-phenole der Formel (VIId)

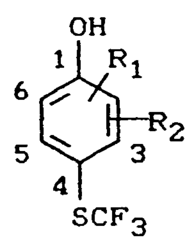

(VIId),

in der

| | |
|---|---|
| $R_1$ und $R_2$ | unabhängig voneinander jeweils für $C_1$- bis $C_4$-Alkyl, gegebenenfalls substituiertes $C_1$- bis $C_4$-Alkoxy, gegebenenfalls substituiertes $C_1$-bis $C_4$-Alkylthio und/oder Halogen oder |
| $R_1$ | für Wasserstoff und |
| $R_2$ | für $C_2$- bis $C_4$-Alkyl, gegebenenfalls substituiertes $C_1$- bis $C_4$-Alkoxy, gegebenenfalls substituiertes $C_1$- bis $C_4$-Alkylthio oder Fluor stehen, sind ebenfalls wertvolle Zwischenprodukte zur Synthese der erfindungsgemäßen Verbindungen der Formel (I). |

Als Substituenten bei gegebenenfalls substituiertem $C_1$-bis $C_4$-Alkoxy und bei gegebenenfalls substituiertem $C_1$-bis $C_4$-Alkylthio kommen bevorzugt Halogene, insbesondere Fluor in Frage.

Soweit $R_1$ und $R_2$ Halogen bedeuten kommen Fluor, Chlor, Brom und/oder Iod in Frage.

Vorzugsweise stehen $R_1$ und $R_2$ unabhängig voneinander jeweils für Methyl, Ethyl, Isopropyl, Methoxy, Fluormethoxy, Fluorethoxy, Fluorpropoxy, Fluorbutoxy, Fluormethylthio, Fluorethylthio, Fluorpropylthio, Fluorbutylthio, Fluor, Chlor oder Brom oder

$R_1$ für Wasserstoff und $R_2$ für Ethyl, Isopropyl, Methoxy, Fluormethoxy, Fluorethoxy, Fluorpropoxy, Fluorbutoxy, Fluormethylthio, Fluorethylthio, Fluorpropylthio, Fluorbutylthio, Fluor, Chlor oder Brom,

$C_1$- bis $C_4$-Alkyl steht vorzugsweise für Methyl, Ethyl oder Isopropyl, $C_2$- bis $C_4$-Alkyl vorzugsweise für Ethyl oder Isopropyl. Gegebenenfalls substituiertes $C_1$- bis $C_4$-Alkoxy steht vorzugsweise für Methoxy, Difluormethoxy, Difluorchlormethoxy, Trifluormethoxy, Trifluorethoxy, Tetrafluorethoxy, Trifluorchlorethoxy, Hexafluorpropoxy oder Hexafluorbutoxy.

Gegebenenfalls substituiertes $C_1$- bis $C_4$-Alkylthio steht vorzugsweise für Difluormethylthio, Difluorchlormethylthio, Trifluormethylthio, Trifluorethylthio, Tetrafluorethylthio, Trifluorchlorethylthio, Hexafluorpropylthio

oder Hexafluorbutylthio.

Halogen steht vorzugsweise für Fluor, Chlor oder Brom.

$R_1$ und $R_2$ können am aromatischen Ring beliebige Positionen einnehmen, d.h. bei $R_1$ in 2-Position kann $R_2$ in 3-, 5- oder 6-Position vorliegen und bei $R_1$ in 3-Position kann $R_2$ in 2-, 5- und 6-Position vorliegen. Die möglichen Isomeren, bei denen sich $R_1$ in 4- oder 5-Position befindet sind identisch mit den vorgenannten.

Besonders bevorzugte, von der Formel (VIId) umfaßte Einzelverbindungen enthalten folgende Reste $R_1$ und $R_2$ in den jeweils angegebenen Positionen:

$R_1$ = 3-H, $R_2$ = 1-Ethyl; $R_1$ = 3-H, $R_2$ = 2-Isopropyl; $R_1$ = 3-H, $R_2$ = 2-Methoxy; $R_1$ = $R_2$ = 2,6-Dimethyl; $R_1$ = $R_2$ = 2,3-Dimethyl; $R_1$ = $R_2$ = 3,5-Dimethyl; $R_1$ = $R_2$ = 2,5-Dimethyl; $R_1$ = 2-Methyl, $R_2$ = 5-Chlor; $R_1$ = 2-Methyl, $R_2$ = 3-Chlor; $R_1$ = 2-Ethyl, $R_2$ = 3-Methyl; $R_1$ = 2-Ethyl, $R_2$ = 3-Fluor; $R_1$ = 2-Methyl, $R_2$ = 3-Fluor; $R_1$ = 2-Methyl, $R_2$ = 6-Fluor; $R_1$ = 2-Methyl, $R_2$ = 5-Fluor; $R_1$ = 3-Methyl, $R_2$ = 6-Chlor und $R_1$ = 2-Methyl, $R_2$ = 6-Chlor.

Die vorliegende Erfindung betrifft auch ein Verfahren zur Herstellung von Verbindungen der Formel (VIId), das dadurch gekennzeichnet ist, daß man ein Phenol der Formel (VIIe)

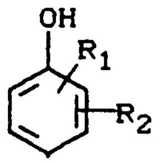

(VIIe),

in der

$R_1$ und $R_2$ die bei Formel (VIId) angegebene Bedeutung haben

mit Trifluormethylsulfenchlorid umsetzt.

Die erfindungsgemäße Umsetzung zur Herstellung von Verbindungen der Formel (VIId) kann bei Normaldruck, erhöhtem Druck oder erniedrigtem Druck durchgeführt werden. Vorzugsweise arbeitet man bei Normaldruck.

Die erfindungsgemäße Umsetzung zur Herstellung von Verbindungen der Formel (VIId) kann beispielsweise bei Temperaturen im Bereich -20 bis +100 °C durchgeführt werden. Vorzugsweise arbeitet man bei Temperaturen im Bereich von 0 bis 60 °C.

Die Reaktionspartner können im Prinzip in beliebigen Mengenverhältnissen eingesetzt werden. Vorzugsweise setzt man auf 1 Mol Trifluormethylsulfenchlorid 1 bis 10 Mole des jeweiligen Phenols der Formel (VIIe) ein. Besonders bevorzugt setzt man auf 1 Mol Trifluormethylsulfenchlorid 1,05 bis 8 Mole des jeweiligen Phenols der Formel (VIIe) ein. Bei der Verwendung von Phenol im Überschuß kann man im allgemeinen einen quantitativen Verbrauch des Trifluormethylsulfenchlorids und eine Bildung von weniger Nebenprodukten erreichen.

Die erfindungsgemäße Umsetzung zur Herstellung von Verbindungen der Formel (VIId) kann in Anwesenheit oder Abwesenheit von Katalysatoren durchgeführt werden. Vorzugsweise arbeitet man in Gegenwart von Katalysatoren, weil dann im allgemeinen auch bei relativ niedrigen Temperaturen vorteilhaft gearbeitet werden kann. Als Katalysatoren kommen beispielsweise Lewis-Säuren oder Basen in Frage. Beispiele für Lewis-Säuren sind Eisentrichlorid, Titantetrachlorid, Bortrifluorid, Antimonpentachlorid und Aluminiumtrichlorid. Lewis-Säuren können z.B. in Mengen von 0,01 bis 0,2 Mol pro Mol Trifluormethylsulfenchlorid eingesetzt werden. Beispiele für Basen sind Alkalimetallcarbonate, Triphenylphosphin und tertiäre Stickstoffbasen. Bevorzugt sind tertiäre Amine wie Pyridin, Picolin, Triethylamin, 1,5-Diazabicyclo[4.3.0]-non-5-en und 1,8-Diazabicyclo[5.4.0]-undec-7-en. Basen können z.B. in gleicher oder höherer molarer Menge als Trifluormethylsulfenchlorid eingesetzt werden.

Die erfindungsgemäße Umsetzung zur Herstellung von Verbindungen der Formel (VIId) kann in Gegenwart oder Abwesenheit von Lösungsmitteln durchgeführt werden. Geeignete Lösungsmittel sind z.B. Ether oder Halogenalkane.

Phenole der Formel (VIIe) sind bekannt und gut zugänglich. Trifluormethylsulfenchlorid ist ebenfalls bekannt und gut zugänglich.

Nach Durchführung der erfindungsgemäßen Umsetzung zur Herstellung von Verbindungen der Formel (VIId) erhält man häufig Reaktionsgemische, die neben dem gewünschten Produkt auch Bis-trifluormethylmercapto-phenole und gegebenenfalls unumgesetztes Ausgangsphenol enthalten und deshalb aufgearbeitet werden müssen. Die Auftrennung des Reaktionsgemisches ist in den meisten Fällen durch Destillation über eine Kolonne möglich, besonders dann, wenn wenig oder keine Bis-trifluormethylmercapto-phenole vorliegen. Teilweise kristallisiert eine Komponente, z.B. überschüssiges Ausgangsphenol oder das gebildete 4-Trifluormethylmercapto-phenol, auch aus den Reaktionsgemisch aus und kann dann durch Filtration abgetrennt werden.

Eine besondere Aufarbeitungsform des Reaktionsgemisches besteht darin, daß man zunächst gegebenenfalls vorhandene Lösungsmittel, Katalysatoren und/oder Hydrochloride von Basen abtrennt, z.B. durch Filtration und/oder einfache Destillation, und anschließend säulenchromatographisch an Kieselgel trennt. Als mobile Phase kann beispielsweise ein Kohlenwasserstoff, insbesondere Toluol, verwendet werden. Im allgemeinen fällt dann als erste Fraktion das Bis-trifluormethylmercapto-substituierte Phenol und als zweite Fraktion das 4-Trifluormethylmercapto-phenol der Formel (VIId) an. Letzteres kann man in reiner Form gewinnen, indem man die für die säulenchromatographische Trennung eingesetzte mobile Phase, z.B. Toluol, aus der entsprechenden Fraktion entfernt, z.B. durch Destillation.

Weitere wertvolle Ausgangsstoffe zur Synthese erfindungsgemäßer Verbindungen der Formel (I) sind 4-Trifluormethylsulfonylphenole der Formel (VIIf)

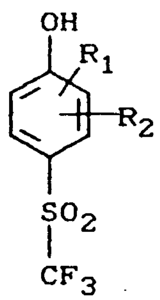

(VIIf),

in der $R_1$ und $R_2$ die bei Formel (VIId) angegebenene Bedeutung haben. Auch die bevorzugten und besonders bevorzugten Bedeutungen von $R_1$ und $R_2$ in Formel (VIIf) sind wie bei Formel (VIId) angegeben.

4-Fluormethylsulfonyl-phenolen der Formel (VIIf) können erhalten werden, indem ein 4-Trifluormethylmercaptophenol der Formel (VIId) bei erhöhter Temperatur in Gegenwart einer Säure mit einem aktivierten Sauerstoff enthaltenden Oxidationsmittel oxidiert wird.

Für diese Oxidation sind beispielsweise Temperaturen im Bereich 50 bis 120 °C geeignet. Vorzugsweise arbeitet man bei 65 bis 100 °C.

Als Säuren kommen z.B. organische Säuren in Frage, wie gegebenenfalls durch Halogen substituierte aliphatische Carbonsäuren mit 1 bis 6 C-Atomen, aber auch Mineralsäuren wie Phosphorsäure oder Schwefelsäure. Bevorzugt sind Essigsäure, Propionsäure, Chloressigsäure und Trifluoressigsäure. Besonders bevorzugt ist Essigsäure.

Als aktivierten Sauerstoff enthaltende Oxidationsmittel kommen beispielsweise Wasserstoffperoxid, Percarbonsäuren, Caro'sche Säure und deren Salze, Peroxodisulfat und molekularer Sauerstoff in Kombination mit Katalysatoren in Frage. Bevorzugt sind Wasserstoffperoxid, Caro'sche Säure und deren Salze, insbesondere Wasserstoffperoxid, welches beispielsweise in 10 bis 50 gew.-%iger wäßriger Lösung eingesetzt werden kann. Das jeweils eingesetzte Oxidationsmittel kann gegebenenfalls in Wasser und/oder einem organischen Lösungsmittel gelöst sein. Die eingesetzten aktiven Sauerstoff enthaltenden Oxidationsmittel müssen nicht unbedingt in der eingesetzten Form mit der Verbindung der Formel (VIId) reagieren, sondern sie können auch vor dieser Reaktion ganz oder teilweise in andere aktivierten Sauerstoff enthaltende Oxidationsmittel umgewandelt werden. Beispielsweise kann aus Wasserstoffperoxid und Schwefelsäure Caro'sche Säure oder aus Wasserstoffperoxid und Essigsäure Peressigsäure entstehen. Ebenso kann man das jeweils gewünschte aktivierten Sauerstoff enthaltende Oxidationsmittel auch erst in situ entstehen lassen, z.B. Caro'sche Säure aus Wasserstoffperoxid und Schwefelsäure.

Das Oxidationsmittel wird vorzugsweise in der stöchiometrisch erforderlichen Menge oder in einem Überschuß von bis zu 100 Mol-% eingesetzt.

Das erfindungsgemäße Verfahren zur Herstellung von 4-Trifluormethylsulfonyl-phenolen der Formel (VIIf) kann in Anwesenheit oder Abwesenheit von Lösungsmitteln durchgeführt werden. Als Lösungsmittel sind beispielsweise Ether, wie Dioxan oder Diglyme geeignet. Beim Arbeiten in Gegenwart organischer Säuren, insbesondere Essigsäure, kann die Säure auch als Lösungsmittel fungieren.

Die Aufarbeitung des Reaktionsgemisches kann beispielsweise so erfolgen, daß man eventuell vorhandenes überschüssiges Oxidationsmittel zerstört und dann das 4-Trifluormethylsulfonyl-phenol der Formel (VIIf) durch Eingießen in Wasser ausfällt oder durch Abdestillieren von Lösungsmittel und/oder Säure zur Kristallisation bringt und abschließend jeweils filtriert.

Es ist ausgeprochen überraschend, daß es erfindungsgemäß gelingt, 4-Trifluormethylsulfonyl-phenole mit guten Ausbeuten aus den entsprechenden Mercapto-phenolen zu erhalten, denn es sind eine ganze Reihe von Verfahren bekannt, bei denen aus Phenolen durch Oxidation mit Wasserstoffperoxid oder Persäuren die entsprechenden Chinone entstehen.

Anilin-Derivate der Formel (VIII) sind bekannt oder erhältlich in Analogie zu bekannten Verfahren (vgl. z.B. EP 34 771; J. Chem. Soc. Perkin Trans I, 1976, 1279-1285).

Benzonitril-Derivate der Formel (X) sind bekannt oder erhältlich in Analogie zu bekannten Verfahren (vgl. z.B. US 39 50 379; J. Med. Chem. 29, 427-433 [1986]).

Als Verdünnungsmittel zur Durchführung des erfindungsgemäßen Verfahrens kommen inerte organische oder anorganische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid, Alkohole wie Methanol, Ethanol oder Propanol, Basen wie Pyridin sowie gegebenenfalls auch deren Gemische mit Wasser oder reines Wasser.

Das erfindungsgemäße Verfahren wird gegebenenfalls in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, Ammoniak sowie primäre, sekundäre oder tertiäre Amine, wie Triethylamin, N,N-Dimethylanilin, Pyridin, N,N-Dimethylaminopyridin, Diazabicyclooctan, (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 0 °C und 180 °C, vorzugsweise bei Temperaturen zwischen 20 °C und 150 °C.

Zur Durchführung des erfindungsgemäßen Verfahrens setzt man pro Mol an Thioharnstoff-Derivat der Formel (II) im allgemeinen 1.0 bis 1.5 Mol, vorzugsweise 1.0 bis 1.2 Mol an Keton-Derivat der Formel (III) und gegebenenfalls 1.0 bis 1.2 Mol an Reaktionshilfsmittel ein.

Die Reaktionsdurchführung, Aufarbeitung und Isolierung der Reaktionsprodukte erfolgt nach allgemein üblichen Methoden. Eventuell auftretende Nebenprodukte können mit üblichen chromatographischen Methoden abgetrennt werden.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Verbindungen der Formel (I) und ihre Säureadditions-Salze weisen antimikrobielle, insbesondere starke antibakterielle und antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizien können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quaternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit-und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise von 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise von 5 bis 150 mg/kg Körpergewicht je 24 Stunden und bei parenteraler Applikation in Gesamtmengen von etwa 2,5 bis etwa 50, vorzugsweise von 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

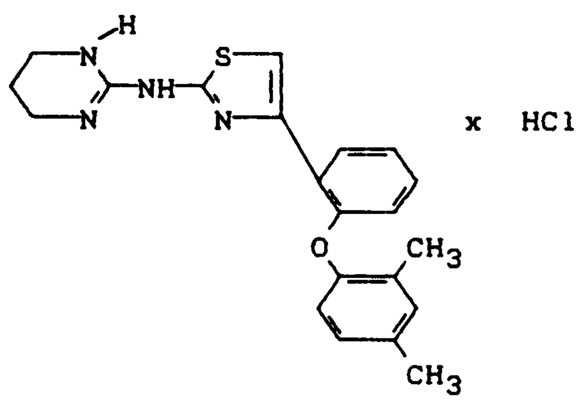

Zu 27,45 g (0,1 Mol) 2-(2,4-Dimethylphenoxy)-phenacylchlorid in 100 ml Aceton gibt man 15,8 g (0,12 Mol) N-(1,4,5,6-Tetrahydropyrimidinyl)-thioharnstoff (vgl. z.B. DE-OS 32 20 118), erhitzt 2 Stunden auf Rückflußtemperatur, kühlt ab, saugt ausgefallenes Produkt ab, wäscht mit Aceton und trocknet.

Man erhält 37,8 g (91,4 % der Theorie) an 4-[2-(2,4-Dimethylphenoxy)-phenyl]-2-[2-(1,4,5,6-tetrahydropyrimidinyl-amino]-thiazol Hydrochlorid vom Schmelzpunkt 160 °C.

Beispiel 2

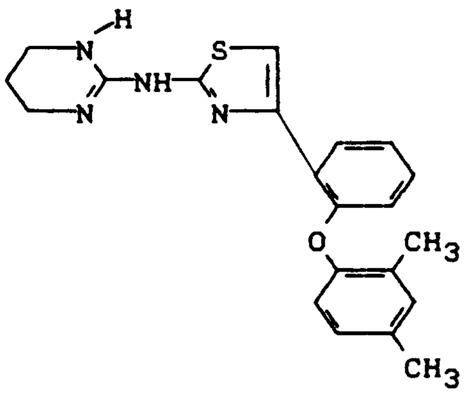

20,72 g (0,05 Mol) 4-[2-(2,4-Dimethylphenoxy)-phenyl]-2-[2-(1,4,5,6-tetrahydropyrimidinyl)-amino]-thiazol Hydrochlorid werden mit 300 ml 1N Natronlauge 30 Minuten bei Raumtemperatur gerührt. Der unlösliche Feststoff wird abgesaugt, mit Wasser neutral gewaschen und getrocknet.

Man erhält 16,2 g (86 % der Theorie) an 4-[2-(2,4-Dimethylphenoxy)-phenyl]-2-[2-(1,4,5,6-tetrahydropyrimidinyl)-amino]-thiazol vom Schmelzpunkt 191 - 192 °C.

Beispiel 3

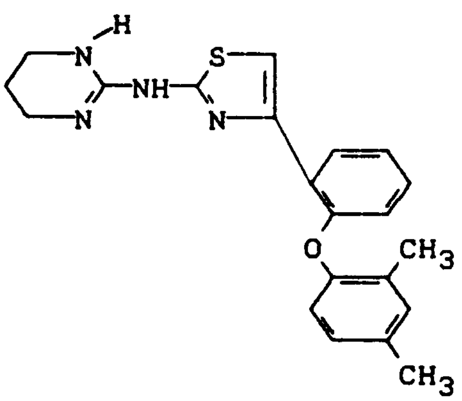

x $H_3PO_4$

Zu 15,12 g (0,04 Mol) 4-[2-(2,4-Dimethylphenoxy)-phenyl]-2-[2-(1,4,5,6-tetrahydropyrimidinyl)-amino]-thiazol in 200 ml Ethanol gibt man 4,72 g (0,041 Mol) 85prozentige Phosphorsäure, erhitzt 1 Stunde auf Rückflußtemperatur, kühlt ab, saugt ausgefallenen Feststoff ab, wäscht mit Petrolether nach und trocknet.

Man erhält 18,66 g (98 % der Theorie) an 4-[2-(2,4-Dimethylphenoxy)-phenyl]-2-[2-(1,4,5,6-tetrahydropyrimidinyl)-amino]-thiazol Dihydrogenphosphat vom Schmelzpunkt 218 °C.

In entsprechender Weise und gemäß den allgemeinen Angaben zur Herstellung erhält man die folgenden substituierten 2-Aminothiazole der allgemeinen Formel (I):

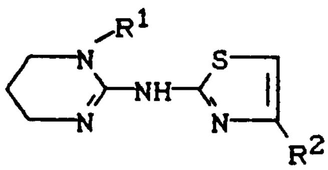

(I)

| Bsp. Nr. | $R^1$ | $R^2$ | Säureadditionssalz mit | Schmelzpunkt /°C |
|---|---|---|---|---|
| 4 | H | 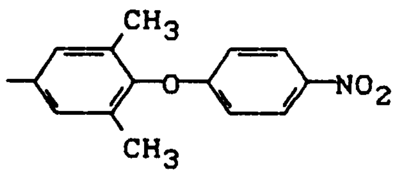 | - | 246-247 |
| 5 | H | 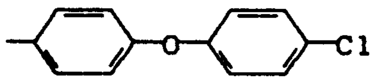 | HBr | 244-245 |
| 6 | H | 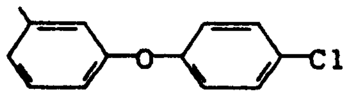 | HBr | 168-170 |
| 7 | H | 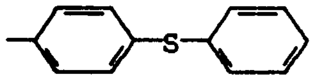 | HBr | 200-201 |

| Bsp. Nr. | $R^1$ | $R^2$ | Säureaddi-tionssalz mit | Schmelz-punkt /° C |
|---|---|---|---|---|
| 8 | H | 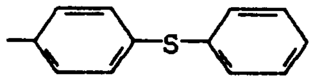 | 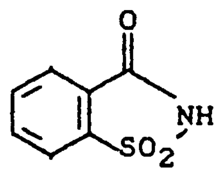 | 233-234 |
| 9 | H | 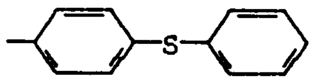 | 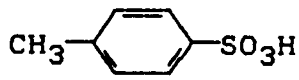 | 200-201 |
| 10 | H | 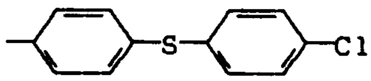 | HCl | 236-237 |
| 11 | H | 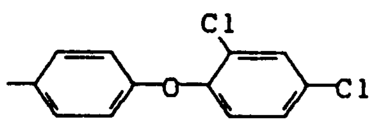 | HBr | 240-242 |
| 12 | H | 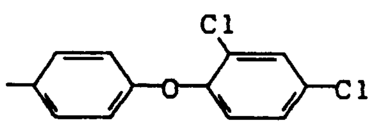 | - | 167-169 |
| 13 | H | 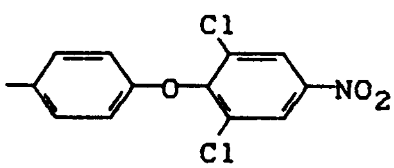 | HCl | 285-287 |
| 14 | H | 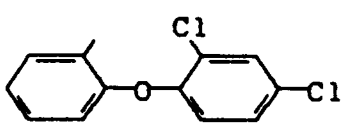 | HBr | 207-208 |
| 15 | H | 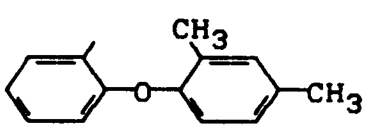 | HBr | 212-214 |

| Bsp. Nr. | $R^1$ | $R^2$ | Säureadditionssalz mit | Schmelzpunkt /°C |
|---|---|---|---|---|
| 16 | H | 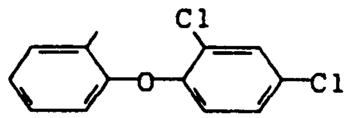 | $CH_3$-$SO_3H$ | 197-198 |
| 17 | H | 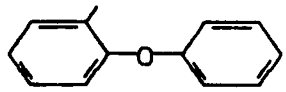 | HBr | 229-230 |
| 18 | H | 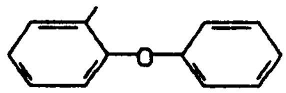 | - | 199-200 |
| 19 | H | 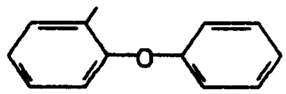 | HCl | 214-215 |
| 20 | H | 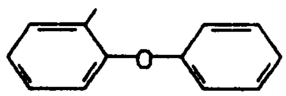 | 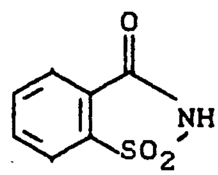 | 225-226 |
| 21 | H | 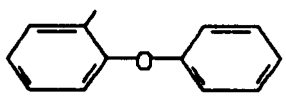 | 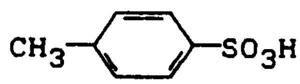 | 174-175 |
| 22 | H | 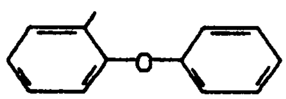 | $CH_3$-$SO_3H$ | 277-279 |
| 23 | H | 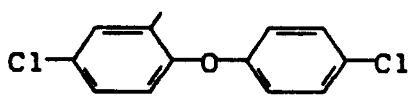 | HBr | 240-241 |
| 24 | H | 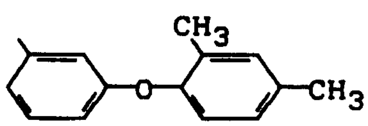 | HBr | 106-107 |

| Bsp. Nr. | $R^1$ | $R^2$ | Säureadditionssalz mit | Schmelzpunkt /°C |
|---|---|---|---|---|
| 25 | H | 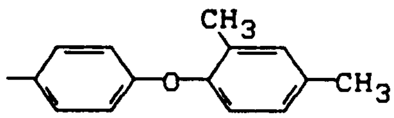 | HBr | 227-228 |
| 26 | H | 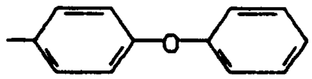 | HBr | 219 |
| 27 | H | 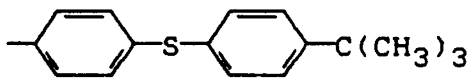 | HBr | 166-168 |
| 28 | H | 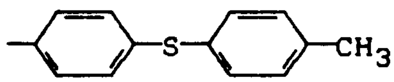 | HBr | 218-220 |
| 29 | H | 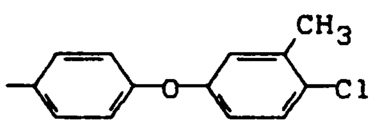 | HBr | 248-250 |
| 30 | H | 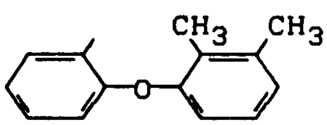 | $CH_3$-$SO_3H$ | 217 |
| 31 | H | 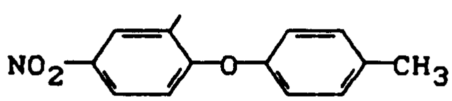 | HCl | 290-292 |
| 32 | H | 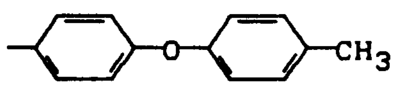 | HBr | 227-229 |
| 33 | H | 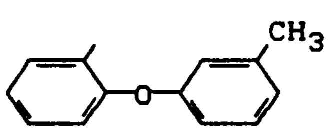 | HBr | 253-254 |

| Bsp. Nr. | $R^1$ | $R^2$ | Säureadditionssalz mit | Schmelzpunkt /°C |
|---|---|---|---|---|
| 34 | H | 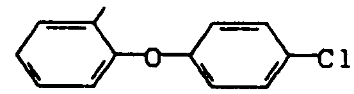 | HBr | 244-245 |
| 35 | H | 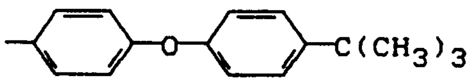 | HBr | 241-243 |
| 36 | H | 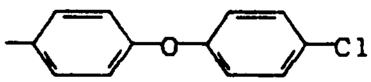 | HBr | 165-167 |
| 37 | H | 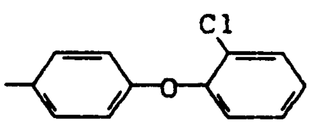 | HBr | 230-231 |
| 38 | H | 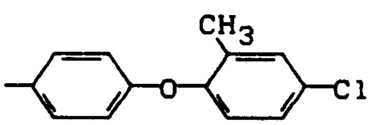 | HBr | 263-264 |
| 39 | H | 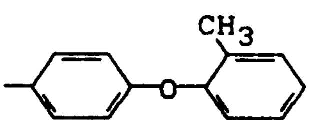 | HBr | 211-213 |
| 40 | H | 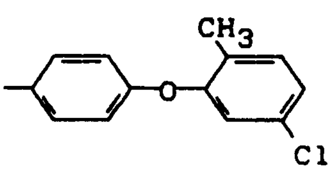 | HBr | 236-238 |
| 41 | H | 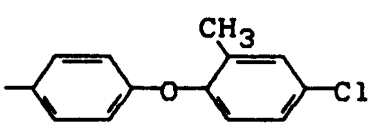 | HBr | 193-195 |
| 42 | H | 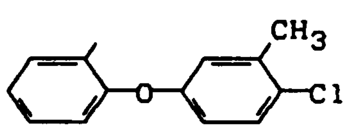 | HBr | 248-249 |

| Bsp. Nr. | $R^1$ | $R^2$ | Säureadditionssalz mit | Schmelzpunkt /° C |
|---|---|---|---|---|
| 43 | H | 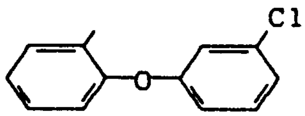 | HBr | 249-250 |
| 44 | H | 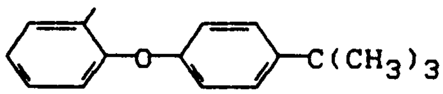 | HCl | 189-190 |
| 45 | H | 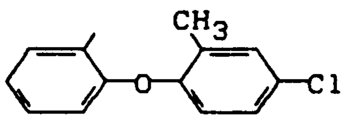 | HBr | 232-233 |
| 46 | H | 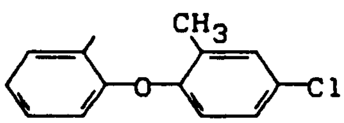 | HCl | 238 |
| 47 | H | 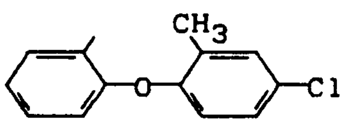 | $CH_3$-$SO_3H$ | 216-217 |
| 48 | H | 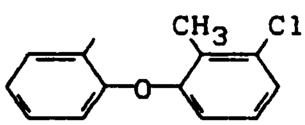 | HBr | 265-266 |
| 49 | H | 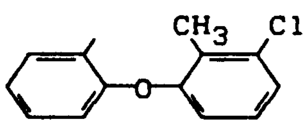 | HCl | 216 |
| 50 | H | 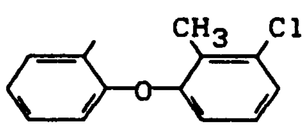 | $CH_3$-$SO_3H$ | 228-229 |

| Bsp. Nr. | $R^1$ | $R^2$ | Säureadditionssalz mit | Schmelzpunkt /°C |
|---|---|---|---|---|
| 51 | H | 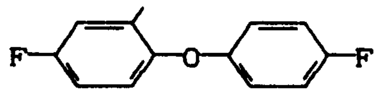 | HBr | 286-287 |
| 52 | H | 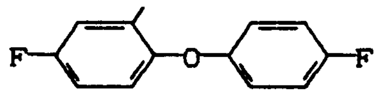 | HCl | 273-274 |
| 53 | H | 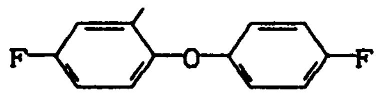 | $CH_3$-$SO_3H$ | 235-236 |
| 54 | H | 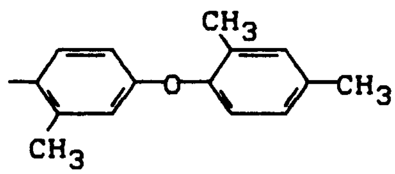 | - | 228-229 |
| 55 | H | 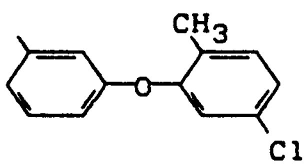 | HBr | 229-230 |
| 56 | H | 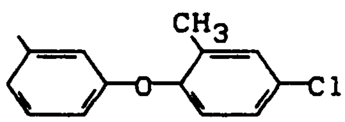 | HBr | 277-278 |
| 57 | H | 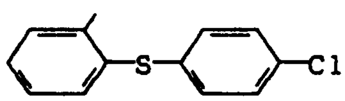 | HBr | 200 |
| 58 | H | 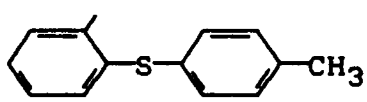 | HBr | 216 |

| Bsp. Nr. | $R^1$ | $R^2$ | Säureadditionssalz mit | Schmelzpunkt /° C |
|---|---|---|---|---|
| 59 | H | 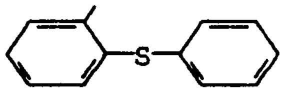 | HBr | 144 |
| 60 | H | 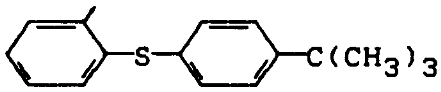 | - | 201 |
| 61 | H | 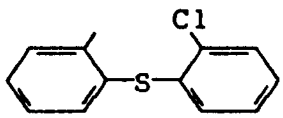 | HBr | 199-200 |
| 62 | H | 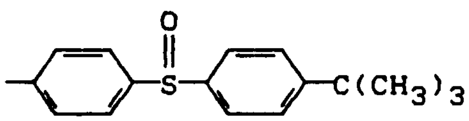 | HBr | 162 |
| 63 | H | 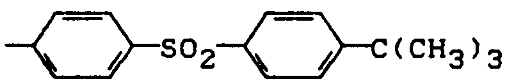 | HBr | 255-256 |
| 64 | H | 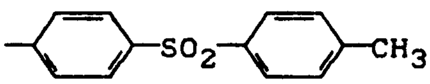 | HBr | 283-284 |
| 65 | H | 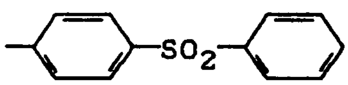 | HBr | 286-287 |
| 66 | H | 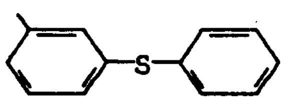 | - | 214 |
| 67 | H | 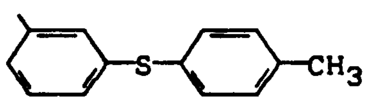 | HBr | 225 |
| 68 | H | 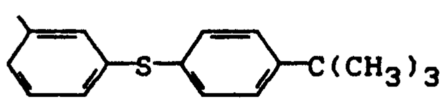 | - | 199 |

| Bsp. Nr. | $R^1$ | $R^2$ | Säureadditionssalz mit | Schmelzpunkt /° C |
|---|---|---|---|---|
| 69 | H | 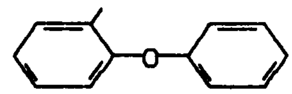 | $CH_3$-$SO_3H$ | 〉250 |
| 70 | H | 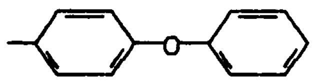 | $CH_3$-$SO_3H$ | 203 |
| 71 | H | 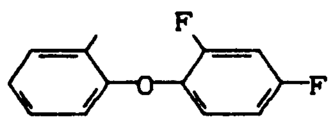 | HCl | 236 |
| 72 | H | 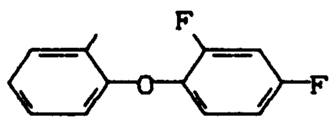 | - | 199 |
| 73 | H | 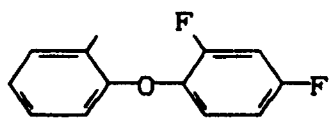 | $CH_3$-$SO_3H$ | 220-222 |
| 74 | H | 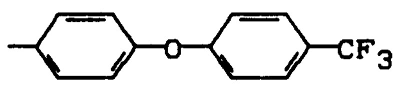 | HCl | 〉250 |
| 75 | H | 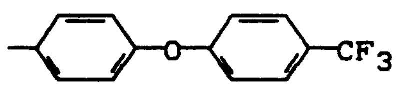 | - | 22-223 |
| 76 | H | 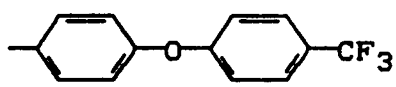 | $CH_3$-$SO_3H$ | 207 |
| 77 | H | 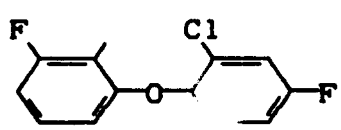 | HCl | 172 |

| Bsp. Nr. | $R^1$ | $R^2$ | Säureadditionssalz mit | Schmelzpunkt /° C |
|---|---|---|---|---|
| 78 | H | 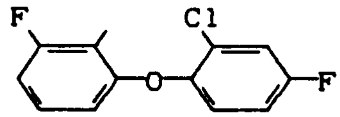 | - | 168 (Zers.) |
| 79 | H | 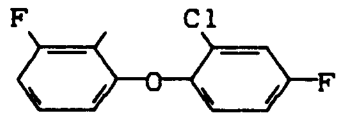 | $CH_3-SO_3H$ | 169 (Zers.) |
| 80 | H | 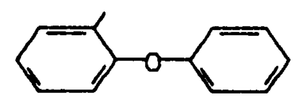 | 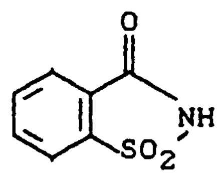 | 216 |
| 81 | H | 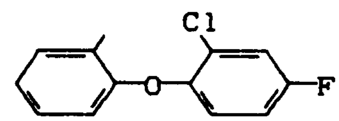 | HCl | 188 |
| 82 | H | 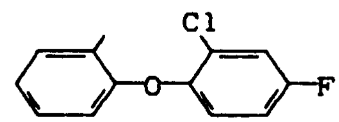 | - | 234-235 |
| 83 | H | 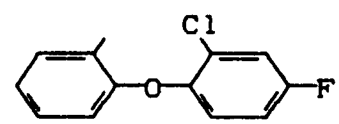 | $CH_3-SO_3H$ | 234 |
| 84 | H | 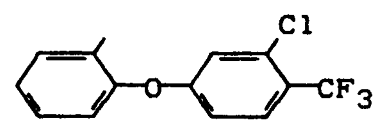 | - | 124-125 |
| 85 | H | 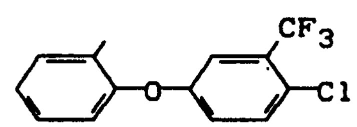 | - | 128 (Zers.) |

| Bsp. Nr. | $R^1$ | $R^2$ | Säureadditionssalz mit | Schmelzpunkt /° C |
|---|---|---|---|---|
| 86 | H | 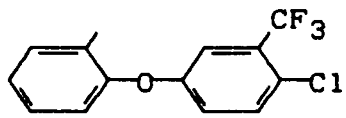 | $CH_3$-$SO_3H$ | 240 |
| 87 | H | 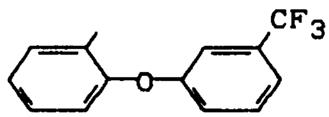 | HCl | 172 |
| 88 | H | 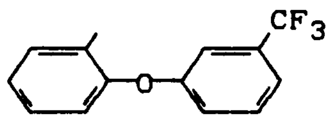 | - | 158 |
| 89 | H | 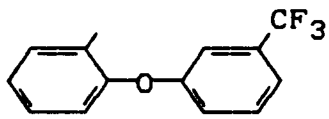 | $CH_3$-$SO_3H$ | 135 (Zers.) |
| 90 | H | 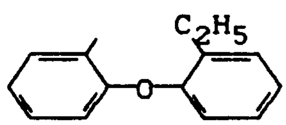 | HCl | 136 |
| 91 | H | 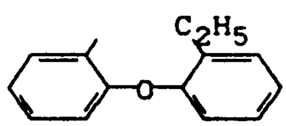 | - | 149 |
| 92 | H | 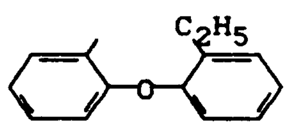 | $CH_3$-$SO_3H$ | 164 |
| 93 | H | 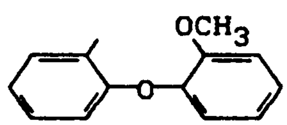 | HCl | 250-252 |

| Bsp. Nr. | $R^1$ | $R^2$ | Säureadditionssalz mit | Schmelzpunkt /°C |
|---|---|---|---|---|
| 94 | H | 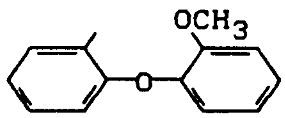 | - | 240 |
| 95 | H | 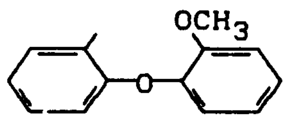 | $CH_3$-$SO_3H$ | 230 (Zers.) |
| 96 | H | 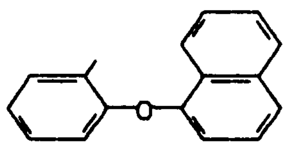 | HCl | 220 |
| 97 | H | 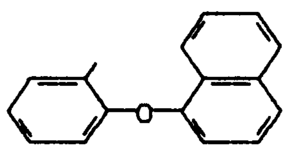 | - | 223 |
| 98 | H | 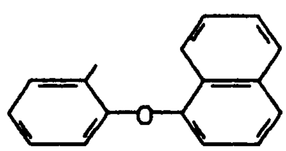 | $CH_3$-$SO_3H$ | 〉250 |
| 99 | H | 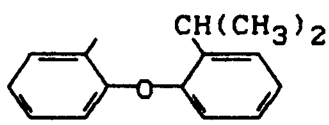 | HCl | 134-135 |
| 100 | H | 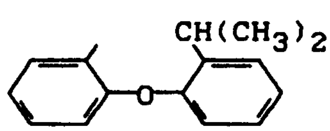 | - | 225 |
| 101 | H | 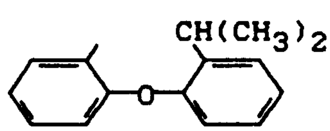 | $CH_3$-$SO_3H$ | 162 |

| Bsp. Nr. | $R^1$ | $R^2$ | Säureadditionssalz mit | Schmelzpunkt /° C |
|---|---|---|---|---|
| 102 | H | 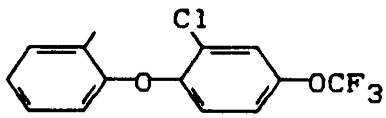 | HCl | 138 |
| 103 | H | 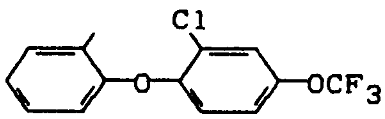 | - | 205 |
| 104 | H | 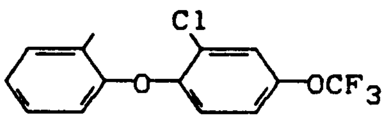 | $CH_3$-$SO_3H$ | 208 |
| 105 | H | 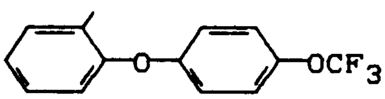 | HCl | 162 |
| 106 | H | 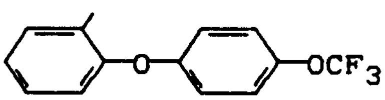 | - | 173 |
| 107 | H | 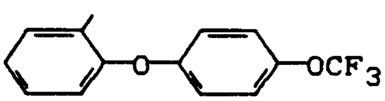 | $CH_3$-$SO_3H$ | 220 |
| 108 | H | 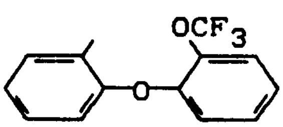 | HCl | 196 |
| 109 | H | 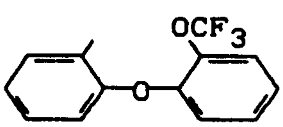 | - | 155 |
| 110 | H | 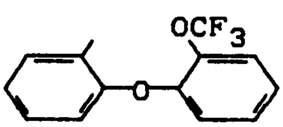 | $CH_3$-$SO_3H$ | 196 |

| Bsp. Nr. | $R^1$ | $R^2$ | Säureadditionssalz mit | Schmelzpunkt /° C |
|---|---|---|---|---|
| 111 | H | 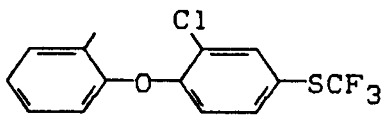 | HCl | 250-251 |
| 112 | H | 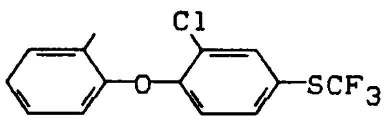 | - | 238 |
| 113 | H | 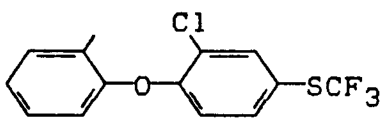 | $CH_3-SO_3H$ | 176 (Zers.) |
| 114 | H | 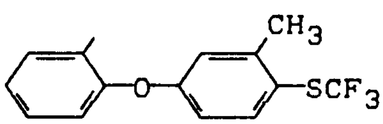 | HCl | 127 |
| 115 | H | 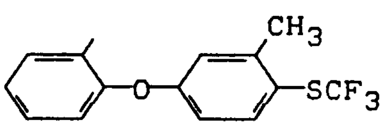 | - | 134 |
| 116 | H | 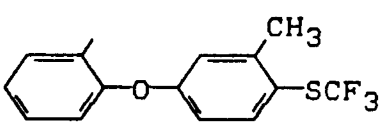 | $CH_3-SO_3H$ | 217 |
| 117 | H | 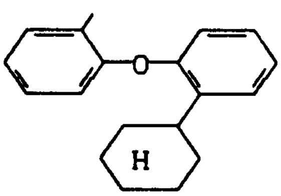 | HCl | 150 (Zers.) |
| 118 | H | 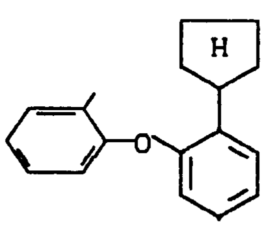 | - | 145-146 |

| Bsp. Nr. | $R^1$ | $R^2$ | Säureadditionssalz mit | Schmelzpunkt /° C |
|---|---|---|---|---|
| 119 | H | 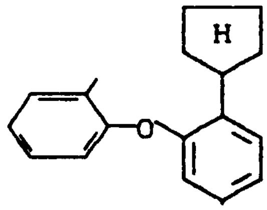 | $CH_3-SO_3H$ | 177 |
| 120 | H | 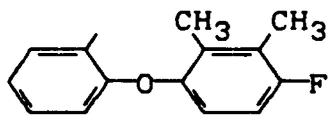 | - | 235-237 |
| 121 | H | 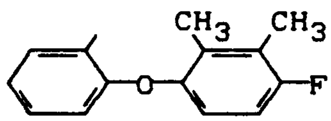 | - | 235-237 |
| 122 | H | 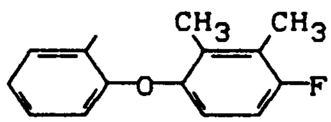 | $CH_3-SO_3H$ | 〉250 |
| 123 | H | 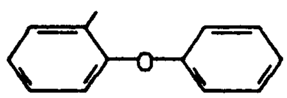 | $CH_3-CH(OH)-COOH$ | 179 |
| 124 | H | 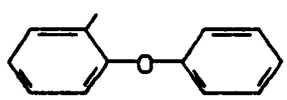 | $HOOC-CH_2-CH_2-COOH$ | 160 |
| 125 | H | 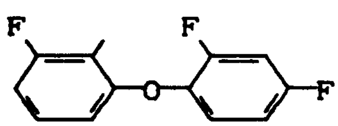 | - | 103-105 (Zers.) |

| Bsp. Nr. | $R^1$ | $R^2$ | Säureaddi-tionssalz mit | Schmelz-punkt /° C |
|---|---|---|---|---|
| 126 | H | 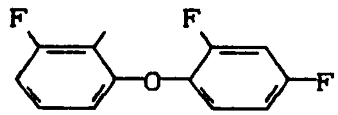 | $CH_3-SO_3H$ | 107 (Zers.) |
| 127 | H | 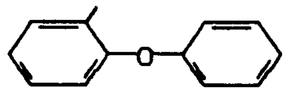 | OH<br>\|<br>$HOOC-CH_2-C-CH_2-COOH$<br>\|<br>COOH | 112 |
| 128 | H | 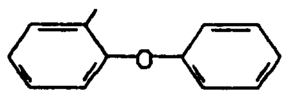 | $CH_3-(CH_2)_7-CH$<br>‖<br>$HOOC-(CH_2)_7-CH$ | 169 |
| 129 | H | 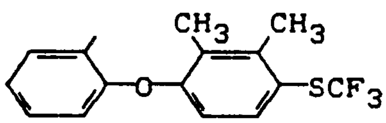 | HCl | 156 |
| 130 | H | 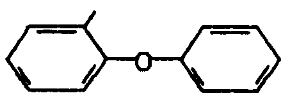 | $HOOC-(CH_2)_4-COOH$ | 109 |
| 131 | H | 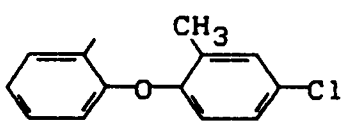 | $CH_3-SO_3H$ | 215 |
| 132 | H | 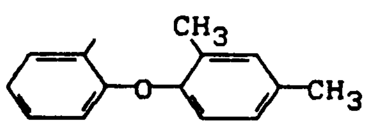 | OH<br>\|<br>$HOOC-CH_2-C-CH_2-COOH$<br>\|<br>COOH | 192 (Zers.) |
| 133 | H | 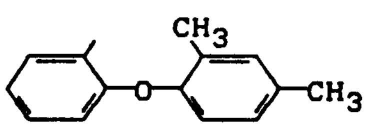 | $CH_3-CH-COOH$<br>\|<br>OH | 126 |

| Bsp. Nr. | $R^1$ | $R^2$ | Säureadditionssalz mit | Schmelzpunkt /° C |
|---|---|---|---|---|
| 134 | H | 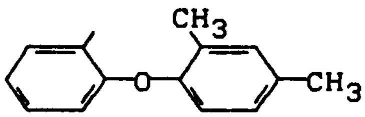 | $HOOC-CH_2-CH_2-COOH$ | 150 (Zers.) |
| 135 | H | 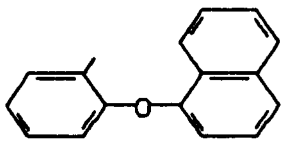 | 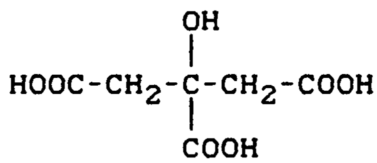 | 173 (Zers.) |
| 136 | H | 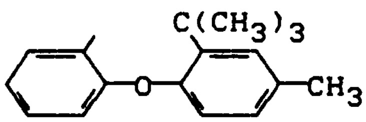 | - | 232 °C |
| 137 | H | 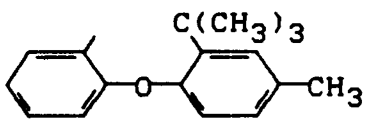 | $CH_3-SO_3H$ | 〉250 |
| 138 | H | 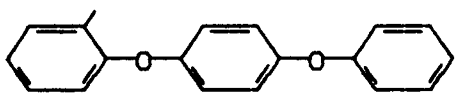 | HCl | 128 |
| 139 | H | 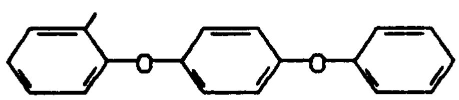 | - | 159 |
| 140 | H | 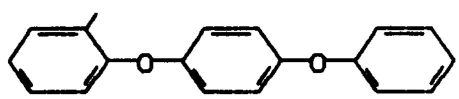 | $CH_3-SO_3H$ | 218 |
| 141 | H | 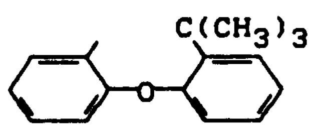 | - | 240 |

| Bsp. Nr. | $R^1$ | $R^2$ | Säureaddi-tionssalz mit | Schmelz-punkt /°C |
|---|---|---|---|---|
| 142 | H | 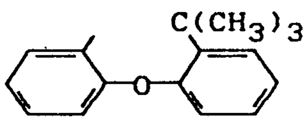 | $CH_3$-$SO_3H$ | 210 (Zers.) |
| 143 | H | 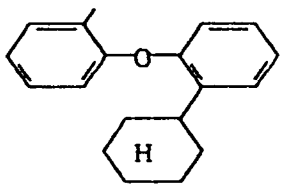 | - | 198 |
| 144 | H | 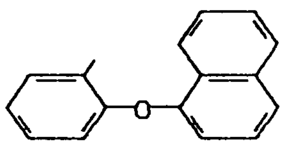 | - | 〉250 |
| 145 | H | 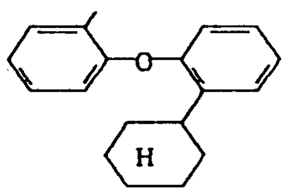 | $CH_3$-$SO_3H$ | 180 |
| 146 | H | 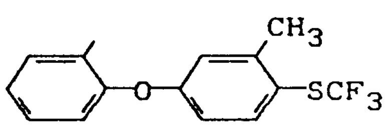 | $CH_2$-COOH<br>\|<br>HOOC-$CH_2$-CH-OH | 158 |
| 147 | H | 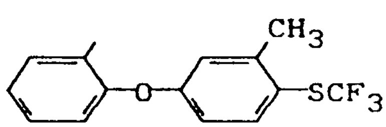 | $CH_3$-CH-COOH<br>\|<br>OH | 137 |
| 148 | H | 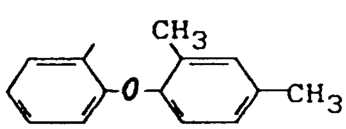 | $C_2H_5O$-$SO_3H$ | 215 |

| Bsp. Nr. | $R^1$ | $R^2$ | Säureadditionssalz mit | Schmelzpunkt /°C |
|---|---|---|---|---|
| 149 | H | 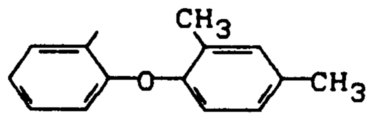 | $HNO_3$ | 193 (Zers.) |
| 150 | H | 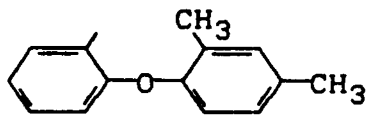 | $H_2SO_4$ | 245 |
| 151 | H | 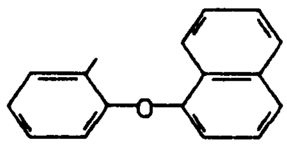 | $CH_3-CH(OH)-COOH$ | 200 (Zers.) |
| 152 | H | 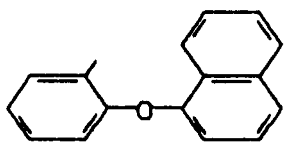 | - | 222 |
| 153 | H | 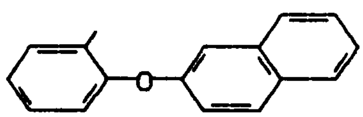 | $CH_3-SO_3H$ | 195 |
| 154 | H | 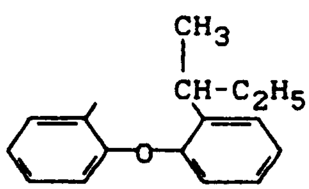 | HCl | 113 |
| 155 | H | 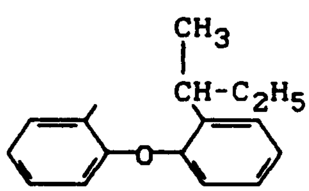 | - | 130 |

| Bsp. Nr. | $R^1$ | $R^2$ | Säureadditionssalz mit | Schmelzpunkt /° C |
|---|---|---|---|---|
| 156 | H | 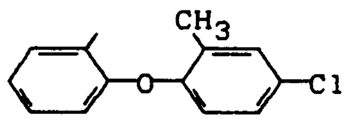 | $HNO_3$ | 130 (Zers.) |
| 157 | H | 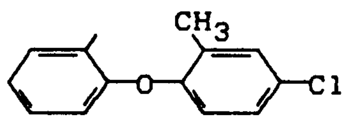 | $CH_3-CH(OH)-COOH$ | 212 |
| 158 | H | 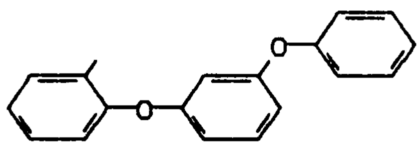 | - | 89 |
| 159 | H | 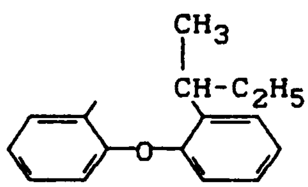 | $CH_3-SO_3H$ | 158 |
| 160 | H | 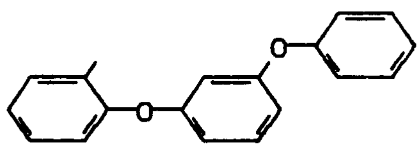 | $CH_3-SO_3H$ | 114 |
| 161 | H | 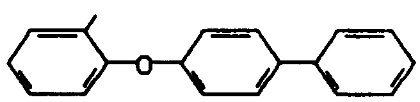 | HCl | 228-229 |
| 162 | H | 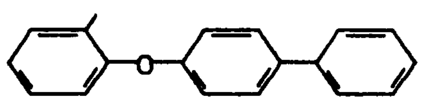 | - | 222 |
| 163 | H | 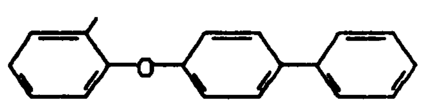 | $CH_3-SO_3H$ | 221 |

| Bsp. Nr. | $R^1$ | $R^2$ | Säureadditionssalz mit | Schmelzpunkt /° C |
|---|---|---|---|---|
| 164 | $CH_3$ | 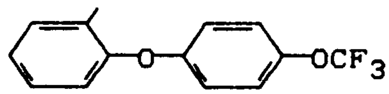 | HCl | 173-174 |
| 165 | $CH_3$ | 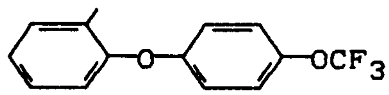 | $CH_3$-$SO_3H$ | |
| 166 | $CH_3$ | 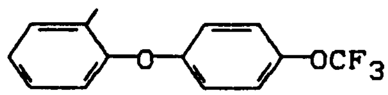 | - | 196 (Zers.) |
| 167 | $CH_3$ | 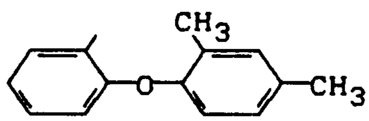 | HCl | 119-120 |
| 169 | $CH_3$ | 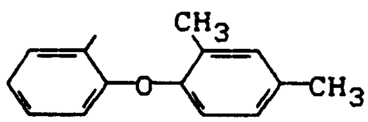 | - | 116 |
| 169 | $CH_3$ | 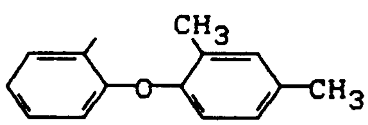 | $CH_3$-$SO_3H$ | 143 (Zers.) |
| 170 | H | 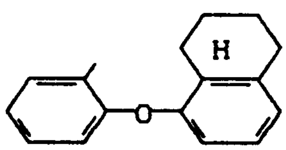 | - | 240 (Zers.) |
| 171 | H | 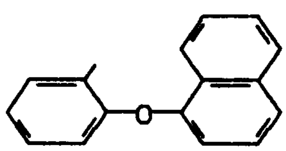 | $H_3PO_4$ | 122 |

| Bsp. Nr. | $R^1$ | $R^2$ | Säureadditionssalz mit | Schmelzpunkt /°C |
|---|---|---|---|---|
| 172 | H | 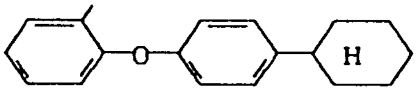 | HCl | 170-171 |
| 173 | $CH_3$ | 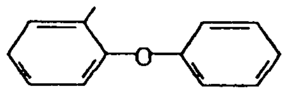 | HBr | 263 |
| 174 | $CH_3$ | 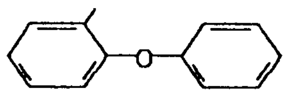 | - | 226 |
| 175 | $CH_3$ | 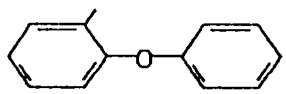 | $CH_3$-$SO_3H$ | 138 |
| 176 | H | 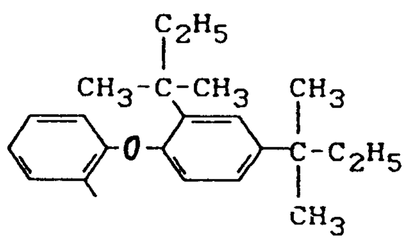 | - | 187 |
| 177 | H | 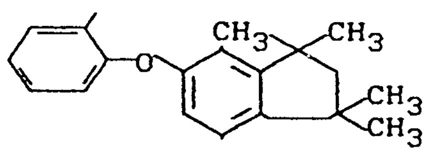 | - | 160 |
| 178 | H | 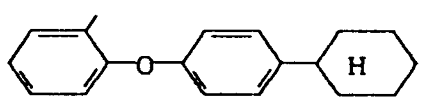 | - | 208-210 |
| 179 | $CH_3$ | 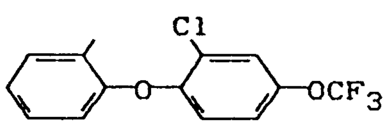 | HCl | 192 |
| 180 | $CH_3$ | 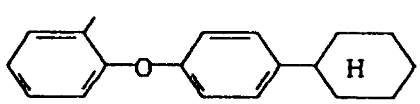 | - | 162 |

| Bsp. Nr. | $R^1$ | $R^2$ | Säureadditionssalz mit | Schmelzpunkt /° C |
|---|---|---|---|---|
| 181 | H | 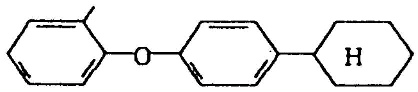 | $CH_3-SO_3H$ | 〉250 |
| 182 | $CH_3$ | 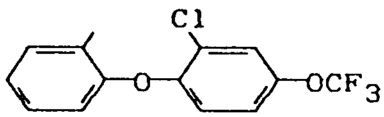 | - | 203 |
| 183 | $CH_3$ | 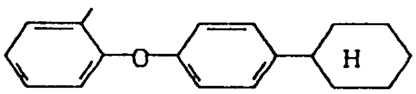 | HCl | 165-166 |
| 184 | $CH_3$ | 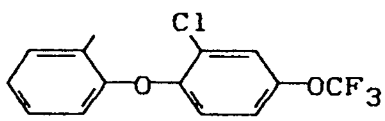 | $CH_3-SO_3H$ | 165 |
| 185 | H | 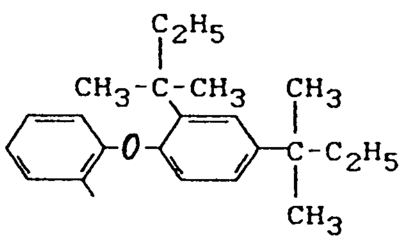 | $CH_3-SO_3H$ | 188 |
| 186 | H | 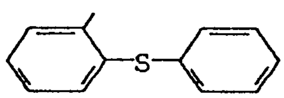 | - | 140 |
| 187 | H | 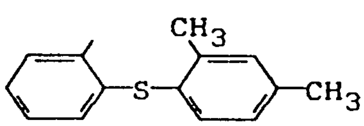 | HBr | 212-214 |
| 188 | $CH_3$ | 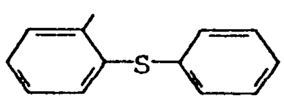 | HBr | 242-244 |

| Bsp. Nr. | $R^1$ | $R^2$ | Säureadditionssalz mit | Schmelzpunkt /° C |
|---|---|---|---|---|
| 189 | H | 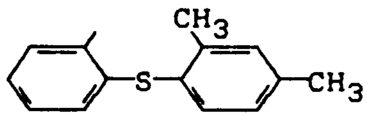 | - | 215 |
| 190 | $CH_3$ | 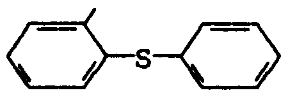 | - | 225 |
| 191 | H | 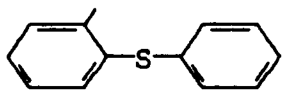 | $H_3PO_4$ | 140 |
| 192 | $CH_3$ | 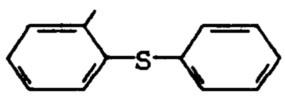 | $H_3PO_4$ | 228 |
| 193 | H | 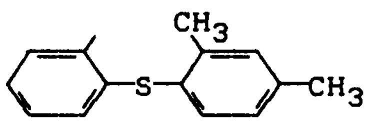 | $H_3PO_4$ | 201 |
| 194 | H | 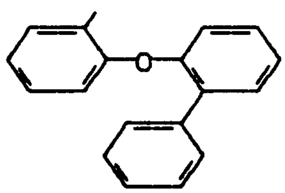 | - | 221 |
| 195 | H | 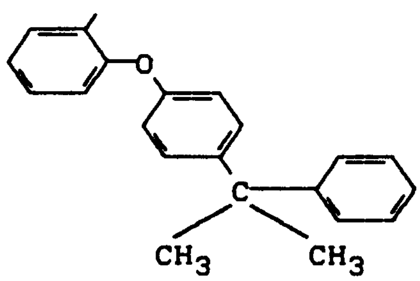 | HCl | 212 (Zers.) |

| Bsp. Nr. | $R^1$ | $R^2$ | Säureadditionssalz mit | Schmelzpunkt /° C |
|---|---|---|---|---|
| 196 | H | 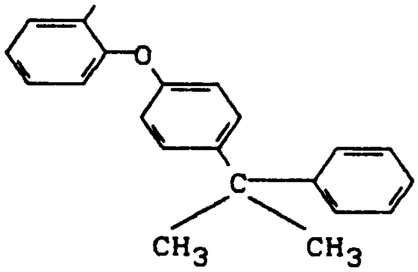 | - | 208 |
| 197 | H | 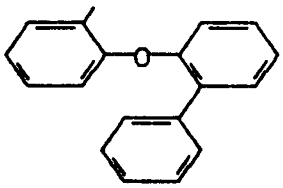 | $H_3PO_4$ | 152 |
| 198 | H | 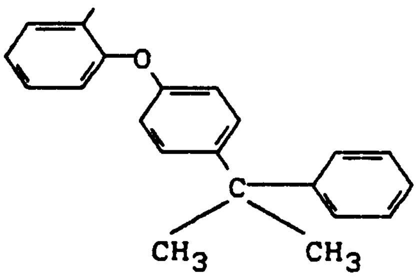 | $H_3PO_4$ | 205 |
| 199 | H | 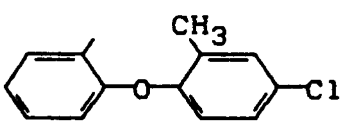 | HCl | >250 |
| 200 | H | 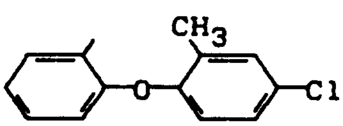 | - | >250 |
| 201 | H | 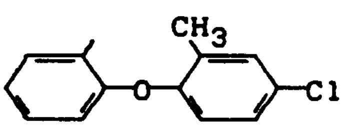 | $H_3PO_4$ | 255 |

| Bsp. Nr. | $R^1$ | $R^2$ | Säureadditionssalz mit | Schmelzpunkt /° C |
|---|---|---|---|---|
| 202 | H | 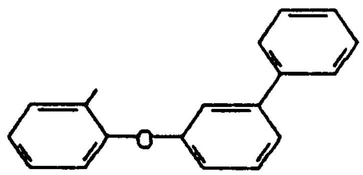 | HCl | 212 |
| 203 | H | 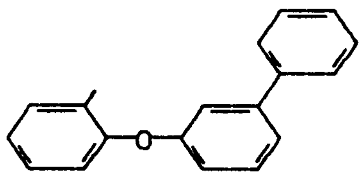 | - | 208 |
| 204 | H | 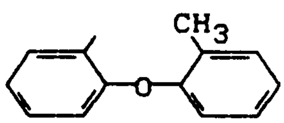 | HCl | 220 |
| 205 | H | 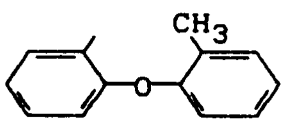 | - | 225-227 |
| 206 | H | 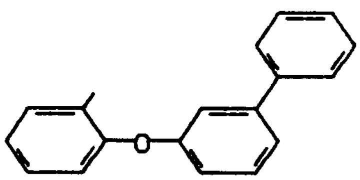 | $H_3PO_4$ | 115 |
| 207 | H | 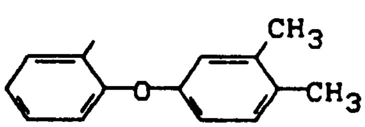 | HCl | 131 |
| 208 | H | 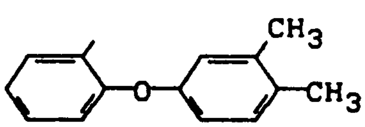 | - | 192 |

| Bsp. Nr. | $R^1$ | $R^2$ | Säureadditionssalz mit | Schmelzpunkt /° C |
|---|---|---|---|---|
| 209 | H | 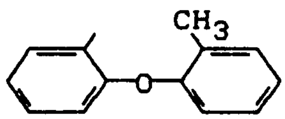 | $H_3PO_4$ | 225 |
| 210 | H | 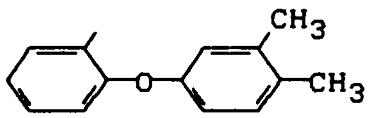 | $H_3PO_4$ | 133 (Zers.) |
| 211 | H | 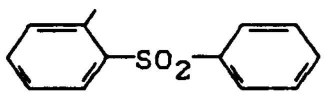 | - | 212-214 |
| 212 | H | 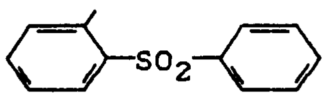 | $H_3PO_4$ | 198 |
| 213 | H | 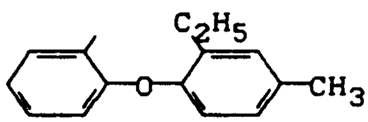 | - | 148 |
| 214 | H | 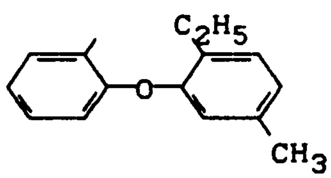 | HCl | 236 |
| 215 | H | 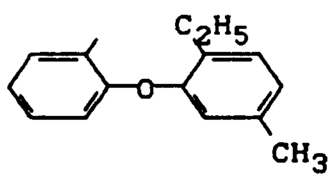 | - | 245 |
| 216 | H | 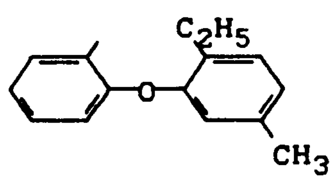 | $H_3PO_4$ | 233 |

| Bsp. Nr. | $R^1$ | $R^2$ | Säureadditionssalz mit | Schmelzpunkt /° C |
|---|---|---|---|---|
| 217 | H | 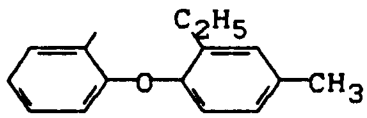 | $H_3PO_4$ | 149 |
| 218 | H | 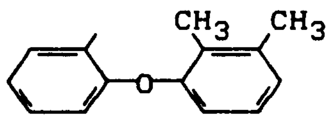 | - | 240 |
| 219 | H | 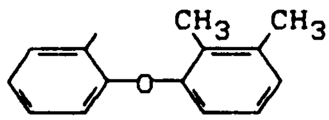 | $H_3PO_4$ | 192 |
| 220 | H | 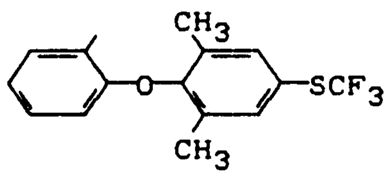 | - | 198 |
| 221 | H | 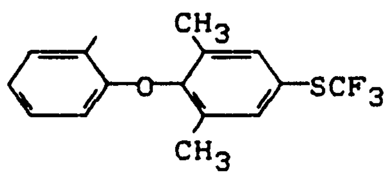 | $H_3PO_4$ | 180 |
| 222 | H | 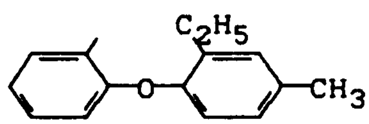 | $H_2SO_4$ | 196 |
| 223 | H | 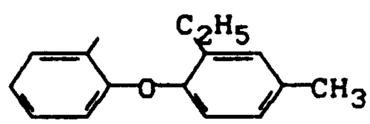 | $HNO_3$ | 155 |
| 224 | H | 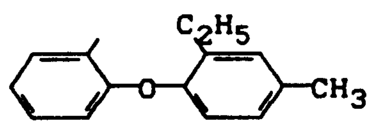 | $CH_3-CH(OH)-COOH$ | Öl |

| Bsp. Nr. | $R^1$ | $R^2$ | Säureadditionssalz mit | Schmelzpunkt /° C |
|---|---|---|---|---|
| 225 | H | 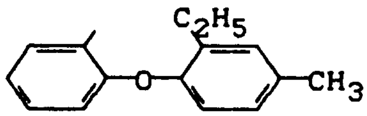 | $HOOC-(CH_2)_2-COOH$ | 70 |
| 226 | H | 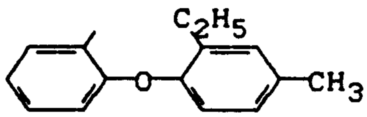 | OH<br>\|<br>$HOOC-CH_2-C-CH_2-COOH$<br>\|<br>COOH | 83 |
| 227 | H | 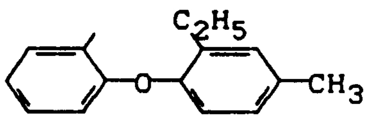 | $CH_3-COOH$ | 56 |
| 228 | H | 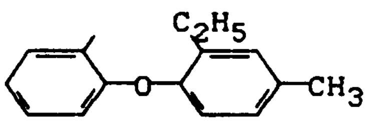 | $HOOC-CH=CH-COOH$ (cis) | 153 |
| 229 | H | 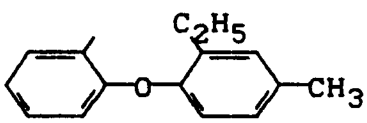 | $HOOC-COOH$ | Öl |
| 230 | H | 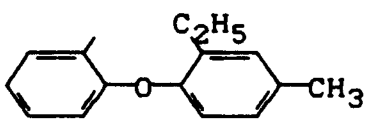 | $HOOC-(CH_2)_3-COOH$ | 198 |
| 231 | H | 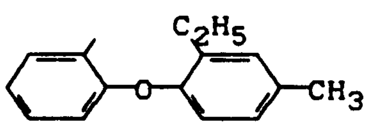 | $HOOC-CH_2-CH-COOH$<br>\|<br>OH | Öl |
| 232 | H | 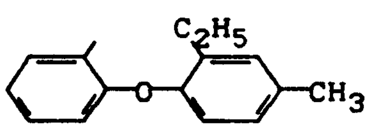 | $HOOC-CH=CH-COOH$ (trans) | 182 |

| Bsp. Nr. | $R^1$ | $R^2$ | Säureadditionssalz mit | Schmelzpunkt /° C |
|---|---|---|---|---|
| 233 | H | 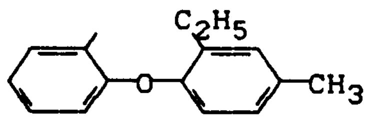 | $HOOC-(CH_2)_4-COOH$ | Öl |
| 234 | H | 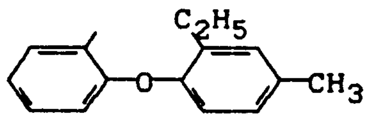 | $HOOC-(CH_2)_7-CH$<br>‖<br>$CH_3-(CH_2)_7-CH$ | Öl |
| 235 | H | 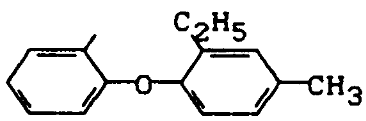 | 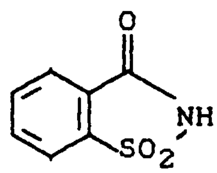 | 218 |
| 236 | H | 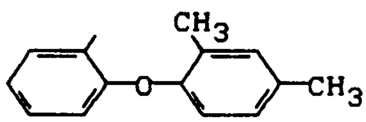 | HOOC-CH<br>‖<br>HOOC-CH (cis) | 186 (Zers.) |
| 237 | H | 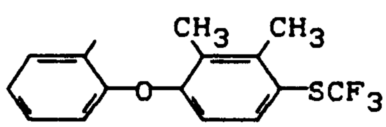 | - | 190 |
| 238 | H | 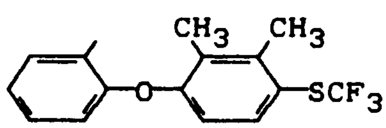 | $H_3PO_4$ | 199 (Zers.) |
| 239 | H | 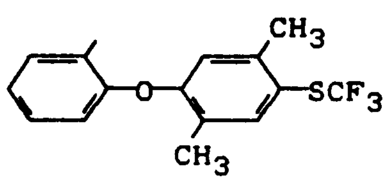 | - | 195 |
| 240 | H | 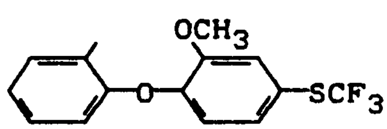 | HCl | 171 |

| Bsp. Nr. | $R^1$ | $R^2$ | Säureadditionssalz mit | Schmelzpunkt /° C |
|---|---|---|---|---|
| 241 | H | 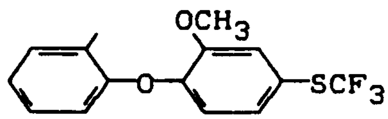 | - | 164 |
| 242 | H | 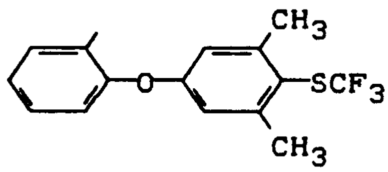 | $H_3PO_4$ | 212 |
| 243 | H | 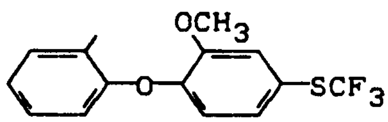 | $H_3PO_4$ | 190 |
| 244 | H | 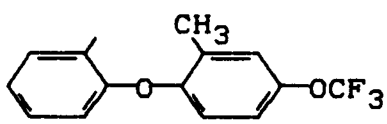 | HCl | 178 |
| 245 | H | 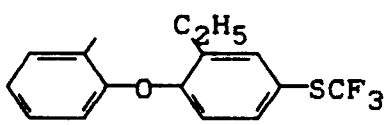 | - | 162 |
| 246 | H | 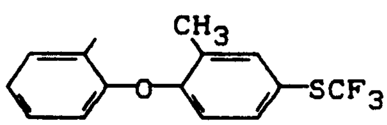 | HCl | 225-226 |
| 247 | H | 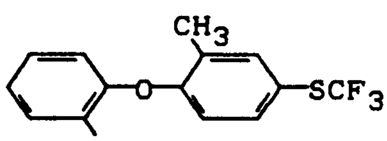 | - | 233 |
| 248 | H | 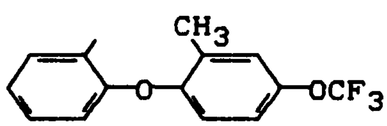 | - | 201 |

| Bsp. Nr. | $R^1$ | $R^2$ | Säureaddi-tionssalz mit | Schmelz-punkt /° C |
|---|---|---|---|---|
| 249 | H | 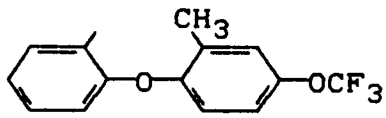 | $H_3PO_4$ | 190 |
| 250 | H | 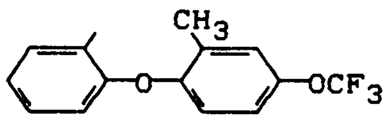 | $CH_3$-CH-COOH<br>\|<br>OH | 112 |
| 251 | H | 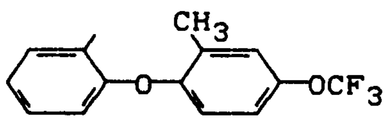 | HOOC-$(CH_2)_2$-COOH | 132 |
| 252 | H | 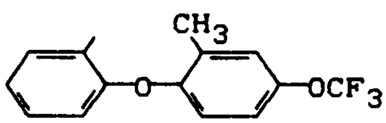 | OH<br>\|<br>HOOC-$CH_2$-C-$CH_2$-COOH<br>\|<br>COOH | 143 |
| 253 | H | 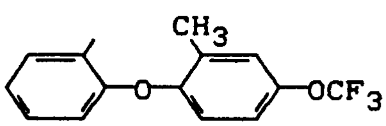 | HOOC-CH<br>‖<br>HOOC-CH (cis) | 180 (Zers.) |
| 254 | H | 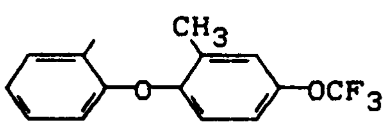 | HOOC-COOH | 98 |
| 255 | H | 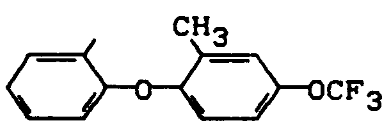 | HOOC-$CH_2$-CH-COOH<br>\|<br>OH | 138 |
| 256 | H | 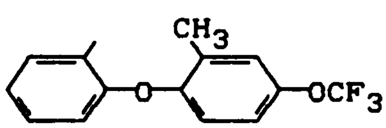 | CH-COOH<br>‖<br>HOOC-CH<br>(trans) | 112 |

| Bsp. Nr. | $R^1$ | $R^2$ | Säureaddi-tionssalz mit | Schmelz-punkt /° C |
|---|---|---|---|---|
| 257 | H | 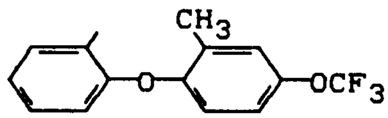 | $HOOC-(CH_2)_4-COOH$ | 107 |
| 258 | H | 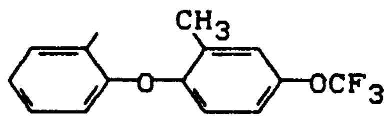 | 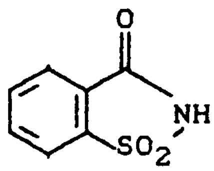 | 159 |
| 259 | H | 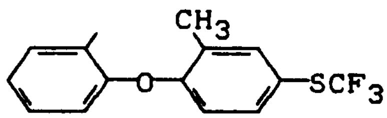 | $H_3PO_4$ | 110 (Zers.) |
| 260 | H | 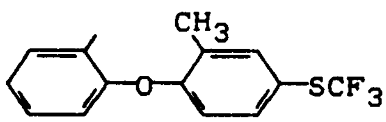 | $CH_3-CH-COOH$<br>\|<br>OH | 138 |
| 261 | H | 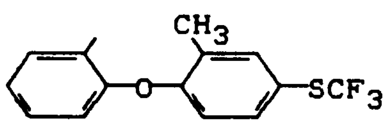 | $CH_2-COOH$<br>\|<br>$HOOC-CH_2$ | 151 |
| 262 | H | 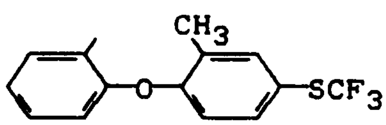 | OH<br>\|<br>$HOOC-CH_2-C-CH_2-COOH$<br>\|<br>COOH | 189 |
| 263 | H | 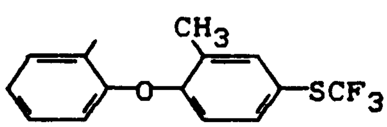 | HOOC-CH=CH-COOH (cis) | 211 |
| 264 | H | 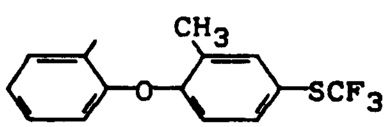 | HOOC-COOH | 238 |

| Bsp. Nr. | $R^1$ | $R^2$ | Säureadditionssalz mit | Schmelzpunkt /° C |
|---|---|---|---|---|
| 265 | H | 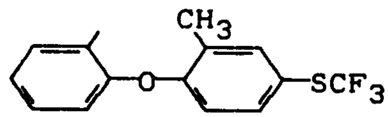 | $HOOC-CH_2-CH(OH)-COOH$ | 220 (Zers.) |
| 266 | H | 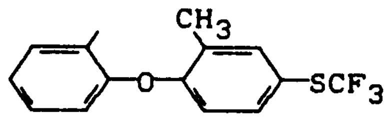 | HOOC-CH=CH-COOH (trans) | 189 |
| 267 | H | 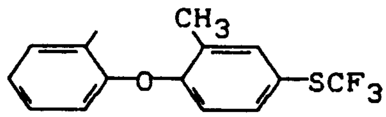 | $HOOC-(CH_2)_4-COOH$ | 142 |
| 268 | H | 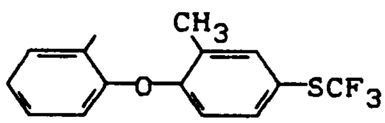 | 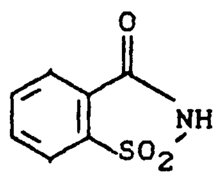 | 60 |
| 269 | H | 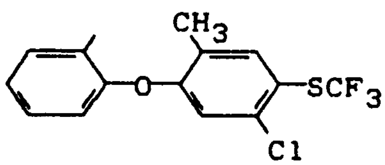 | HCl | 232 |
| 270 | H | 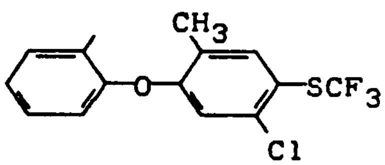 | - | 230-231 |
| 271 | H | 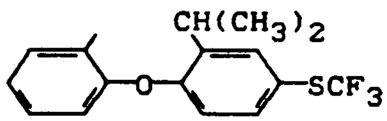 | HCl | 181 |
| 272 | H | 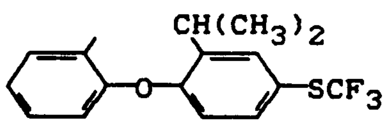 | - | 174-175 |

| Bsp. Nr. | $R^1$ | $R^2$ | Säureadditionssalz mit | Schmelzpunkt /° C |
|---|---|---|---|---|
| 273 | H | 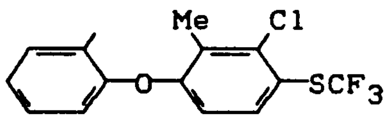 | HCl | 211 |
| 274 | H | 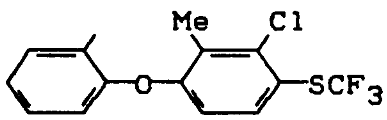 | - | 204-205 |
| 275 | H | 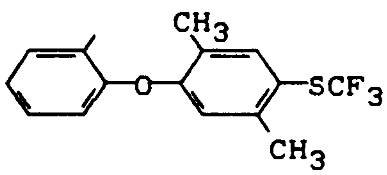 | HCl | Öl |
| 276 | H | 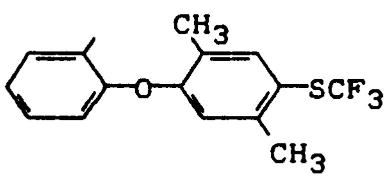 | - | 250 |
| 277 | H | 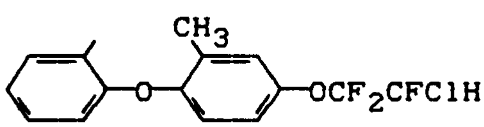 | HCl | 112 (Zers.) |
| 278 | H | 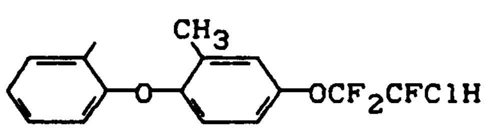 | - | 114 |
| 279 | H | 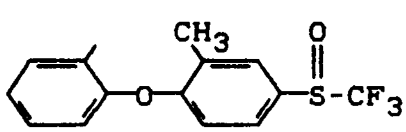 | HBr | 247-8 |
| 280 | H | 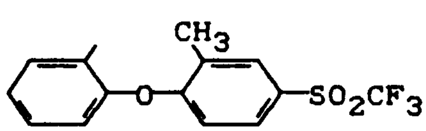 | HBr | > 250 |

| Bsp. Nr. | $R^1$ | $R^2$ | Säureadditionssalz mit | Schmelzpunkt /°C |
|---|---|---|---|---|
| 281 | H | 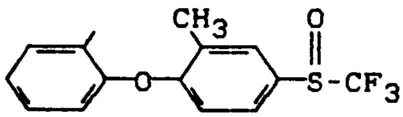 | - | 245 |
| 282 | H | 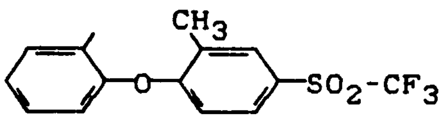 | - | 〉 250 |
| 283 | H | 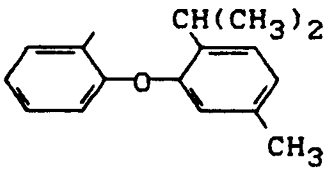 | - | 190 |
| 284 | H | 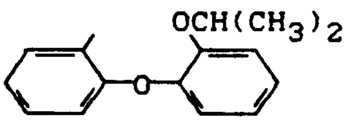 | - | 135 |
| 285 | H | 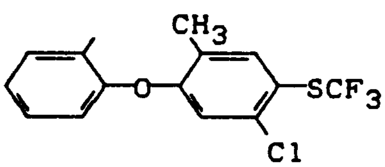 | 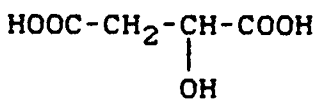 | 179 (Zers.) |
| 286 | H | 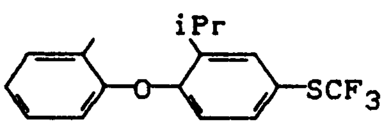 | 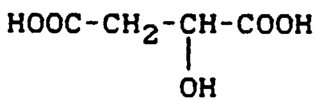 | 68 |
| 287 | H | 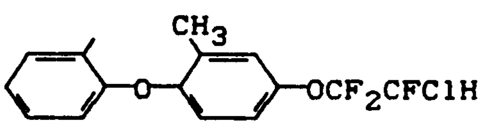 | 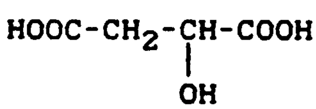 | 34 |

| Bsp. Nr. | $R^1$ | $R^2$ | Säureadditionssalz mit | Schmelzpunkt /° C |
|---|---|---|---|---|
| 288 | H | 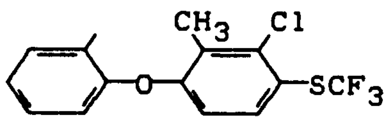 | $HOOC-CH_2-\underset{OH}{CH}-COOH$ | 62 |
| 289 | H | 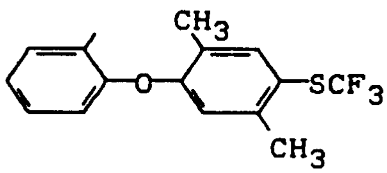 | $HOOC-CH_2-\underset{OH}{CH}-COOH$ | 130 |
| 290 | H | 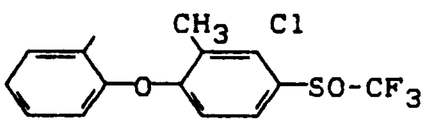 | $HOOC-CH_2-\underset{OH}{CH}-COOH$ | 142 |
| 291 | H | 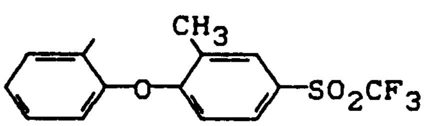 | $HOOC-CH_2-\underset{OH}{CH}-COOH$ | > 250° C |
| 292 | H | 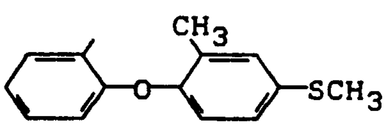 | HCl | Öl |
| 293 | H | 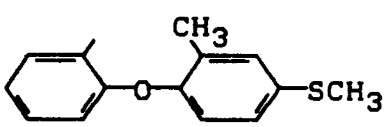 | - | Öl |
| 294 | H | 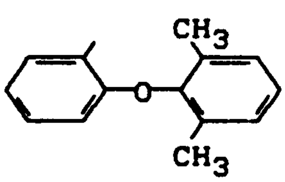 | HCl | 225 |

| Bsp. Nr. | $R^1$ | $R^2$ | Säureaddi-tionssalz mit | Schmelz-punkt /° C |
|---|---|---|---|---|
| 295 | H | 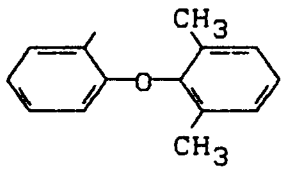 | - | 236 |
| 296 | H | 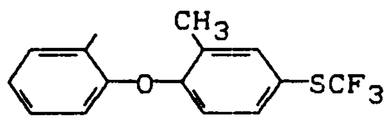 | HOOC-$CH_2$-CH-COOH<br>\|<br>OH | 148 |
| 297 | H | 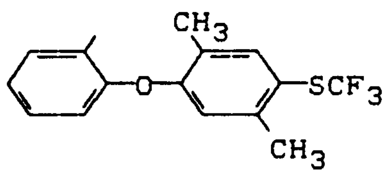 | $H_3PO_4$ | 122 (Zers.) |
| 298 | H | 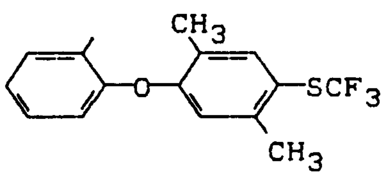 | HOOC-COOH | 146 |
| 299 | H | 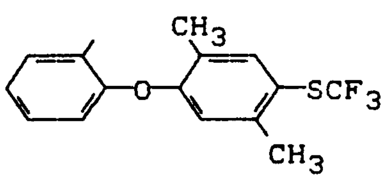 | HOOC-CH<br>‖<br>CH-COOH<br>(trans) | 196 |
| 300 | H | 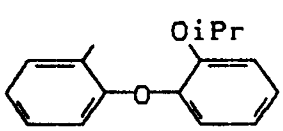 | HOOC-$CH_2$-CH-COOH<br>\|<br>OH | 236 |
| 301 | H | 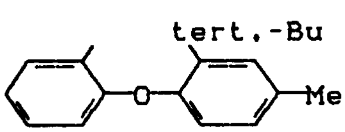 | HOOC-$CH_2$-CH-COOH<br>\|<br>OH | 55 |

| Bsp. Nr. | $R^1$ | $R^2$ | Säureadditionssalz mit | Schmelzpunkt /° C |
|---|---|---|---|---|
| 302 | H | 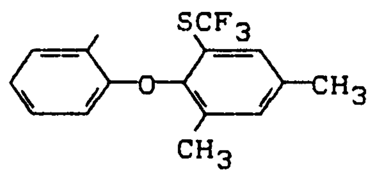 | HCl | 109 |
| 303 | H | 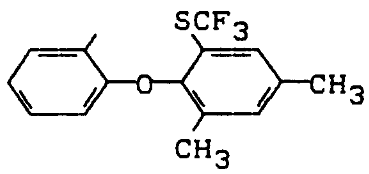 | - | 117 |
| 304 | H | 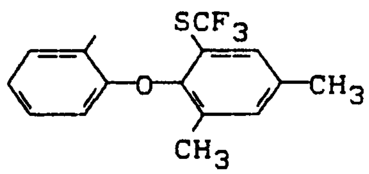 | $HOOC-CH_2-CH-COOH$<br>\|<br>OH | 95 |
| 305 | H | 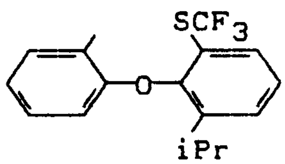 | HCl | 98 |
| 306 | H | 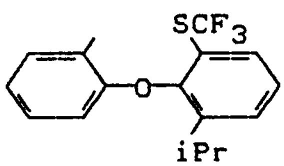 | - | 105 |
| 307 | H | 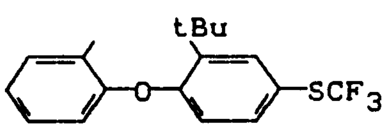 | - | Öl |
| 308 | H | 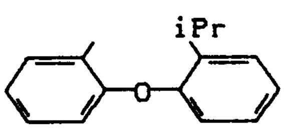 | $HOOC-CH_2-CH-COOH$<br>\|<br>OH | Öl |
| 309 | H | 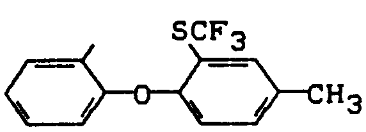 | HCl | 201 |

| Bsp. Nr. | $R^1$ | $R^2$ | Säureadditionssalz mit | Schmelzpunkt /° C |
|---|---|---|---|---|
| 310 | H | 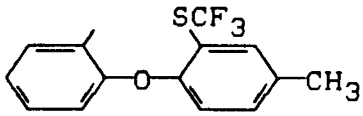 | | |
| 311 | H | 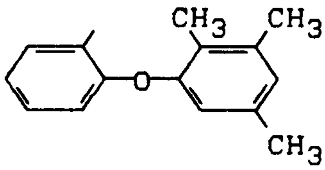 | - | 149 |
| 312 | H | 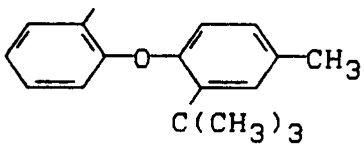 | $H_3PO_4$ | 229 |
| 313 | H | 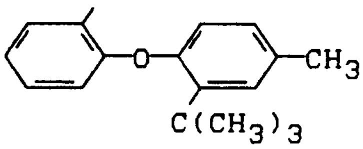 | 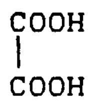 | 198 |
| 314 | H | 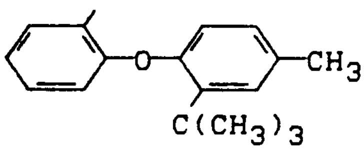 | 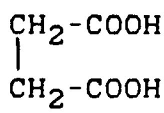 | 97 |
| 315 | H | 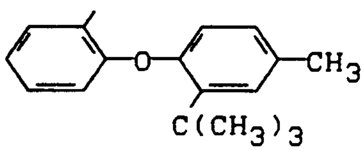 | 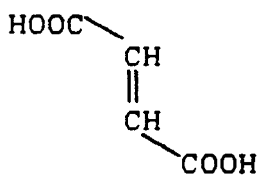 (trans) | 192 |
| 316 | H | 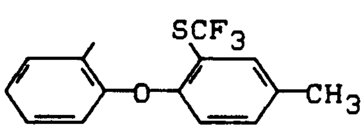 | $H_3PO_4$ | 132 |
| 317 | H | 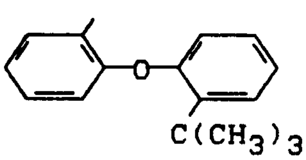 | $H_3PO_4$ | 220 |

| Bsp. Nr. | $R^1$ | $R^2$ | Säureadditionssalz mit | Schmelzpunkt /° C |
|---|---|---|---|---|
| 318 | H | 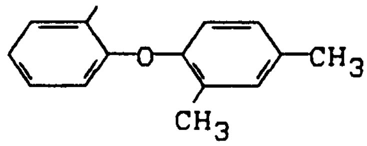 | 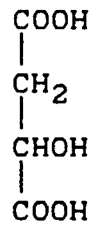 | 126 |
| 319 | H | 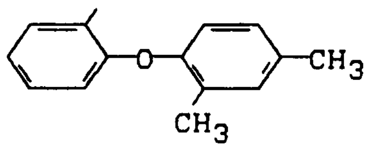 | 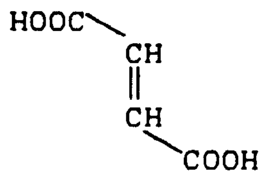 (trans) | 175 |
| 320 | H | 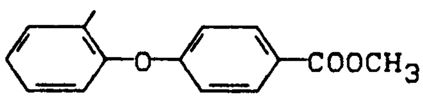 | - | 125 |
| 321 | H | 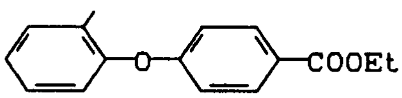 | - | 173 |
| 322 | H | 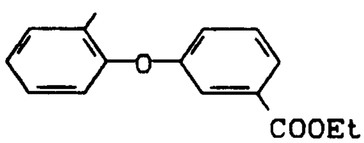 | HCl | 199-200 |
| 323 | H | 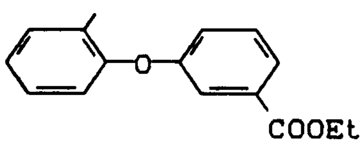 | - | 193 |

Beispiel 324

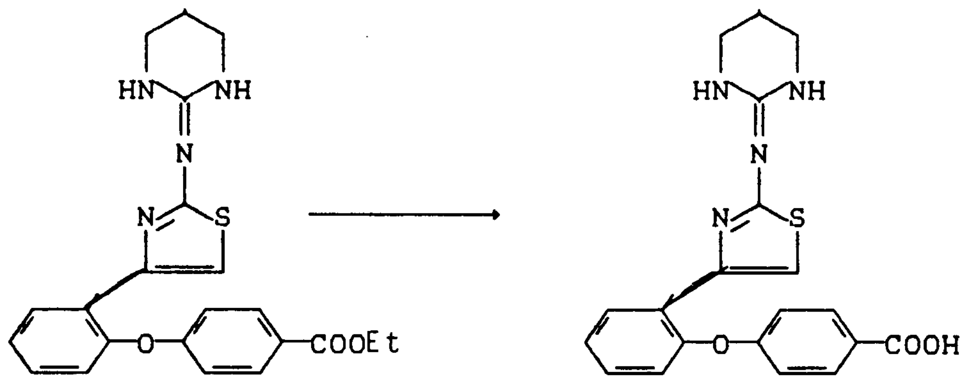

Zu 6,3 g (0,0155 Mol) des Carbonsäureesters aus Beispiel 321, suspendiert in 150 ml EtOH, wird eine Lösung von 3,45 g KOH in 40 ml EtOH getropft. Man läßt 2 h bei Raumtemperatur nachrühren, saugt ab, engt das Filtrat i.Vak. ein und chromatografiert den Rückstand mit Methylenchlorid/Methanol 1:1 als Laufmittel an Kieselgel.
Man erhält an Produkt 4,74 g (78 % der Theorie) mit einem Schmelzpunkt von über 250 °C.

Beispiel 325

Analog zu Beispiel 323 erhält man durch Hydrolyse des Esters aus Beispiel 324 die Säure

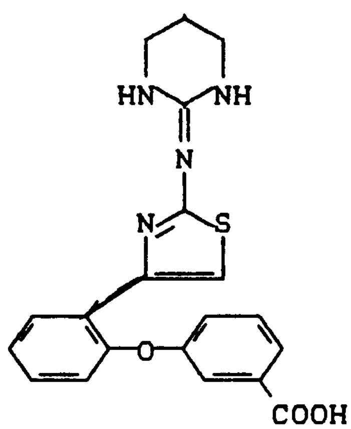

Schmelzpunkt > 250 °C

Herstellung der Ausgangsverbindungen

Beispiel (III-1):

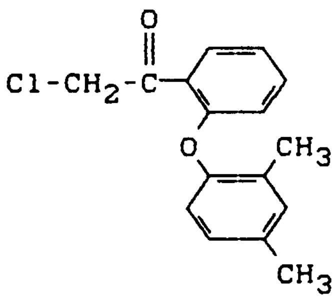

Zu 24 g (0,1 Mol) 2-(2,4-Dimethylphenoxy)-acetophenon in 100 ml Dichlormethan gibt man 14,85 g (0,11 Mol) Sulfurylchlorid, rührt bis zum Ende der Chlorwasserstoffentwicklung (ca. 2 Stunden) bei Raumtemperatur, wäscht nacheinander mit jeweils 300 ml Wasser und 300 ml gesättigter Natriumhydrogencarbonatlösung, trocknet über Magnesiumsulfat und engt im Vakuum ein.

Man erhält 18,9 g (69 % der Theorie) an ω-Chlor-2-(2,4-dimethylphenoxy)-acetophenon als Öl, welches ohne zusätzliche Reinigung weiter umgesetzt wird.
$^1$H-NMR ($CDCl_3$/Tetramethylsilan): $\delta$ = 4,92 ppm.

Beispiel (III-2)

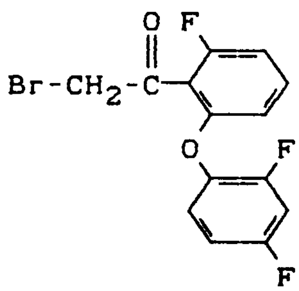

Zu 13,3 g (0,05 Mol) 2-(2,4-Difluorphenoxy)-6-fluoracetophenon in 100 ml Eisessig gibt man bei Raumtemperatur unter Rühren nacheinander 1 ml konzentrierte Salzsäure und dann tropfenweise im Verlauf von ca. 2 Stunden 9 g (0,056 Mol) Brom in 20 ml Eisessig. Nach beendeter Zugabe rührt man weitere 30 Minuten bei Raumtemperatur nach, gibt dann die Mischung in 450 ml Eiswasser, extrahiert mehrfach mit Dichlormethan, wäscht die vereinigten organischen Phasen mit Wasser, trocknet über Magnesiumsulfat und entfernt das Lösungmittel im Vakuum.

Man erhält 12,2 g (70 % der Theorie) an ω-Brom-2-(2,4-difluorphenoxy)-6-fluoracetophenon als Öl, welches ohne zusätzliche Reinigung weiter umgesetzt wird.
$^1$H-NMR ($CDCl_3$/Tetramethylsilan): $\delta$ = 4,51 ppm.

Beispiel III-3

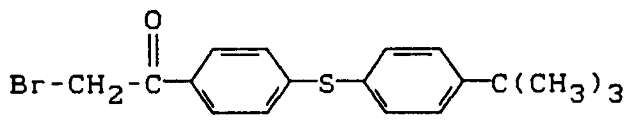

Zu 14,2 g (0,05 Mol) 4-(4-t-Butylphenylthio)-acetophenon (vgl. z.B. US 45 36 517) in 50 ml Dichlormethan gibt man bei Raumtemperatur tropfenweise unter Rühren 8 g (0,05 Mol) Brom in 30 ml Dichlormethan, rührt nach beendeter Zugabe weitere 2 Stunden bei Raumtemperatur, gießt dann die Reaktionsmischung in 400 ml Wasser, extrahiert mehrfach mit Dichlormethan, wäscht die vereinigten organischen Phasen mit gesättigter wässriger Natriumhydrogencarbonatlösung, trocknet über Magnesiumsulfat und entfernt das Lösungsmittel im Vakuum.

Man erhält 17,0 g (94 % der Theorie) an ω-Brom-4-(4-t-butylphenylthio)-acetophenon vom Schmelzpunkt 81 °C.

In entsprechender Weise und gemäß den allgemeinen Angaben zur Herstellung erhält man die folgenden ω-Halogenacetophenoneder Formel (III):

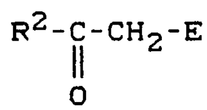

(III)

| Bsp. Nr. | $R^2$ | E | $^1H$-NMR*): |
|---|---|---|---|
| III-4 | 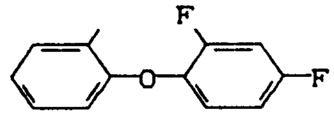 | Cl | 4,86 |
| III-5 | 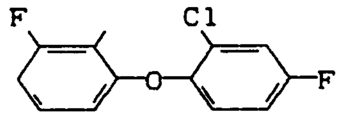 | Cl | 4,84 |
| III-6 | 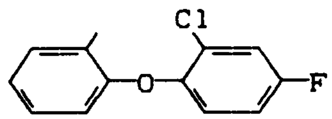 | Cl | 4,84 |
| III-7 | 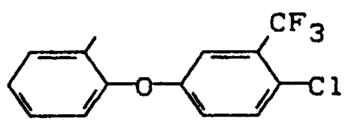 | Cl | 4,79 |
| III-8 | 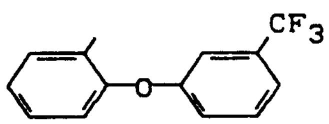 | Cl | 4,81 |
| III-9 | 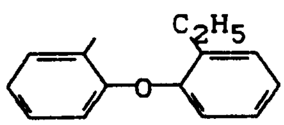 | Cl | 4,89 |

| Bsp. Nr. | $R^2$ | E | $^1$H-NMR*): |
|---|---|---|---|
| III-10 | 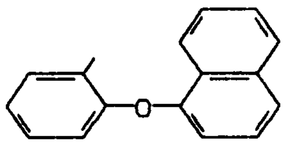 | Cl | 4,98 |
| III-11 | 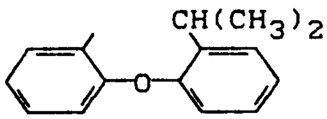 | Cl | 4,79 |
| III-12 | 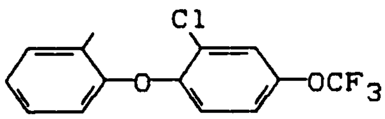 | Cl | 4,76 |
| III-13 | 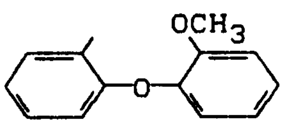 | Cl | 4,80 |
| III-14 | 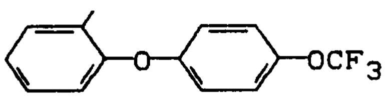 | Cl | 4,79 |
| III-15 | 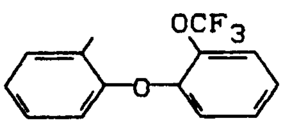 | Cl | 4,77 |
| III-16 | 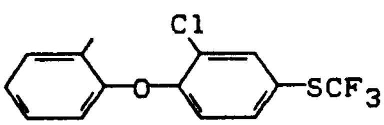 | Cl | 4,75 |
| III-17 | 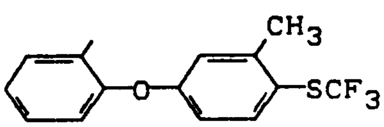 | Cl | 4,78 |

| Bsp.Nr. | $R^2$ | E | $^1H$-NMR*): |
|---|---|---|---|
| III-18 | 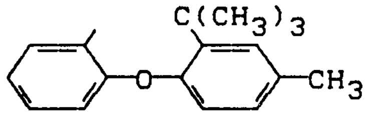 | Cl | 4,82 |
| III-19 | 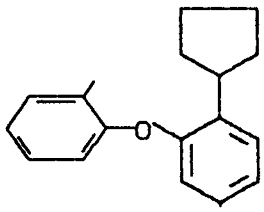 | Cl | 4,86 |
| III-20 | 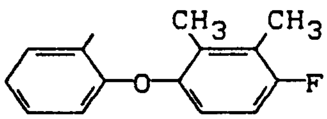 | Cl | 4,83 |
| III-21 | 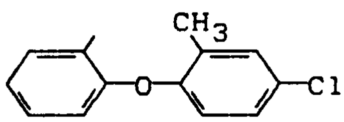 | Cl | 4,81 |
| III-22 | 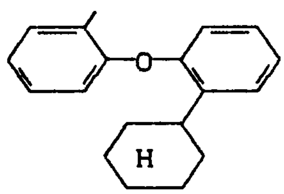 | Cl | 4,84 |
| III-23 | 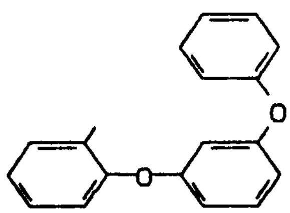 | Cl | 4,91 |
| III-24 | 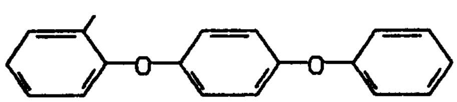 | Cl | 4,86 |

| Bsp. Nr. | $R^2$ | E | $^1$H-NMR*): |
|---|---|---|---|
| III-25 | 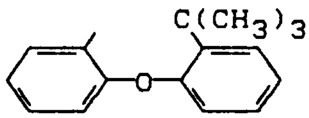 | Cl | 4,79 |
| III-26 | 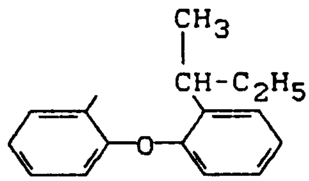 | Cl | 4,88 |
| III-27 | 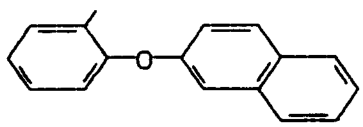 | Cl | 4,97 |
| III-28 | 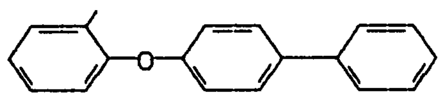 | Cl | 4,84 |
| III-29 | 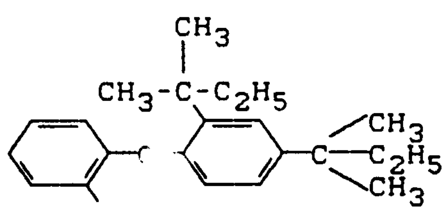 | Cl | 4,81 |
| III-30 | 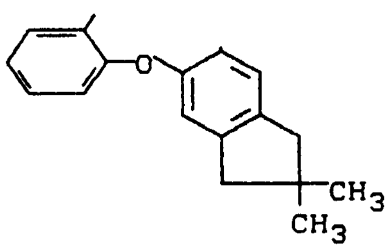 | Cl | 4,87 |
| III-31 | 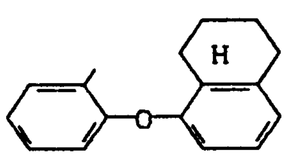 | Cl | 4,85 |

| Bsp. Nr. | $R^2$ | E | $^1$H-NMR*): |
|---|---|---|---|
| III-32 | 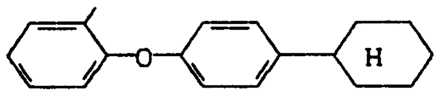 | Cl | 4,79 |
| III-33 | 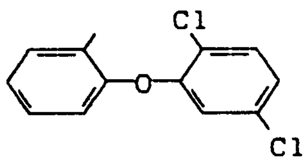 | Cl | 4,82 |
| III-34 | 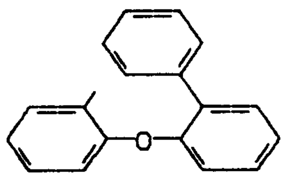 | Cl | 4,85 |
| III-35 | 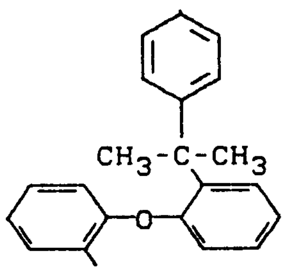 | Cl | 4,81 |
| III-36 | 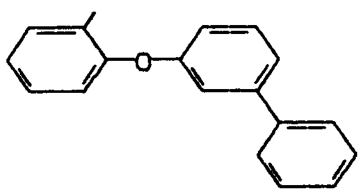 | Cl | 4,85 |
| III-37 | 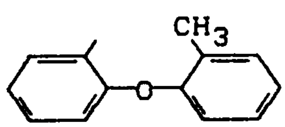 | Cl | 4,90 |
| III-38 | 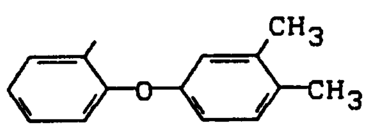 | Cl | 4,84 |

| Bsp. Nr. | $R^2$ | E | $^1$H-NMR*): |
|---|---|---|---|
| III-39 | 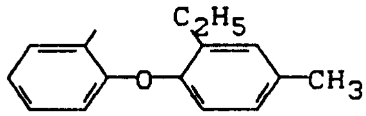 | Cl | 4,88 |
| III-40 | 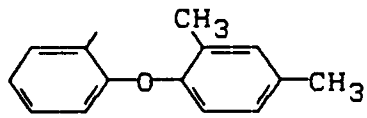 | Cl | 4,90 |
| III-41 | 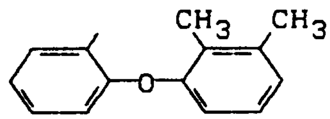 | Cl | 4,90 |
| III-42 | 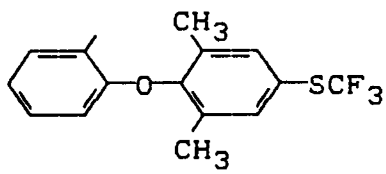 | Cl | 4,95 |
| III-43 | 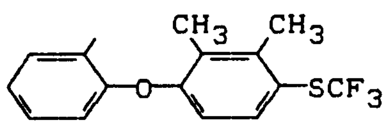 | Cl | 4,81 |
| III-44 | 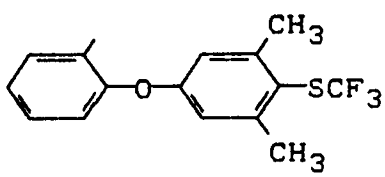 | Cl | 4,75 |
| III-45 | 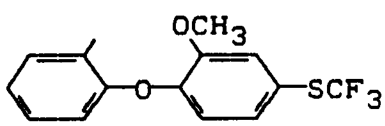 | Cl | 4,91 |
| III-46 | 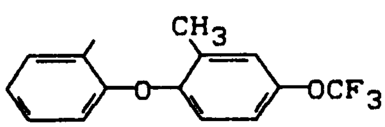 | Cl | 4,82 |

| Bsp. Nr. | $R^2$ | E | $^1H$-NMR*): |
|---|---|---|---|
| III-47 | 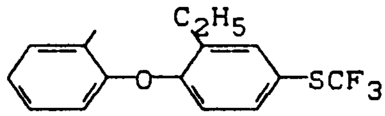 | Cl | 4,79 |
| III-48 | 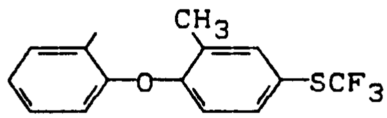 | Cl | 4,78 |
| III-49 | 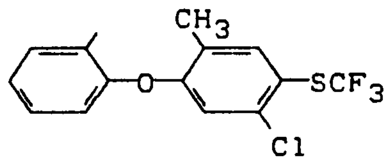 | Cl | 4,72 |
| III-50 | 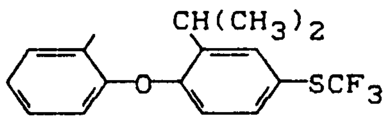 | Cl | 4,80 |
| III-51 | 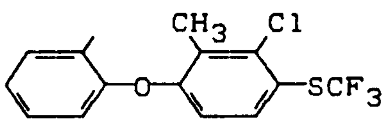 | Cl | 4,75 |
| III-52 | 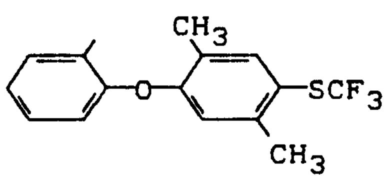 | Cl | 4,81 |
| III-53 | 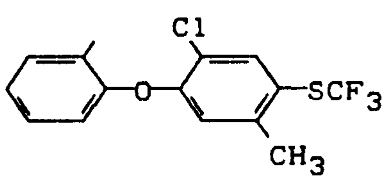 | Cl | 4,76 |
| III-54 | 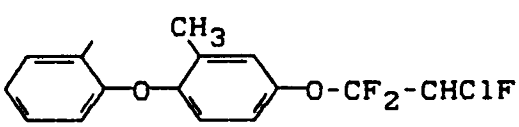 | Cl | 4,78 |

| Bsp. Nr. | $R^2$ | E | $^1H$-NMR*): |
|---|---|---|---|
| III-55 | 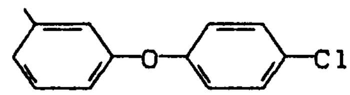 | Br | 4,73 |
| III-56 | 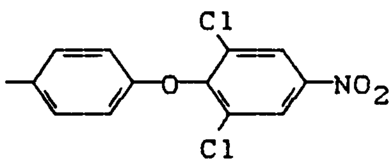 | Br | Fp 131-132° C |
| III-57 | 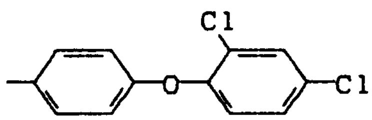 | Br | 4,68 |
| III-58 | 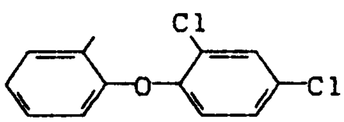 | Br | 4,79 |
| III-59 | 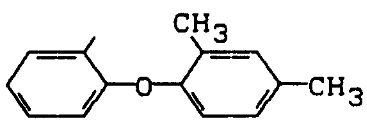 | Br | 4,85 |
| III-60 | 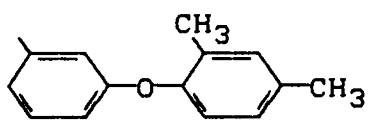 | Br | 4,82 |
| III-61 | 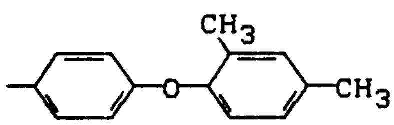 | Br | 4,80 |
| III-62 | 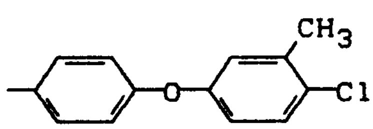 | Br | 4,82 |

| Bsp. Nr. | $R^2$ | E | $^{1}$H-NMR*): |
|---|---|---|---|
| III-63 | 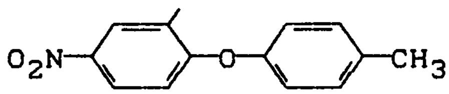 | Br | 4,79 |
| III-64 | 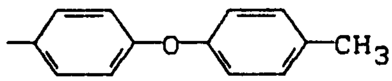 | Br | 4,82 |
| III-65 | 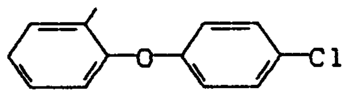 | Br | 4,79 |
| III-66 | 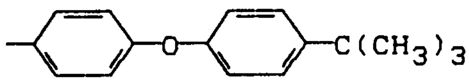 | Br | 4,81 |
| III-67 | 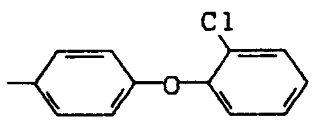 | Br | 4,79 |
| III-68 | 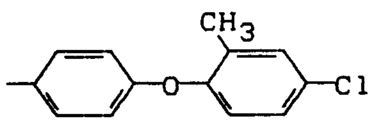 | Br | 4,81 |
| III-69 | 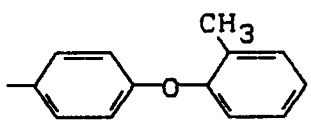 | Br | 4,83 |
| III-70 | 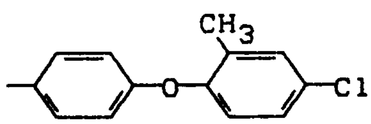 | Br | 4,85 |
| III-71 | 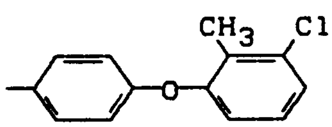 | Br | 4,81 |

| Bsp. Nr. | $R^2$ | E | $^1H$-NMR*): |
|---|---|---|---|
| III-72 | 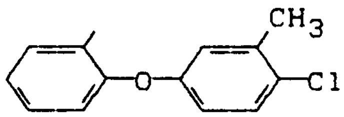 | Br | 4,83 |
| III-73 | 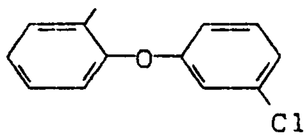 | Br | 4,79 |
| III-74 | 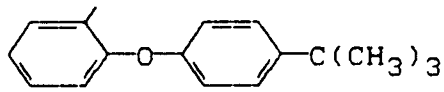 | Br | 4,82 |
| III-75 | 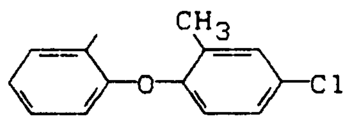 | Br | 4,81 |
| III-76 | 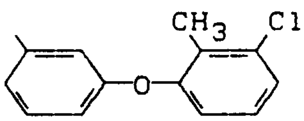 | Br | 4,83 |
| III-77 | 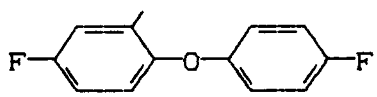 | Br | Fp 77-78° C |
| III-78 | 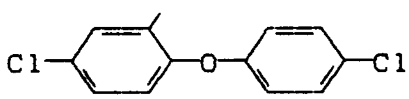 | Br | 4,77 |
| III-79 | 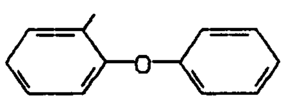 | Br | 4,88 |
| III-80 | 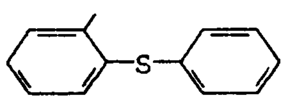 | Br | Fp 58° C |

| Bsp. Nr. | $R^2$ | E | $^1$H-NMR*): |
|---|---|---|---|
| III-81 | 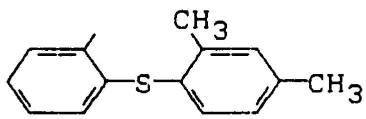 | Br | Fp 85-86° C |
| III-82 | 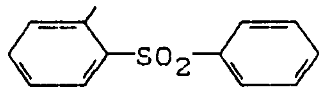 | Br | 4,58 |
| III-83 | 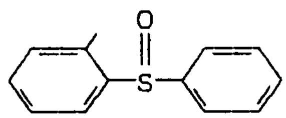 | Br | 4,50 |
| III-84 | 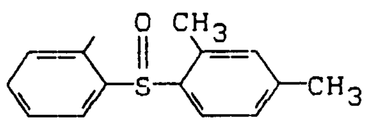 | Br | 4,61 |
| III-85 | 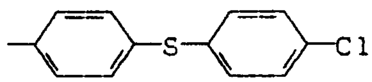 | Br | Fp 80 °C |
| III-86 | 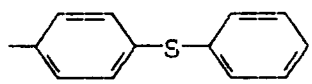 | Br | 4,60 |
| III-87 | 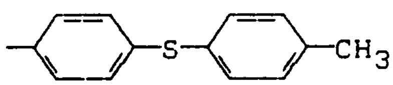 | Br | 4,61 |
| III-88 | 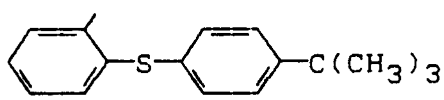 | Br | 4,64 |
| III-89 | 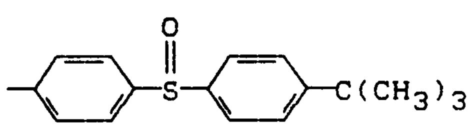 | Br | Fp 150° C |
| III-90 | 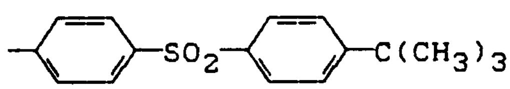 | Br | Fp 162 °C |

| Bsp. Nr. | $R^2$ | E | $^1$H-NMR*): |
|---|---|---|---|
| III-91 | 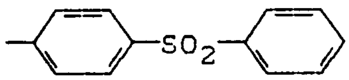 | Br | Fp 118-119 °C |
| III-92 | 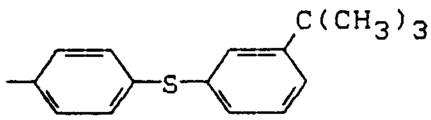 | Br | 4,62 |
| III-93 | 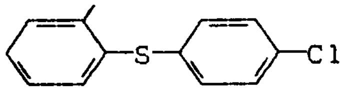 | Br | Fp 89 °C |
| III-94 | 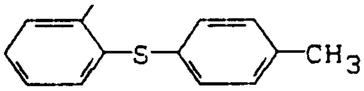 | Br | Fp 88-89 °C |
| III-95 | 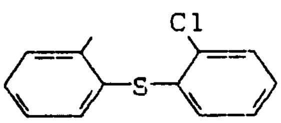 | Br | 4,61 |
| III-96 | 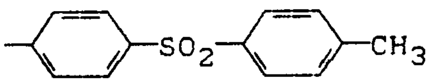 | Br | Fp 120 °C |
| III-97 | 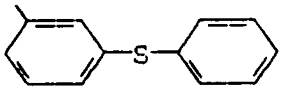 | Br | 4,60 |
| III-98 | 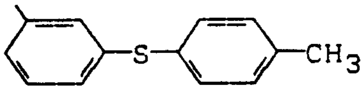 | Br | 4,59 |

| Bsp. Nr. | $R^2$ | E | $^1H$-NMR*): |
|---|---|---|---|
| III-99 | 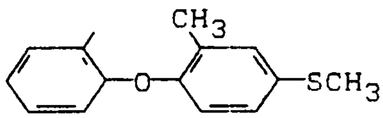 | Cl | 4.89 |
| III-100 | 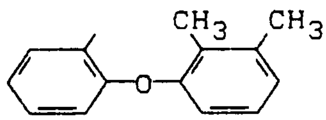 | Cl | 4.88 |
| III-101 | 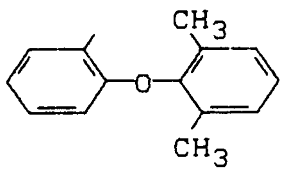 | Cl | Fp. 117-118° C |
| III-102 | 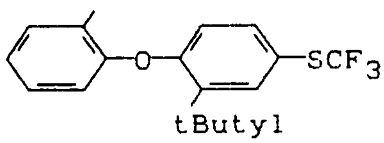 | Cl | 4.87 |
| III-103 | 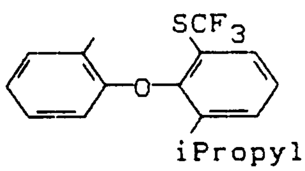 | Cl | 4.81 |
| III-104 | 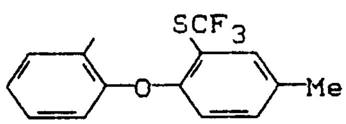 | Cl | 4.83 |
| III-105 | 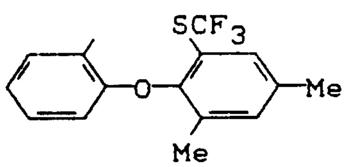 | Cl | 4.87 |

| Bsp. Nr. | $R^2$ | E | $^1$H-NMR*): |
|---|---|---|---|
| III-106 | 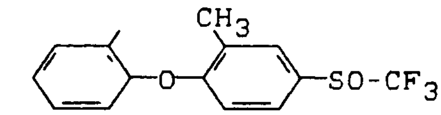 | Br | 4.58 |
| III-107 | 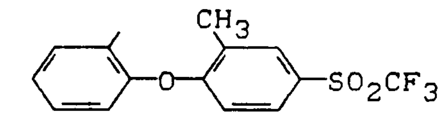 | Br | 4.59 |
| III-108 | 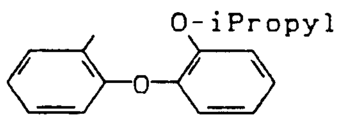 | Cl | 4.86 |
| III-109 | 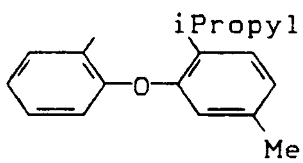 | Cl | 4.89 |
| III-110 | 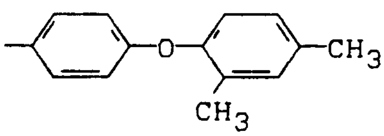 | Cl | 4.90 |
| III-111 | 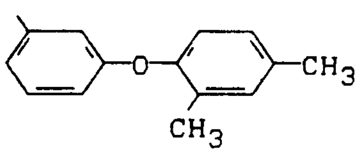 | Cl | 4.91 |
| III-112 | 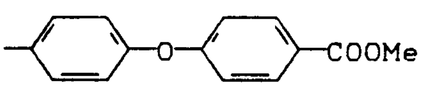 | Cl | Fp.: 126-127° C |
| III-113 | 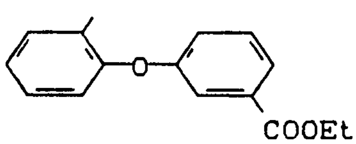 | Cl | 4.88 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung der $-CH_2$-E-Protonen als δ-Wert in ppm.

Beispiel IV-1

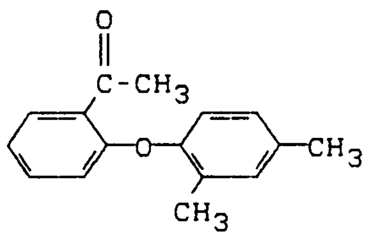

Zu 36,6 g (0,3 Mol) 2,4-Dimethylphenol (vgl. z.B. US 32 80 200) in 100 ml Dioxan gibt man 13,8 g (0,25 Mol) fein gepulvertes Kaliumhydroxid, erhitzt für eine Stunde auf Rückflußtemperatur, gibt dann bei 90 °C nacheinander 0,5 g fein verteiltes Kupferpulver und 30 g (0,15 Mol) 2-Bromacetophenon (vgl. z.B. J. Org. Chem. 46, 2169-2171 [1981]) zu, erhitzt für weitere 8 Stunden auf Rückflußtemperatur, läßt abkühlen, gibt dann die Reaktionsmischung in 300 ml Wasser, extrahiert mehrfach mit Dichlormethan, trocknet über Magnesiumsulfat, engt im Vakuum ein und destilliert den Rückstand im Hochvakuum.

Man erhält 24,2 g (67,5 % der Theorie) an 2-(2,4-Dimethylphenoxy)-acetophenon vom Siedepunkt 140-143 °C bei 0,2 mbar.

Beispiel IV-2

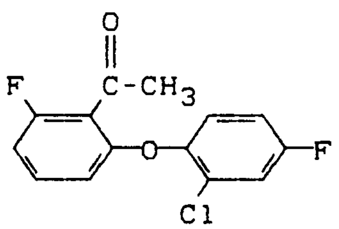

Zu einer Grignardlösung [hergestellt in üblicher Art und Weise aus 8,4 g Magnesiumspänen, 35 g (0,37 Mol) Brommethan, 0,1 g Kupfer-(I)-chlorid und 85 ml Ether] tropft man bei Raumtemperatur unter Rühren im Verlauf von ca. 30 Minuten eine Lösung von 69,2 g (0,262 Mol) 2-(2-Chlor-4-fluorphenoxy)-6-fluorbenzonitril (Herstellung analog US 39 50 379 bzw. J. Med. Chem. 29, 427-433 [1986]) in 500 ml Ether, rührt nach beendeter Zugabe weitere 5 Stunden bei Raumtemperatur, gibt dann die Reaktionsmischung in 1700 ml Eiswasser, gibt bei 0 °C bis 3 °C tropfenweise unter Rühren 20prozentige Schwefelsäure zu, bis sich ein pH-Wert von 2 einstellt (ca. 100 ml), rührt 10 Minuten bei Raumtemperatur und trennt dann die organische Phase ab. [Übliche Aufarbeitung der organischen Phase liefert 33 g unumgesetztes Ausgangsmaterial]. Die wässrige Phase wird 48 Stunden bei Raumtemperatur stehen gelassen, mit Dichlormethan extrahiert, über Magnesiumsulfat getrocknet und im Vakuum eingeengt. Das zurückbleibende Öl kristallisiert beim Stehenlassen.

Man erhält 22,6 g (59 % der Theorie) an 2-(2-Chlor-4-fluorphenoxy)-6-fluoracetophenon vom Schmelzpunkt 53 °C -54 °C.

Beispiel IV-3

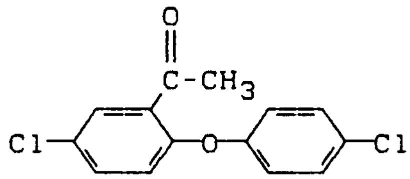

Zu 84,3 g (0,3 Mol) 5-Chlor-2-(4-chlorphenoxy)-anilin (vgl. z.B. EP 34 771), 284 ml Wasser und 72,2 ml konzentrierter Salzsäure gibt man bei 0 °C bis 5 °C tropfenweise unter Rühren eine Lösung von 27,6 g (0,4 Mol) Natriumnitrit in 31,7 ml Wasser, rührt nach beendeter Zugabe weitere 15 Minuten, filtriert, gibt 16,4 g (0,2 Mol) Natriumacetat zum Filtrat und gibt die so erhältliche Lösung bei 10 °C bis 15 °C tropfenweise unter Rühren in eine Mischung aus 253 ml Wasser, 123 g (1,5 Mol) Natriumacetat, 2,5 g (0,015 Mol) Natriumsulfat, 15,9 g (0,1 Mol) Kupfer-(II)-sulfat und 28,3 g (0,48 Mol) Acetaldoxim. Nach beendeter Zugabe rührt man eine Stunde bei Raumtemperatur, stellt dann durch Zugabe von ca. 40 ml konzentrierter Salzsäure den pH-Wert auf 7 ein, gibt anschließend portionsweise 292 ml konzentrierte Salzsäure zu und erhitzt 3 Stunden auf Rückflußtemperatur. Das Reaktionsgemisch wird einer Wasserdampfdestillation unterzogen, sich abscheidendes Öl in Dichlormethan aufgenommen, über Natriumsulfat getrocknet, eingeengt und im Hochvakuum destilliert.

Man erhält 29,5 g (35 % der Theorie) an 5-Chlor-2-(4-Chlorphenoxy)-acetophenon vom Siedepunkt 80-85 °C bei 1,5 mbar.

Beispiel IV-4

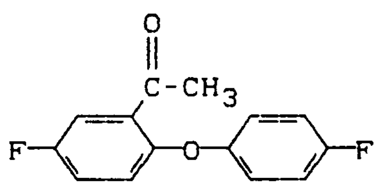

Zu 3,02 g (0,0385 Mol) Acetylchlorid in 250 ml Dichlormethan gibt man zunächst tropfenweise unter Rühren 7,21 g (0,035 Mol) 4,4'-Difluordiphenylether (vgl. z.B. Synth. Commun. 17, 685-692 [1987]) und anschließend portionsweise 6,05 g (0,0455 Mol) Aluminiumtrichlorid, rührt dann 5 Stunden bei Raumtemperatur, gießt die Reaktionsmischung in Eiswasser, extrahiert mit Dichlormethan, trocknet über Natriumsulfat, engt im Vakuum ein und chromatographiert den Rückstand an Kieselgel (Laufmittel: Dichlormethan).

Man erhält als 2. Fraktion 2,2 g (25,3 % der Theorie) an 5-Fluor-2-(4-fluorphenoxy)-acetophenon; $^1$H-NMR ($CDCl_3$) : $\delta$ = 2,63 ppm
und als 3. Fraktion 0,8 g (9,2 % der Theorie) an 2-Fluor-5-(4-fluorphenoxy)-acetophenon; $^1$H-NMR ($CDCl_3$): $\delta$ = 2,61 ppm.

Beispiel IV-5

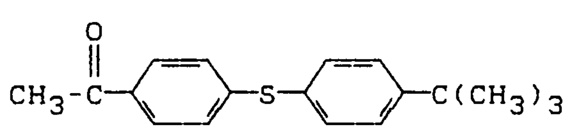

Zu 4,2 g (0,075 Mol) Kaliumhydroxid in 125 ml Dimethylformamid gibt man 13,19 g (0,079 Mol) 4-t-Butylthiophenol, rührt 1 Stunde bei Raumtemperatur, gibt dann 12,4 g (0,062 Mol) 4-Bromacetophenon zu und erhitzt 16 Stunden auf Rückflußtemperatur. Zur Aufarbeitung gießt man die erkaltete Reaktionsmischung in 200 ml Wasser, extrahiert mehrfach mit Dichlormethan, wäscht die vereinigten organischen Phasen mit Wasser, trocknet über Magnesiumsulfat, engt im Vakuum ein und destilliert den Rückstand im Hochvakuum.

Man erhält 14,3 g (81 % der Theorie) an 4-(4-t-Butylphenylthio)acetophenon vom Siedepunkt 173-175 °C bei 0,3 mbar und vom Schmelzpunkt 50 °C.

Beispiel IV-6

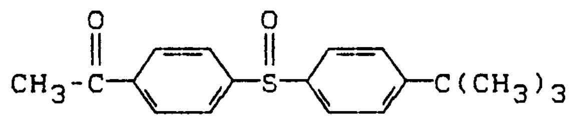

Zu 10,4 g (0,035 Mol) 4-(4-t-Butylphenylthio)-acetophenon in 100 ml absolutem Dichlormethan gibt man bei 25 °C bis 30 °C tropfenweise unter Rühren 8,9 g (0,04 Mol) m-Chlorperbenzoesäure (80prozentig) gelöst in 100 ml Dichlormethan, rührt nach beendeter Zugabe weitere 20 Stunden bei Raumtemperatur, saugt ab, wäscht das Filtrat nacheinander zweimal mit gesättigter wässriger Natriumhydrogencarbonatlösung und dann mit gesättigter wässriger Natriumchloridlösung, trocknet über Magnesiumsulfat, engt im Vakuum ein und kristallisiert den Rückstand aus Ethanol um.

Man erhält 6,93 g (63 % der Theorie) an 4-(4-t-Butylphenylsulfinyl)-acetophenon vom Schmelzpunkt 148 °C.

Beispiel IV-7

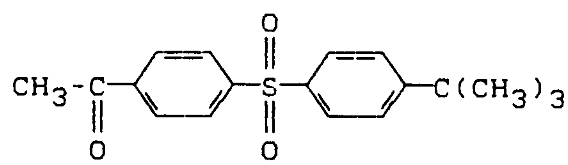

Zu 10,4 g (0,035 Mol) 4-(4-t-Butylphenylthio)-acetophenon in 100 ml absolutem Dichlormethan gibt man bei 25 °C bis 30 °C tropfenweise unter Rühren 18,38 g (0,08 Mol) m-Chlorperbenzoesäure (80prozentig) gelöst in 200 ml Dichlormethan zu, rührt nach beendeter Zugabe weitere 20 Stunden bei Raumtemperatur, saugt ab, wäscht das Filtrat nacheinander zweimal mit gesättigter wässriger Natriumhydrogencarbonatlösung und dann mit gesättigter wässriger Natriumchloridlösung, trocknet über Magnesiumsulfat, engt im Vakuum ein und kristallisiert den Rückstand aus Ethanol um.

Man erhält 6,7 g (58 % der Theorie) an 4-(4-t-Butylphenylsulfonyl)-acetophenon vom Schmelzpunkt 155 °C.

In entsprechender Weise und gemäß den allgemeinen Angaben zur Herstellung erhält man die folgenden Acetophenone der allgemeinen Formel (IV):

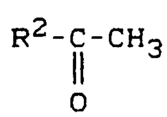

(IV)

| Bsp.Nr. | $R^2$ | physikalische Eigenschaften |
|---|---|---|
| IV-8 | 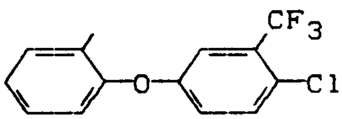 | Kp 125-126 °C/ 0,2 mbar |
| IV-9 | 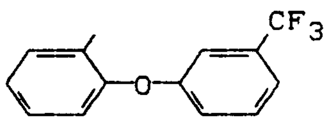 | Kp 106-108 °C/ 0,3 mbar |
| IV-10 | 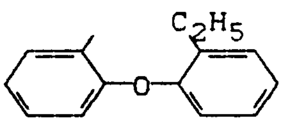 | Kp 126-128 °C/ 0,2 mbar |
| IV-11 | 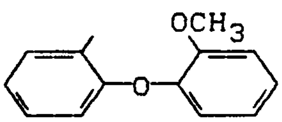 | Kp 130-132 °C/ 0,2 mbar |
| IV-12 | 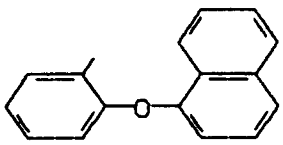 | Kp 156-160 °C/ 0,2 mbar |

| Bsp.Nr. | $R^2$ | physikalische Eigenschaften |
|---|---|---|
| IV-13 | 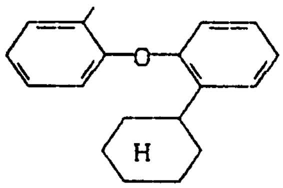 | Kp 156-158 °C/ 0,2 mbar |
| IV-14 | 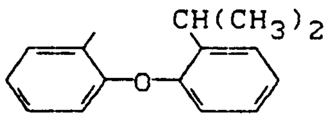 | Kp 136-138 °C/ 0,2 mbar |
| IV-15 | 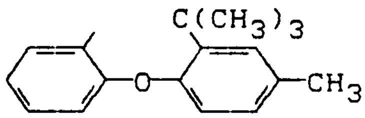 | Kp 150-152 °C/ 0,2 mbar |
| IV-16 | 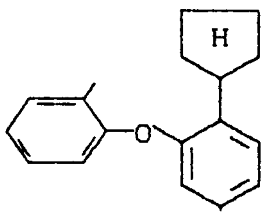 | Kp 157-160 °C/ 0,1 mbar |
| IV-17 | 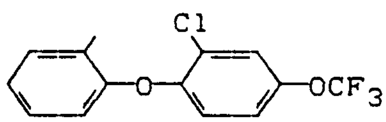 | Kp 128 °C/ 0,2 mbar |
| IV-18 | 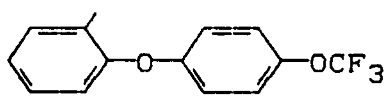 | Kp 123-125 °C/ 0,2 mbar |
| IV-19 | 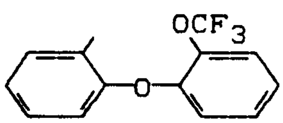 | Kp 108-110 °C/ 0,2 mbar |
| IV-20 | 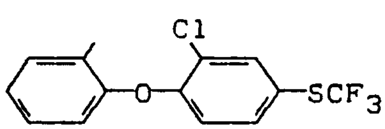 | Kp 140-142 °C/ 0,2 mbar |

| Bsp.Nr. | $R^2$ | physikalische Eigenschaften |
|---|---|---|
| IV-21 | 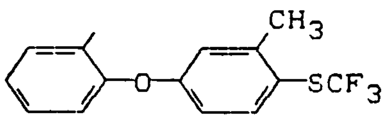 | Kp 135-138 °C/ 0,2 mbar |
| IV-22 | 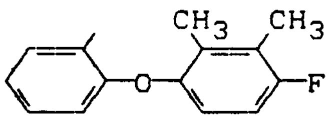 | Kp 137 °C/ 0,1 mbar |
| IV-23 | 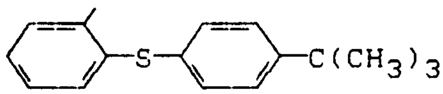 | Fp 170 °C |
| IV-24 | 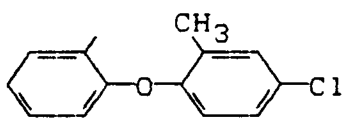 | Kp 150-152 °C/ 0,8 mbar |
| IV-25 | 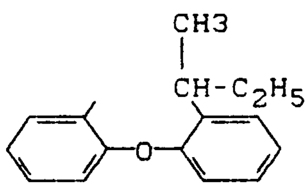 | Kp 132-135 °C/ 0,3 mbar |
| IV-26 | 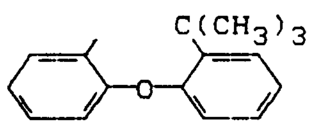 | Kp 140-142 °C/ 0,2 mbar |
| IV-27 | 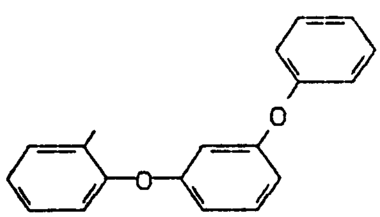 | $^1$H-NMR*): 2,62 |
| IV-28 | 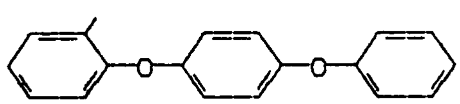 | $^1$H-NMR*): 2,62 |

| Bsp.Nr. | $R^2$ | physikalische Eigenschaften |
|---|---|---|
| IV-29 | 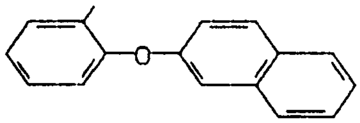 | Kp 168-170 °C/ 0,2 mbar |
| IV-30 | 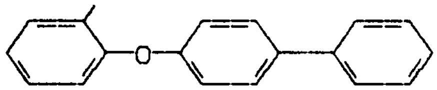 | $^1$H-NMR*): 2,64 |
| IV-31 | 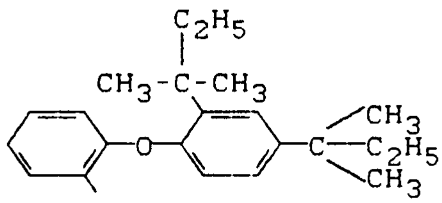 | Kp 186-190 °C/ 0,1 mbar |
| IV-32 | 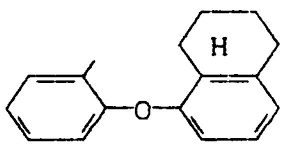 | Kp 177-180 °C/ 0,1 mbar |
| IV-33 | 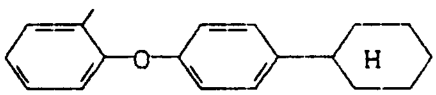 | Kp 166-170 °C/ 0,15 mbar |
| IV-34 | 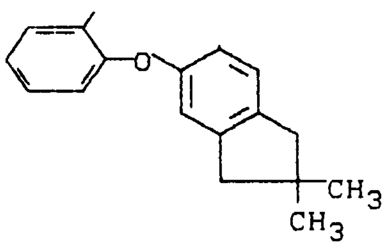 | Kp 180-182 °C/ 0,2 mbar |
| IV-35 | 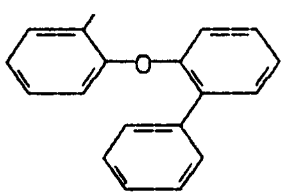 | $^1$H-NMR*): 2,65 |

| Bsp.Nr. | $R^2$ | physikalische Eigenschaften |
|---|---|---|
| IV-36 | 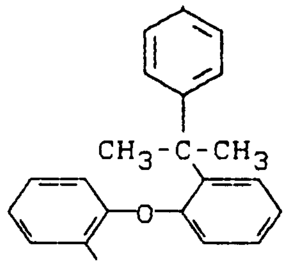 | Fp 99 °C |
| IV-37 | 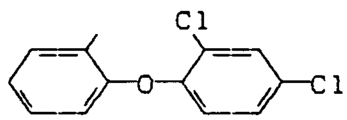 | Kp 118-120 °C/ 0,2 mbar |
| IV-38 | 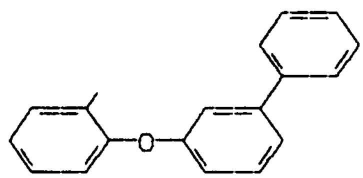 | $^1$H-NMR*): 2,68 |
| IV-39 | 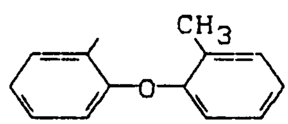 | Kp 135-138 °C/ 0,2 mbar |
| IV-40 | 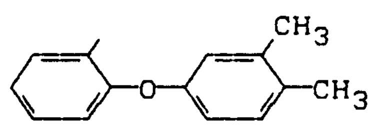 | Kp 146-148 °C/ 0,3 mbar |
| IV-41 | 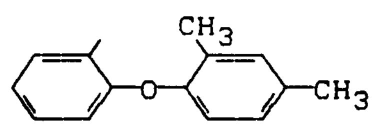 | Kp 133-135 °C/ 0,2 mbar |
| IV-42 | 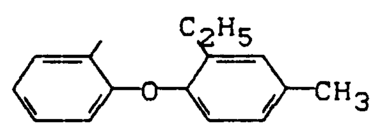 | Kp 138-139 °C/ 0,2 mbar |

| Bsp.Nr. | $R^2$ | physikalische Eigenschaften |
|---|---|---|
| IV-43 | 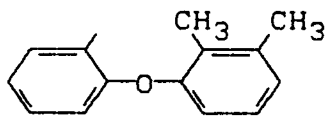 | Kp 150-152 °C/ 0,2 mbar |
| IV-44 | 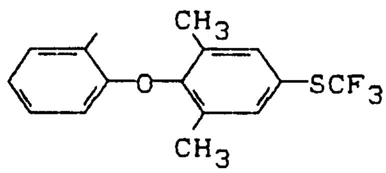 | Kp 145-148 °C/ 0,2 mbar |
| IV-45 | 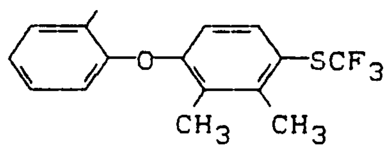 | Kp 138-140 °C/ 0,2 mbar |
| IV-46 | 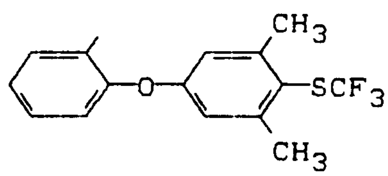 | Kp 140-144 °C/ 0,2 mbar |
| IV-47 | 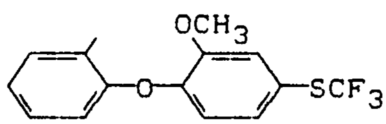 | Kp 147-150 °C/ 0,2 mbar |
| IV-48 | 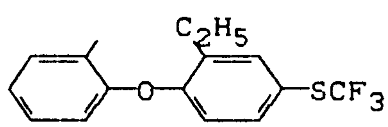 | Kp 153-155 °C/ 0,4 mbar |
| IV-49 | 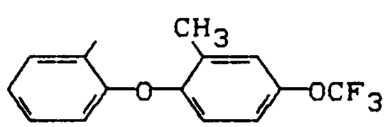 | Kp 118-120 °C/ 0,2 mbar |
| IV-50 | 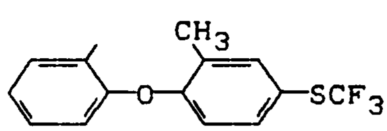 | Kp 133-136 °C/ 0,2 mbar |

| Bsp.Nr. | $R^2$ | physikalische Eigenschaften |
|---|---|---|
| IV-51 | 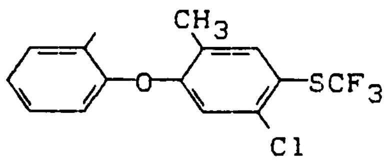 | Kp 143-145 °C/ 0,2 mbar |
| IV-52 | 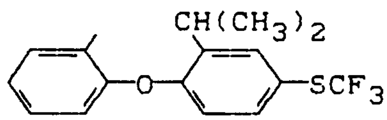 | Kp 130-132 °C/ 0,2 mbar |
| IV-53 | 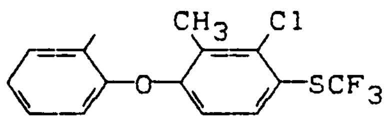 | Kp 142-144 °C/ 0,2 mbar |
| IV-54 | 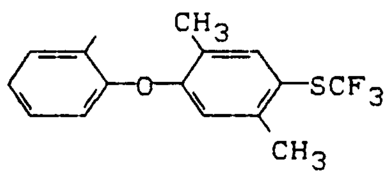 | Kp 143-144 °C/ 0,2 mbar |
| IV-55 | 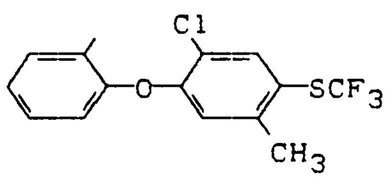 | $^1$H-NMR*): 2,59 |
| IV-56 | 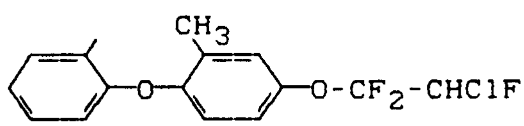 | Kp 171-173 °C/ 0,2 mbar |
| IV-57 | 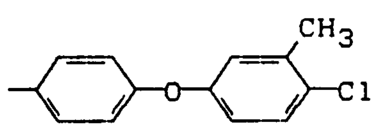 | $^1$H-NMR*): 2,61 |
| IV-58 | 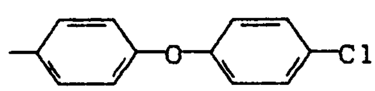 | Kp 138-140 °C/ 0,15 mbar |

| Bsp.Nr. | $R^2$ | physikalische Eigenschaften |
|---|---|---|
| IV-59 | 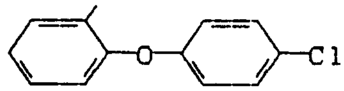 | Kp 136-139 °C/ 0,2 mbar |
| IV-60 | 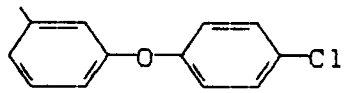 | Kp 135-138 °C/ 0,3 mbar |
| IV-61 | 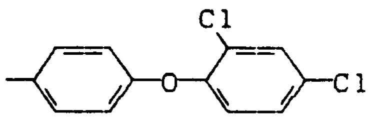 | $^1$H-NMR*): 2,66 |
| IV-62 | 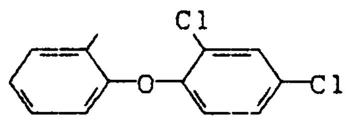 | Kp 140 °C/ 0,3 mbar |
| IV-63 | 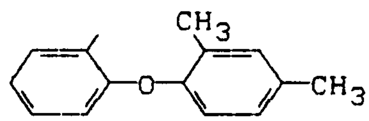 | Kp 141-142 °C/ 0,2 mbar |
| IV-64 | 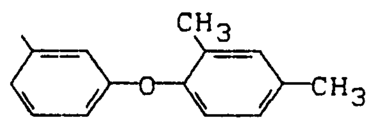 | Kp 127-130 °C/ 0,3 mbar |
| IV-65 | 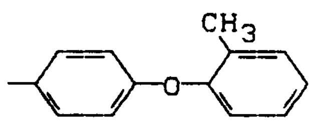 | Kp 127-130 °C/ 0,4 mbar |
| IV-66 | 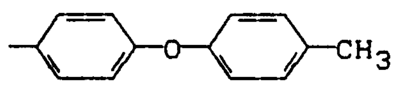 | Fp 46-47 °C |
| IV-67 | 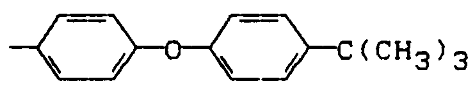 | $^1$H-NMR*): 2,63 |

| Bsp.Nr. | $R^2$ | physikalische Eigenschaften |
|---|---|---|
| IV-68 | 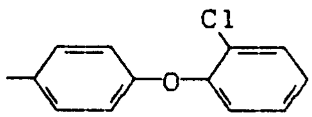 | $^1$H-NMR*): 2,60 |
| IV-69 | 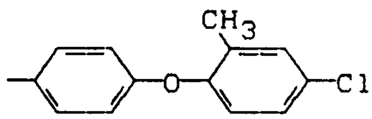 | $^1$H-NMR*): 2,62 |
| IV-70 | 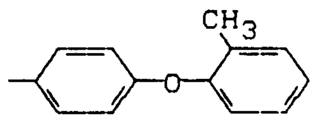 | $^1$H-NMR*): 2,61 |
| IV-71 | 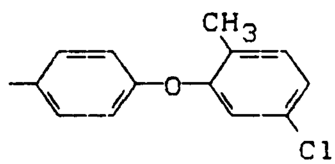 | $^1$H-NMR*): 2,62 |
| IV-72 | 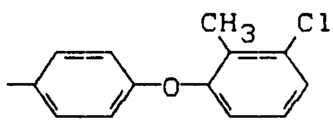 | $^1$H-NMR*): 2,64 |
| IV-73 | 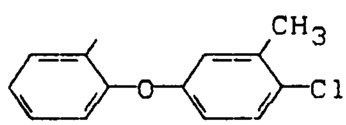 | $^1$H-NMR*): 2,64 |
| IV-74 | 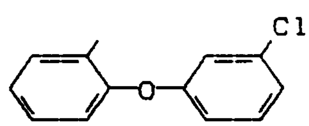 | Fp 43-44 °C |
| IV-75 | 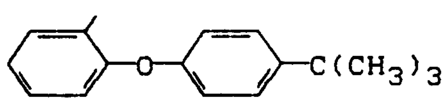 | $^1$H-NMR*): 2,63 |
| IV-76 | 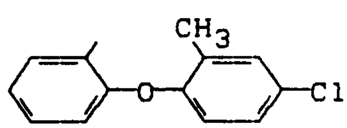 | $^1$H-NMR*): 2,61 |

| Bsp.Nr. | $R^2$ | physikalische Eigenschaften |
|---|---|---|
| IV-77 | 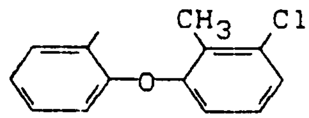 | $^1$H-NMR*): 2,61 |
| IV-78 | 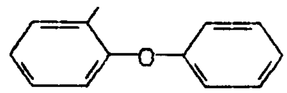 | Kp 118-120 °C/ 0,15 mbar |
| IV-79 | 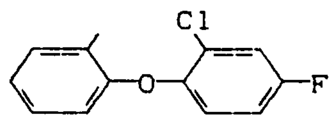 | Fp 59-60 °C |
| IV-80 | 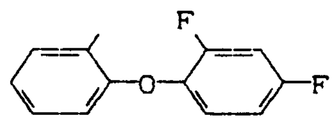 | Kp 106-108 °C/ 0,15 mbar |
| IV-81 | 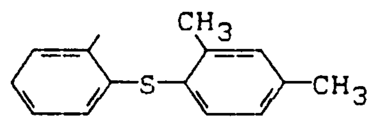 | Fp 111 °C |
| IV-82 | 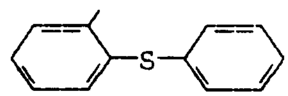 | Fp 71-73 °C |
| IV-83 | 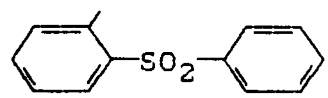 | $^1$H-NMR*): 2,67 |
| IV-84 | 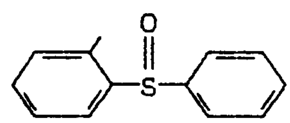 | Fp 132-133 °C |
| IV-85 | 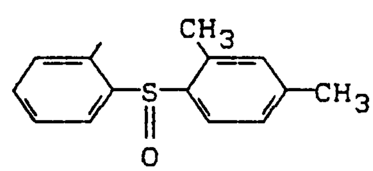 | Fp 148 °C |

| Bsp.Nr. | $R^2$ | physikalische Eigenschaften |
|---|---|---|
| IV-86 | 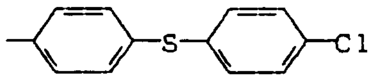 | Fp 50 °C |
| IV-87 | 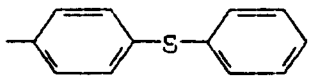 | Fp 56-57 °C |
| IV-88 | 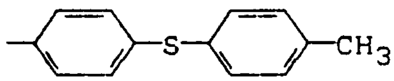 | Fp 86 °C |
| IV-89 | 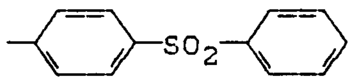 | Fp 134-135 °C |
| IV-90 | 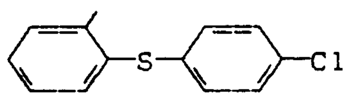 | Fp 83 °C |
| IV-91 | 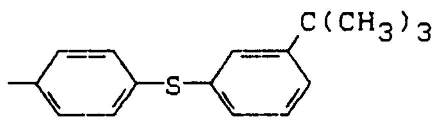 | Kp 178-180 °C/ 0,2 mbar |
| IV-92 | 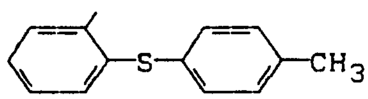 | Fp 99 °C |
| IV-93 | 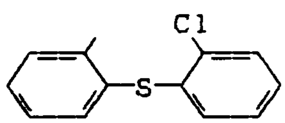 | Kp 175 °C/ 0,2 mbar |
| IV-94 | 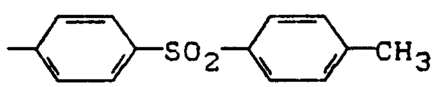 | Fp 157 °C |
| IV-95 | 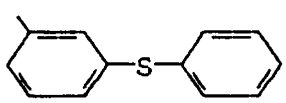 | Kp 145 °C/ 0,3 mbar |

| Bsp.Nr. | $R^2$ | physikalische Eigenschaften |
|---|---|---|
| IV-96 | 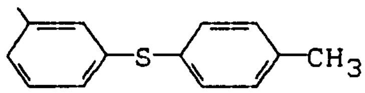 | Kp 148-150 °C/ 0,2 mbar |
| IV-97 | 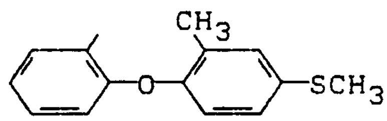 | Kp: 171-173° C/ 0,15 mbar |
| IV-98 | 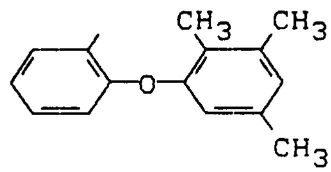 | Kp: 188-190° C/ 0,15 mbar |
| IV-99 | 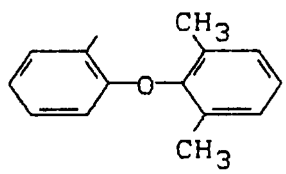 | Kp: 148-150° C/ 0,1 mbar |
| IV-100 | 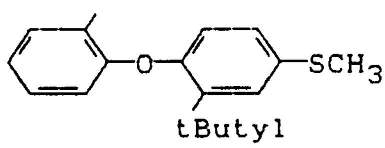 | $^1$H-NMR: 2,73 |
| IV-101 | 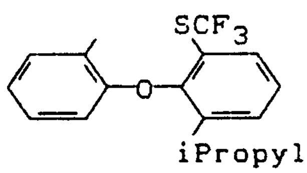 | Kp: 120-122° C/ 0,03 mbar |
| IV-102 | 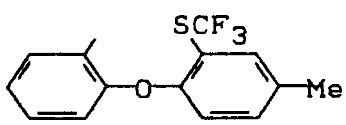 | Kp: 124-125° C/ 0,2 mbar |
| IV-103 | 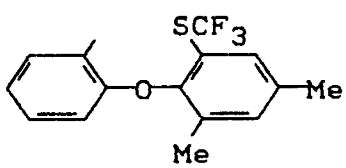 | Kp: 123-125° C/ 0,2 mbar |

| Bsp.Nr. | $R^2$ | physikalische Eigenschaften |
|---|---|---|
| IV-104 | 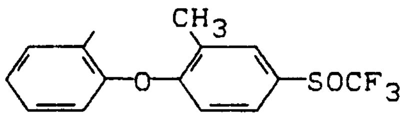 | 2.60 |
| IV-105 | 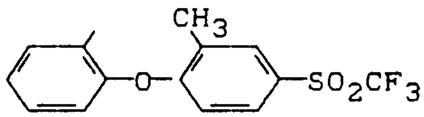 | 2.58 |
| IV-106 | 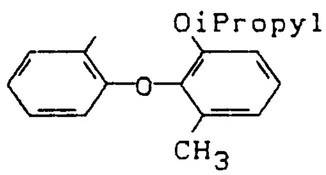 | Kp: 151-153° C/ 0,2 mbar |
| IV-107 | 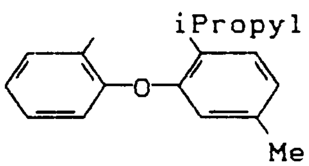 | Kp: 157-159° C/ 0,15 mbar |
| IV-108 | 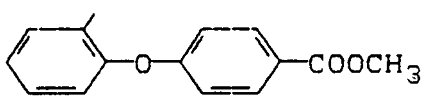 | Kp: 157-160° C/ 0,2 mbar |
| IV-109 | 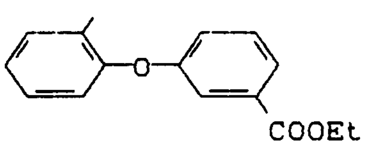 | Kp: 152-155° C/ 0,2 mbar |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Zwischenprodukte:

Beispiel VIIa-1

3-[(α,α,β,β-Tetrafluorethyl)-thio]-phenol

126 g (1 Mol) 4-Mercaptophenol und 11,2 g (0,2 Mol) Kaliumhydroxid wurden in 300 ml Dimethylformamid gelöst. Bei 90 bis 100 °C Innentemperatur wurde die Apparatur mit Stickstoff gespült und dann unter kräftigem Rühren Tetrafluorethylen eingeleitet. Sobald nichts mehr aufgenommen wurde wurden 75 Vol.-% des Dimethylformamids im Vakuum abgezogen, der Rückstand in 500 ml Eiswasser eingerührt, dieses auf einen pH-Wert von 3,5 gebracht, die organische Phase isoliert und destilliert. Es wurden 167 g Produkt mit einem Siedepunkt von 72 bis 73 °C bei 17 mbar erhalten.

Beispiel VIIa-2

2,3,6-Trichlor-4-trifluormethoxy-phenol

89 g (0,5 Mol) 4-Trifluormethoxy-phenol wurden in 150 ml Tetrachlorkohlenstoff gelöst. Nach dem Zusatz von 0,5 g Eisenpulver wurde bei 40 °C Chlor eingeleitet. Der Reaktionsverlauf wurde gaschromatographisch kontrolliert. Nach dem Erreichen eines 98 %igen Anteils an Trichlorprodukt wurde mit Stickstoff ausgeblasen und anschließend der Ansatz fraktioniert destilliert. Es wurden 106 g Produkt mit einem Siedepunkt von 120 bis 122 °C bei 20 mbar erhalten, was einer Ausbeute von 74 % der Theorie entspricht. Der Schmelzpunkt des Produktes lag bei 56 bis 58 °C.

Beispiel VIIa-3

2-Nitro-4-trifluormethoxy-phenol

53,4 g (0,3 Mol) 4-Trifluormethoxyphenol wurden bei 25 °C im Verlaufe von 30 Minuten in 75 ml 30 %ige Salpetersäure eintropfen gelassen. Es wurde 2 Stunden bei 25 °C nachgerührt, dann wurde der Ansatz in 500 ml Eiswasser gegossen und die organische Phase abgetrennt. Deren Destillation lieferte 56 g Produkt mit einem Kochpunkt von 102 bis 103° bei 26 mbar, was einer Ausbeute von 83 % der Theorie entspricht.

Beispiel VIIa-4

2,6-Dinitro-4-trifluormethoxy-phenol

53,4 g (0,3 Mol) 4-Trifluormethoxyphenol wurden bei 60 °C in 150 ml 40 %ige Salpetersäure im Verlaufe von 1 Stunde eintropfen gelassen. Es wurde noch 5 Stunden bei 60 °C nachgerührt und dann wie in Beispiel 3 aufgearbeitet. Es wurden 63 g Produkt mit einem Siedepunkt von 126 bis 128 °C bei 0,1 mbar erhalten, was 78 % der Theorie entspricht.

Beispiel VIIa-5

2-Hydroxy-5-trifluormethoxy-benzoesäure

178 g (1 Mol) 4-Trifluormethoxyphenol und 345 g (2,5 Mol) Kaliumcarbonat wurden in einem Autoklaven vorgelegt, danach 60 bar Kohlendioxid aufgepreßt und 4 Stunden auf 200 °C erwärmt. Nach dem Abkühlen und Entspannen wurde der Rückstand in 1,5 l heißem Wasser gelöst, heiß filtriert, das abgekühlte Filtrat mit Methylenchlorid extrahiert und mit Aktivkohle geklärt. Anschließend wurde ein pH von 1 eingestellt und das ausgefallene Produkt abgesaugt. Es wurden 192 g Produkt mit einem Schmelzpunkt von 129 °C erhalten, was 86 % der Theorie entspricht.

Beispiel VIIa-6

2-Hydroxy-5-trifluormethylthio-benzoesäure

Es wurde verfahren wie in Beispiel 5 beschrieben, jedoch wurden 194 g (1 Mol) 4-Trifluormethylmercapto-phenol eingesetzt und 185,7 g Produkt mit einem Schmelzpunkt von 122 bis 125 °C erhalten was 78 % der Theorie entspricht.

Beispiel VIIa-7

2-Hydroxy-5-trifluormethoxy-benzamid

100 g (0,45 Mol) 2-Hydroxy-5-trifluormethoxy-benzoesäure (erhalten gemäß Beispiel 5) wurden in 350 ml Petrolether vorgelegt. Nach dem Zusatz von 1 ml Pyridin wurden im Verlaufe von 30 Minuten bei 30 °C 56 g $SOCl_2$ zutropfen gelassen und noch 5 Stunden bei 30 °C nachgerührt. Danach wurde von einem schmierigen Reaktionsrückstand abdekantiert und die abdekantierte Lösung mit Ammoniakgas gesättigt. Der ausfallende Feststoff wurde abfiltriert und getrocknet. Es wurden 82 g Produkt mit einem Schmelzpunkt

von 188 bis 190 °C erhalten, was 75 % der Theorie entspricht.

Beispiel VIIa-8

3,4-Bis-trifluormethoxy-phenol

a) Herstellung des als Vorprodukt benötigten 3,4-Bis-trifluormethoxy-anilins
415 g (3 Mol) Veratrol und 5 g Azobisisobutyronitril wurden in 3 l Tetrachlorkohlenstoff gelöst und auf Rückflußtemperatur erwärmt. Unter UV-Belichtung wurden 2,2 kg Chlor eingeleitet, was etwa 30 Stunden in Anspruch nahm, und gleichzeitig 20 g Azobisisobutyronitril gleichmäßig über den Reaktionszeitraum verteilt, zugegeben. Nach dem Reaktionsende wurde mit Stickstoff ausgeblasen, eingeengt und fraktioniert destilliert. Es wurden 650 g 92 %iges 1,2-Bistrichlormethoxy-benzol mit einem Siedepunkt von 106 bis 108 °C bei 0,2 mbar erhalten.

Zu 375 g so erhaltenem 1,2-Bistrichlormethoxy-benzol wurde bei -5 bis 0 °C 50 ml Fluorwasserstoff zutropfen gelassen, was 5 Stunden beanspruchte, 2,5 ml Antimonpentachlorid zugegeben und dann 12 Stunden lang auf 140 °C erwärmt. Der entstehende Chlorwasserstoff wurde bei 25 bar kontinuierlich entspannt. Nach dem Ende der Reaktion wurde überschüssiger Fluorwasserstoff abdestilliert, der Destillationsrückstand in 500 ml Wasser eingerührt und die organische Phase abgetrennt. Durch deren Fraktionierung wurden 178 g 98 %iges 1,2-Bistrifluormethoxy-benzol erhalten.

248 g so erhaltenes 1,2-Bistrifluormethoxybenzol wurden bei 0 °C in 250 g eines Gemisches aus 33 Gew.-% Salpetersäure und 67 Gew.-% Schwefelsäure im Verlaufe von 2 Stunden eintropfen gelassen. Danach wurde 5 Stunden bei 0 °C nachgerührt, anschließend auf Eiswasser gegeben, die organische Phase abgetrennt und destilliert. Es wurden 284 g 99 %iges 3,4-Bistrifluormethoxy-nitrobenzol erhalten.

291 g so erhaltenes 3,4-Bistrifluormethoxy-nitrobenzol wurden in 500 ml Methanol unter Zusatz von 10 g Raney-Nickel bei 40 °C im Verlaufe von 3 Stunden mit 20 bar Wasserstoff hydriert. Nach dem Absaugen des Katalysators wurde fraktioniert destilliert und 242 g 3,4-Bistrifluormethoxy-anilin erhalten.

b) Herstellung von 3,4-Bistrifluormethoxy-phenol
356 g (1,36 Mol) 3,4-Bistrifluormethoxy-anilin wurden in 625 g Wasser und 332 g konzentrierter Salzsäure mit 246 g 40 %iger Natriumnitritlösung diazotiert. Die Diazoniumsalzlösung wurde in eine 120 °C heiße Mischung aus 520 g Wasser, 968 g konzentrierter Schwefelsäure und 1200 g Xylol so zutropfen gelassen, daß dort durch azeotropes Abdestillieren des Wassers eine Innentemperatur von 120 °C aufrechterhalten werden konnte. Anschließend wurde die Xylolphase abgetrennt und durch Extraktion mit Alkalilösung das entstandene 3,4-Bistrifluormethoxyphenol isoliert. Durch Destillation wurden 200 g Produkt mit einem Siedepunkt von 86 bis 88 °C bei 25 mbar erhalten, was 53 % der Theorie entspricht.

Beispiel VIIa-9

3-(α,α,β,β-Tetrafluorethoxy)-4-methyl-phenol

a) Herstellung des als Vorprodukt benötigten 3-(α,α,β,β-Tetrafluorethoxy)-4-methyl-anilins
165 g 3-Hydroxy-4-methyl-acetanilid und 25 g Kaliumhydroxid wurden in 850 g Dimethylformamid auf 95 °C erwärmt. Unter kräftigem Rühren wurden 120 g Tetrafluorethylen innerhalb von 5 Stunden eingeleitet. Nach wäßriger Aufarbeitung wurden 114 g 3-(α,α,β,β-Tetrafluorethoxy)-4-methyl-acetanilid erhalten. 265 g so hergestelltes 3-(α,α,β,β-Tetrafluorethoxy)-4-methyl-acetanilid wurden in 1,25 l Methanol mit 375 ml 25 %iger Natronlaugelösung versetzt und 12 Stunden auf 70 °C erwärmt. Nachdem auf ca. 50 % des ursprünglichen Volumens eingeengt worden war wurde der Ansatz wäßrig aufgearbeitet. Die Destillation der organischen Phase lieferte 192 g 3-(α,α,β,β-Tetrafluorethoxy)-4-methoxyanilin.

b) Herstellung von 3-(α,α,β,β-Tetrafluorethoxy)-4-methyl-phenol
224 g 3-(α,α,β,β-Tetrafluorethoxy)-4-methyl-anilin wurden analog Beispiel 8 umgesetzt. Es wurden 153,5 g Produkt mit einem Siedepunkt von 78 bis 79 °C bei 0,25 mbar erhalten, was 68 % der Theorie entspricht.

Beispiel VIIa-10

2-Methyl-4-trifluormethoxy-phenol

191 g (1 Mol) 2-Methyl-4-trifluormethoxy-anilin wurden analog Beispiel 8 umgesetzt. Es wurden 156 g Produkt mit einem Schmelzpunkt von 72 °C und einem Siedepunkt von 80 bis 180 °C bei 12 mbar erhalten, was 81 % der Theorie entspricht.

Beispiel VIIa-11

2-Methyl-4-($\alpha$,$\alpha$,$\beta$-Trifluor-$\beta$-chlor-ethoxy)-phenol

238,5 g (1 Mol) 2-Methyl-4-($\alpha$,$\alpha$,$\beta$-Trifluor-$\beta$-chlor-ethoxy)-anilin wurden analog beispiel 8 umgesetzt. Es wurden 192,5 g Produkt mit einem Siedepunkt von 83 bis 84 °C bei 0,35 mbar erhalten, was 80 % der Theorie entspricht.

Beispiel VIId-1 bis VIId-12

Allgemeine Arbeitsvorschrift:

In 800 ml Di-tert.-butylether wurden 1,64 Mol Phenol der Formel (VIIe) vorgelegt, 1,8 Mol Pyridin zugegeben und bei 20 °C 1,65 bis 1,70 Mol Trifluormethylsulfenchlorid eingeleitet. Dann wurde unter Rühren 4 Stunden auf 50 °C erwärmt. Danach wurde Stickstoff durch das Reaktionsgemisch geblasen (Gasableitung über einen mit wäßrigem Ammoniak gefüllten Wäscher). Danach wurde Pyridinhydrochlorid durch Filtration abgetrennt, leichtflüchtige Bestandteile (im wesentlichen Di-tert.-butylether) unter vermindertem Druck abdestilliert und so rohes 4-Trifluormethylmercapto-phenol erhalten. Die Reinigung erfolgte durch Feindestillation oder durch Chromatographie, wobei die chromatographische Reinigung wie folgt durchgeführt wurde:

Eine Säule (lichte Weite 45 mm) wurde mit einer Aufschlämmung aus Kieselgel in Toluol bis zu einer Höhe von 40 cm gefüllt. Das rohe 4-Trifluormethylmercapto-phenol wurde in wenig Toluol gelöst in die Säule eingetragen. Danach wurde mit Toluol chromatographiert. Aus der jeweiligen 2. Fraktion wurde das Toluol unter vermindertem Druck abdestilliert und so die 4-Trifluormercaptophenole in reiner Form gewonnen.

Die im einzelnen durchgeführten Beispiele sind in der nachfolgenden Tabelle zusammengefaßt.

**Tabelle**

| Beispiel Nr. | Einsatzprodukt 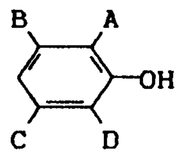 | gereinigtes Endprodukt 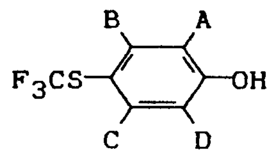 | physikalische Daten des gereinigten Reaktionsproduktes: Siedepunkt °C/mbar | $n_D^{20}$ | Schmelzpunkt °C |
|---|---|---|---|---|---|
| VIId-1 | A = $C_2H_5$; B = C = D = Wasserstoff | | 97-98/10 | 1.5030 | n.b. |
| VIId-2 | A = $CH(CH_3)_2$; B = C = D = Wasserstoff | | 92-94/10 | 1.4955 | n.b. |
| VIId-3 | A = $OCH_3$; B = C = D = Wasserstoff | | 94-96/12 | 1.5070 | n.b. |
| VIId-4 | A = D = $CH_3$; B = C = Wasserstoff | | 122-124/22 | n.b. | 38-40 |
| VIId-5 | A = B = $CH_3$; C = D = Wasserstoff | | 120-123/16 | 1.5174 | n.b. |
| VIId-6 | B = C = $CH_3$; A = D = Wasserstoff | | 127-130/16 | n.b. | 68 |
| VIId-7 | A = $CH_3$; B = Cl; C = D = Wasserstoff | | 125-128/16 | n.b. | 48-50*) |
| VIId-8 | A = $CH_3$; C = Cl; B = D = Wasserstoff | | 107-108/10 | 1.5280 | n.b. *) |
| VIId-9 | A = C = $CH_3$; B = D = Wasserstoff | | 104-106/10 | 1.5090 | n.b. |
| VIId-10 | A = Cl; C=$CH_3$; B = D = Wasserstoff | | 125 /14 | 1.5380 | n.b. |
| VIId-11 | A = $C(CH_3)_3$; B = C = D = Wasserstoff | | 110-112/10 | 1.4945 | n.b. |
| VIId-12 | A = C = D = Wasserstoff; B = F | | 72- 73/10 | 1.5045 | n.b. |

*) In diesen Beispielen wurde das Produkt durch Chromatographie gereinigt, sonst wurden die Produkte durch Feindestillation gereinigt.

n.b. nicht bestimmt

Beispiel VIId-13

4-Trifluormethylmercapto-2,3-dimethylphenol durch $TiCl_4$-Katalyse.

In 500 ml Dichlormethan wurden 100 g 2,3-Dimethylphenol vorgelegt und 10 ml Titantetrachlorid zugegeben. Dann leitete man 50 g Trifluormethylsulfenchlorid bei 20 °C ein und rührte 3 Stunden nach. Danach wurden 100 ml Wasser eingerührt, die Phasen getrennt und die organische Phase destilliert. Nach einem Vorlauf, bestehend aus Dimethylphenol und Produkt, destillierten bei 85-88 °C/6mbar 68 g Produkt.

Beispiele VIId-14 bis VIId-17

Allgemeine Arbeitsvorschrift:

In 80 ml Essigsäure wurden 0,08 Mol eines 4-Trifluormethylmercapto-phenols der Formel (VIId) vorgelegt, 35 g 35 %iges Wasserstoffperoxid zugetropft und das Gemisch für 3 Std. auf 90 °C erwärmt. Nach dem Abkühlen wurde das Gemisch in 200 ml Wasser eingerührt und das ausgefallene Produkt filtriert.

Die im einzelnen durchgeführten Beispiele sind in der folgenden Tabelle zusammengefaßt.

**Tabelle**

| Beispiel Nr. | Einsatzprodukt | Reaktionsprodukt | Schmelzpunkt [°C] | Ausbeute [% d.Th.] |
|---|---|---|---|---|
| | 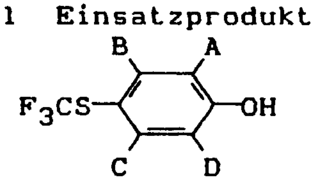 | 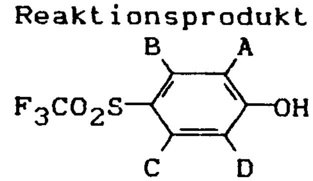 | | |
| VIId-14 | A = $OCH_3$; B = C = D = Wasserstoff | | 121 | 83 |
| VIId-15 | A = $CH_3$; C = Cl; B = D = Wasserstoff | | 111 | 91 |
| VIId-16 | A = B = $CH_3$; C = D = Wasserstoff | | 83 | 74 |
| VIId-17 | A = D = $CH_3$; B = C = Wasserstoff | | 151 | 94 |

Anwendungsbeispiele

In den folgenden Anwendungsbeispielen wurden die nachstehend aufgeführten Verbindungen als Vergleichssubstanzen eingesetzt:

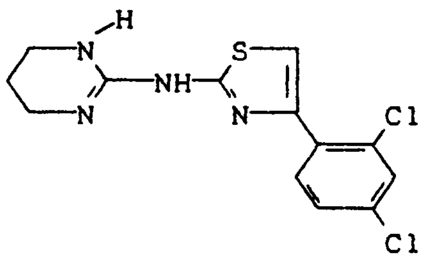

x HCl (A)

4-(2,4-Dichlorphenyl)-2-[2-(1,4,5,6-tetrahydropyrimidinyl)-amino]-thiazol Hydrochlorid

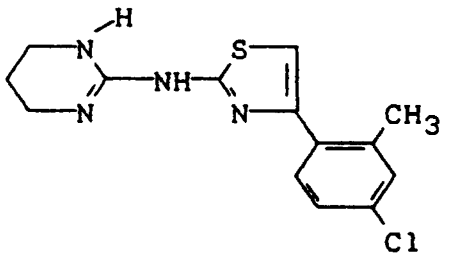

x HCl (B)

4-(4-Chlor-2-methylphenyl)-2-[2-(1,4,5,6-tetrahydropyrimidinyl)-amino]-thiazol Hydrochlorid

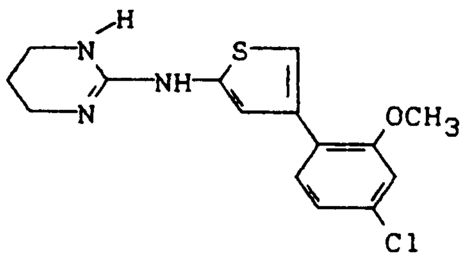

(C)

4-(4-Chlor-2-methoxyphenyl)-2-[2-(1,4,5,6-tetrahydropyrimidinyl)-amino]-thiazol
(alle bekannt aus DE-OS 32 20 118)

Beispiel A

Antimykotische in-vitro-Wirksamkeit

Versuchsbeschreibung:

Die in-vitro-Prüfungen wurden mit Keiminokula von durchschnittlich 5 x $10^3$ Keimen/ml Substrat durchgeführt. Als Nährmedium diente Yeast Nitrogen Base Medium für Hefen und Kimmig-Medium für Schimmelpilze.

Die Bebrütungstemperatur betrug 37 °C bei Hefen und 28 °C bei Schimmelpilzen, die Bebrütungsdauer lag bei 24 bis 96 Stunden bei Hefen und 96 bis 120 Stunden bei Schimmelpilzen.

Die Beurteilung der Fungizide erfolgte durch Ausplattieren und erneutes Bebrüten voll gehemmter Ansätze, wobei fungizide Konzentrationen weniger als 100 Keime CFN (colony forming unit) pro ml enthielten.

In diesem Test zeigen die erfindungsgemäßen Verbindungen der Formel (I) gemäß den Herstellungsbeispielen 3, 6, 16, 25, 30, 35, 42, 45, 47, 48, 50, 92, 98, 116, 132, 133, 134, 135, 137, 147, 149, 150, 151,

153 und 181 eine deutlich bessere antimykotische Wirksamkeit als die aus dem Stand der Technik bekannten Vergleichsverbindungen (A), (B) und (C).

**Tabelle A: Antimykotische in-vitro-Wirksamkeit**

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (A) (bekannt) 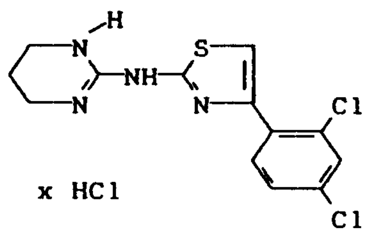 x HCl | 16 | 64 | 16 | 32 |
| (B) (bekannt) 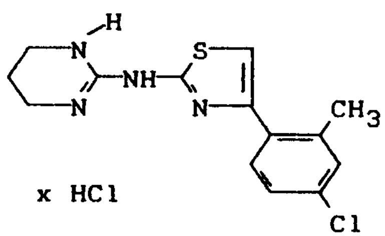 x HCl | 16 | 64 | 64 | >64 |

*) minimale fungizide Konzentration von >99 %

Tabelle A - Fortsetzung: Antimykotische in-vitro-Wirksamkeit

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (C) (bekannt) 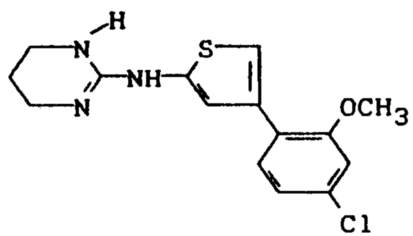 | 〉64 | 〉64 | 〉64 | 〉64 |
| (3) 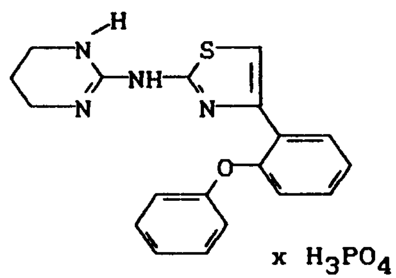 x $H_3PO_4$ | 0,25 | 4 | 32 | 64 |

*) minimale fungizide Konzentration von >99 %

Tabelle A - Fortsetzung: Antimykotische in-vitro-Wirksamkeit

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (6) 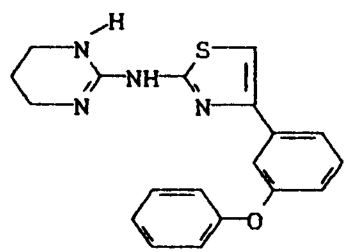 | 0,25 | 4 | 32 | 64 |
| (16) 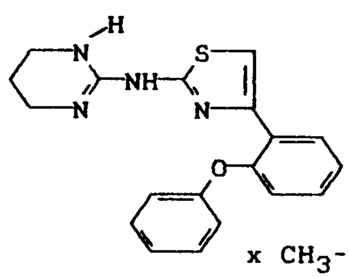 x $CH_3$-$SO_3H$ | 0,25 | 4 | 32 | 64 |

*) minimale fungizide Konzentration von >99 %

Tabelle A - Fortsetzung: Antimykotische in-vitro-Wirksamkeit

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (25) 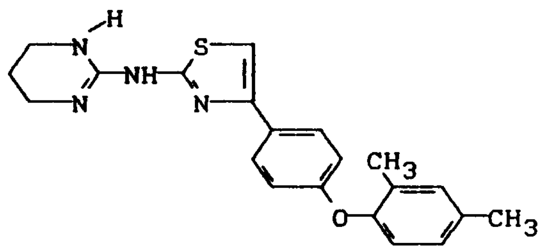 x HBr | 0,5 | 4 | 8 | 8 |
| (30) 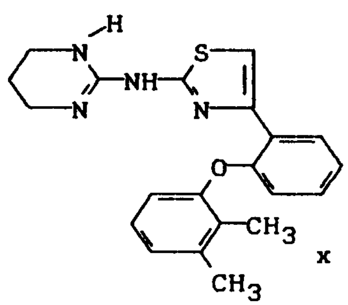 x $CH_3-SO_3H$ | 0,5 | 4 | 8 | 8 |

*) minimale fungizide Konzentration von >99 %

**Tabelle A - Fortsetzung: Antimykotische in-vitro-Wirksamkeit**

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (35) 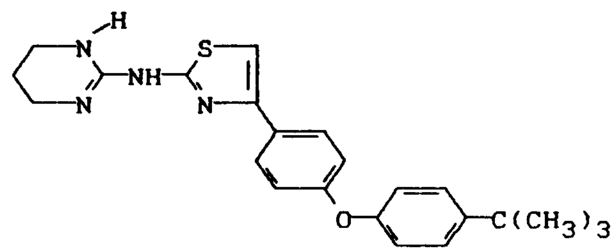 x HBr | 0,5 | 8 | 8 | 8 |
| (42) 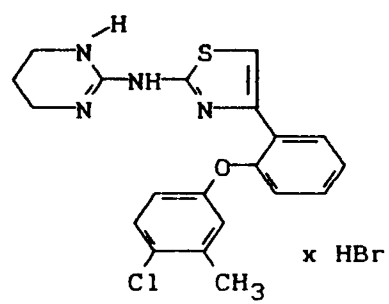 x HBr | 4 | 8 | 8 | 8 |

*) minimale fungizide Konzentration von > 99 %

**Tabelle A - Fortsetzung: Antimykotische in-vitro-Wirksamkeit**

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (45) 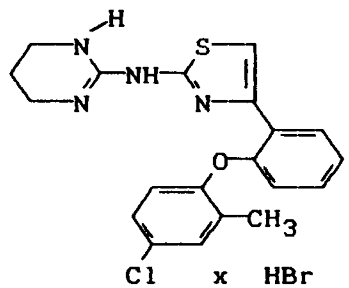 x HBr | 0,25 | 4 | 8 | 8 |
| (47) 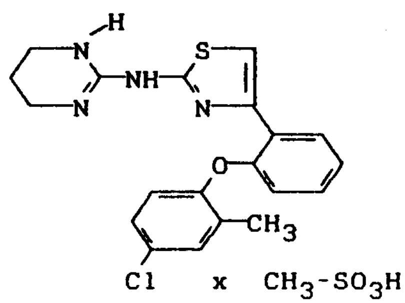 x $CH_3-SO_3H$ | 0,25 | 4 | 8 | 8 |

*) minimale fungizide Konzentration von > 99 %

Tabelle A - Fortsetzung: Antimykotische in-vitro-Wirksamkeit

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (48)<br>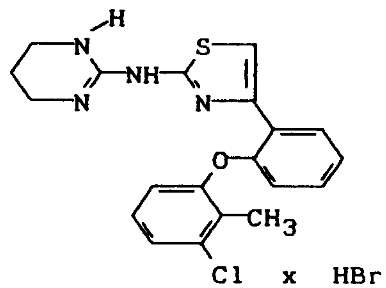<br>x HBr | ≦ 1 | 4 | 8 | 8 |
| (50)<br>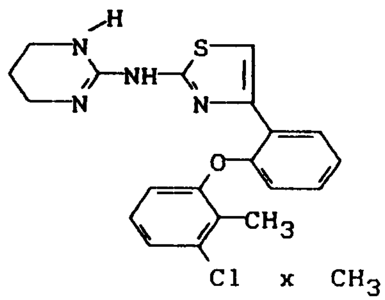<br>x $CH_3$-$SO_3H$ | ≦ 1 | 4 | 8 | 8 |

*) minimale fungizide Konzentration von > 99 %

<u>Tabelle A - Fortsetzung: Antimykotische in-vitro-Wirksamkeit</u>

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (92)<br>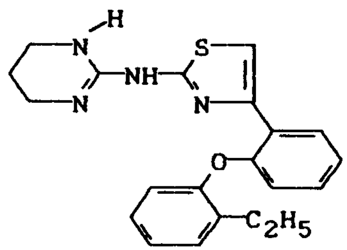 x $CH_3$-$SO_3H$ | 0,5 | 4 | 8 | 8 |
| (98)<br>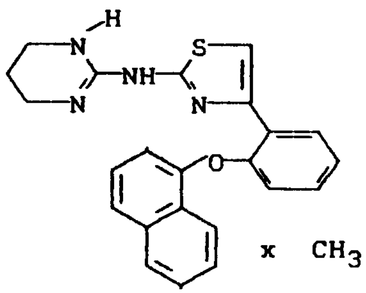 x $CH_3$-$SO_3H$ | $\leqq 1$ | 4 | 8 | 8 |

*) minimale fungizide Konzentration von >99 %

**Tabelle A - Fortsetzung: Antimykotische in-vitro-Wirksamkeit**

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (116) 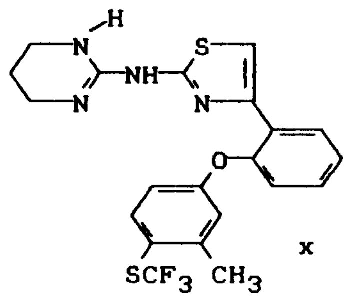 x $CH_3SO_3H$ | 0,5 | 4 | 8 | 8 |
| (132) 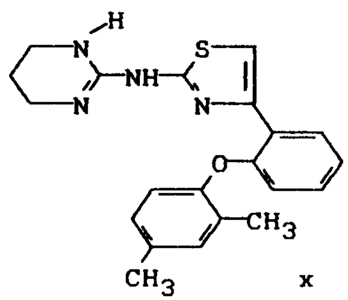 x 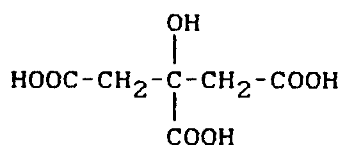 | 0,5 | 4 | 8 | 8 |

*) minimale fungizide Konzentration von >99 %

**Tabelle A - Fortsetzung:** Antimykotische in-vitro-Wirksamkeit

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (133) 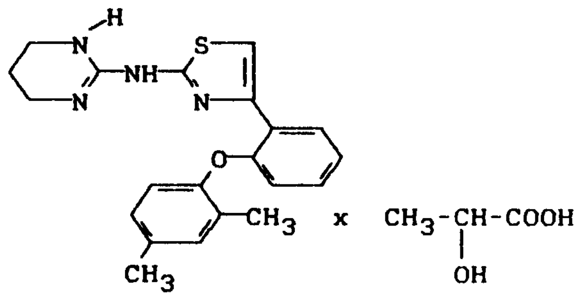 | 0,5 | 4 | 8 | 8 |
| (134) 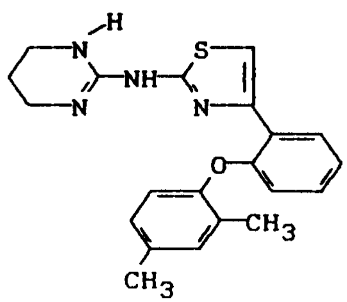 x HOOC-$CH_2$-$CH_2$-COOH | 0,5 | 4 | 8 | 8 |

*) minimale fungizide Konzentration von >99 %

**Tabelle A - Fortsetzung: Antimykotische in-vitro-Wirksamkeit**

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (135) 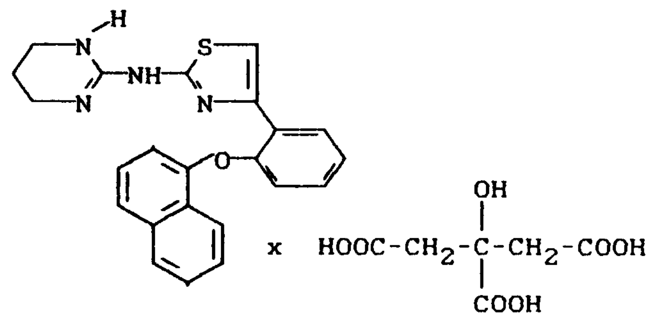 | 0,5 | 4 | 8 | 8 |
| (137) 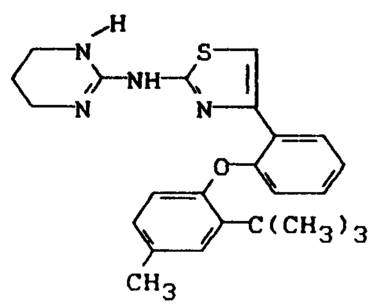 x $CH_3-SO_3H$ | 0,5 | 4 | 8 | 8 |

*) minimale fungizide Konzentration von >99 %

Tabelle A - Fortsetzung: Antimykotische in-vitro-Wirksamkeit

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (147) 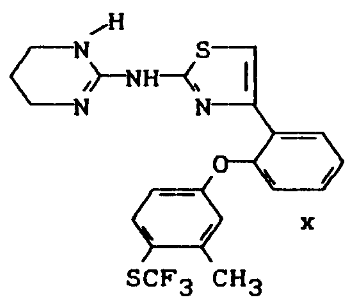 x 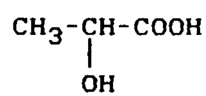 | 0,5 | 4 | 8 | 8 |
| (149) 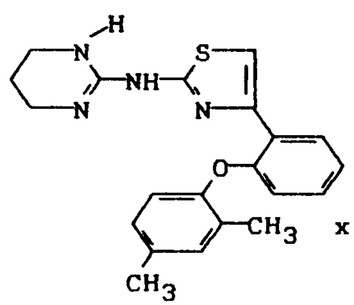 x $HNO_3$ | 0,5 | 4 | 8 | 8 |

*) minimale fungizide Konzentration von > 99 %

Tabelle A - Fortsetzung: Antimykotische in-vitro-Wirksamkeit

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (150) 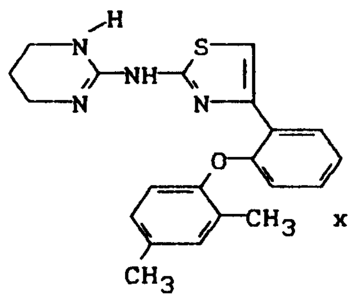 x $H_2SO_4$ | 0,5 | 4 | 8 | 8 |
| (151) 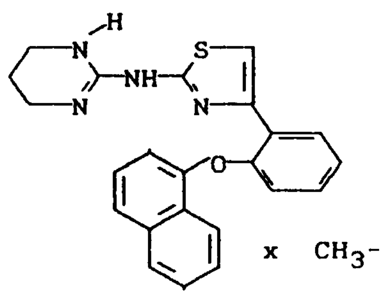 x $CH_3$-CH-COOH (OH) | 0,5 | 4 | 4 | 8 |

*) minimale fungizide Konzentration von >99 %

<u>Tabelle A - Fortsetzung: Antimykotische in-vitro-Wirksamkeit</u>

MFK*)-Werte in µg/ml Nährmedium

| Wirkstoff | Trichophython mentagrophytes | Aspergillus fumigatus | Candida albicans | Torulopsis glabrata |
|---|---|---|---|---|
| (153) 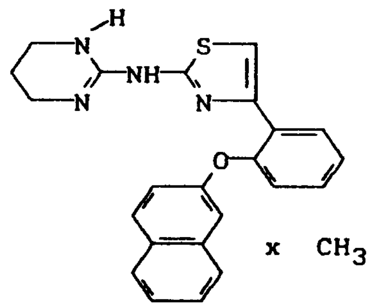 x $CH_3-SO_3H$ | 0,5 | 4 | 4 | 8 |
| (181) 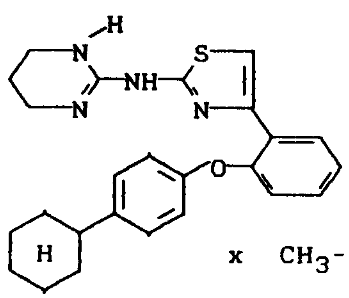 x $CH_3-SO_3H$ | 0,5 | 8 | 4 | 4 |

*) minimale fungizide Konzentration von >99 %

**Patentansprüche**
**Patentansprüche für folgende Vertragsstaaten : AT, BE, CH, DE, FR, GB, IT, LI, LU, NL, SE**

**1.** 2-Aminothiazole der allgemeinen Formel

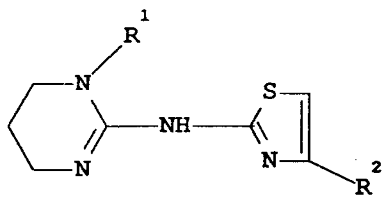

(I)

in welcher

$R^1$ für Wasserstoff oder für geradkettiges oder verzweigtes Alkyl mit 1 bis 4 Kohlenstoffatomen steht und

$R^2$ für einen Rest der Formel

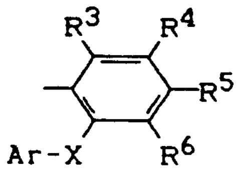
;
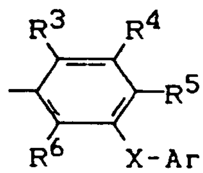
oder
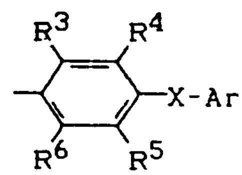

steht,

wobei

$R^3$, $R^4$, $R^5$ und $R^6$ unabhängig voneinander jeweils für Wasserstoff, Fluor, Chlor, Brom, Iod, Nitro, für jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 4 Kohlenstoffatomen in den jeweiligen Alkylteilen oder für jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen -insbesondere Fluor, Chlor, Brom oder Iod-stehen,

X für Sauerstoff, Schwefel, Sulfinyl oder Sulfonyl steht und

Ar für jeweils gegebenenfalls einfach oder mehrfach, gleich oder verschieden substituiertes Phenyl, $\alpha$-Naphthyl, $\beta$-Naphthyl, Tetrahydronaphthyl oder Indanyl steht, wobei als Substituenten jeweils infrage kommen: Fluor, Chlor, Brom, Iod, jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 8 Kohlenstoffatomen in den jeweiligen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, Cycloalkyl mit 3 bis 7 Kohlenstoffatomen, Phenylalkyl oder Phenoxyalkyl mit jeweils 1 bis 4 Kohlenstoffatomen im geradkettigen oder verzweigten Alkylteil sowie Phenyl oder Phenoxy.

**2.** 2-Aminothiazole gemäß Anspruch 1, bei welchen

$R^1$ für Wasserstoff, Methyl oder Ethyl steht,

$R^2$ für einen Rest der Formel

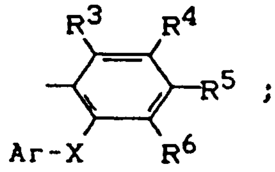 ; 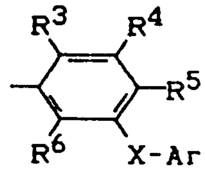 oder 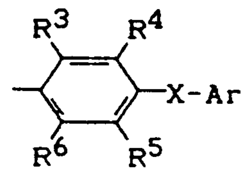

steht,
wobei

| | |
|---|---|
| $R^3$, $R^4$, $R^5$ und $R^6$ | unabhängig voneinander jeweils für Wasserstoff, Fluor, Chlor, Brom, Nitro, Methyl, Ethyl, Methoxy, Ethoxy, Methoxycarbonyl, Ethoxycarbonyl, Dimethylamino, Diethylamino, Methylthio, Ethylthio, Methylsulfinyl, Ethylsulfinyl, Methylsulfonyl oder Ethylsulfonyl oder für Halogenmethyl, Halogenethyl, Halogenmethoxy, Halogenethoxy, Halogenmethylthio, Halogenethylthio, Halogenmethylsulfinyl, Halogenethylsulfinyl, Halogenmethylsulfonyl oder Halogenethylsulfonyl mit jeweils 1 bis 5 gleichen oder verschiedenen Halogenatomen, insbesondere Fluor, Chlor oder Brom stehen, |
| X | für Sauerstoff, Schwefel, Sulfinyl oder Sulfonyl steht und |
| Ar | für jeweils gegebenenfalls ein- bis fünffach gleich oder verschieden substituiertes Phenyl, α-Naphthyl, β-Naphthyl, Tetrahydronaphthyl oder Indanyl steht, wobei als Substituenten jeweils infrage kommen: Fluor, Chlor, Brom, Nitro, jeweils geradkettiges oder verzweigtes Alkyl, Alkoxy, Alkoxycarbonyl oder Dialkylamino mit jeweils 1 bis 6 Kohlenstoffatomen in den jeweiligen Alkylteilen, jeweils geradkettiges oder verzweigtes Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, insbesondere Fluor, Chlor oder Brom, Cyclohexyl mit 3 bis 6 Kohlenstoffatomen, Phenylalkyl oder Phenoxyalkyl mit jeweils 1 bis 3 Kohlenstoffatomen im geradkettigen oder verzweigten Alkylteil sowie Phenyl oder Phenoxy. |

**3.** Verfahren zur Herstellung von 2-Aminothiazolen gemäß Ansprüchen 1 und 2, dadurch gekennzeichnet, daß Thioharnstoff-Derivate der Formel (II)

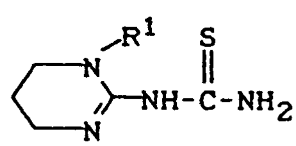 (II)

in welcher
$R^1$ die Bedeutung gemäß Ansprüchen 1 und 2 hat,
mit Acetophenon-Derivaten der Formel (III),

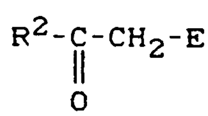 (III)

in welcher
$R^2$ die Bedeutung gemäß Ansprüchen 1 und 2 hat und
E für Hydroxy oder Halogen steht,
gegebenenfalls in Gegenwart eines Verdünnungsmittels und gegebenenfalls in Gegenwart eines Reaktionshilfsmittels umsetzt und gegebenenfalls anschließend eine Säure addiert.

**4.** 2-Aminothiazole gemäß Ansprüchen 1 und 2 zur Bekämpfung von Krankheiten.

**5.** 2-Aminothiazole gemäß Ansprüchen 1 und 2 zur Bekämpfung von Mykosen.

**6.** Arzneimittel enthaltend 2-Aminothiazole gemäß Ansprüchen 1 und 2.

**7.** Verwendung von 2-Aminothiazolen gemäß Ansprüchen 1 und 2 bei der Herstellung von Arzneimitteln.

**8.** Verwendung von 2-Aminothiazolen gemäß Ansprüchen 1 und 2 bei der Herstellung von Arzneimitteln zur Bekämpfung von Mykosen.

**Patentanspruch für folgenden Vertragsstaat : ES**

**1.** Verfahren zur Herstellung von 2-Aminothiazolen der allgemeinen Formel (I) und gegebenenfalls deren physiologisch verträglichen Säureadditionssalze

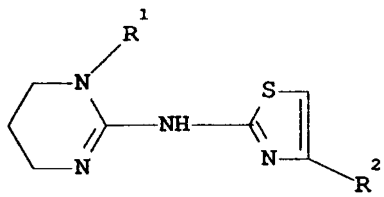

(I)

in welcher

$R^1$ für Wasserstoff oder für geradkettiges oder verzweigtes Alkyl mit 1 bis 4 Kohlenstoffatomen steht und

$R^2$ für einen Rest der Formel

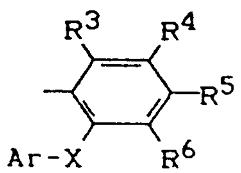 ; 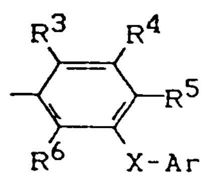 oder 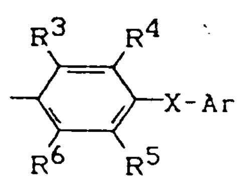

steht,

wobei

$R^3$, $R^4$, $R^5$ und $R^6$ unabhängig voneinander jeweils für Wasserstoff, Fluor, Chlor, Brom, Iod, Nitro, für jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 4 Kohlenstoffatomen in den jeweiligen Alkylteilen oder für jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen -insbesondere Fluor, Chlor, Brom oder Iod-stehen,

X für Sauerstoff, Schwefel, Sulfinyl oder Sulfonyl steht und

Ar für jeweils gegebenenfalls einfach oder mehrfach, gleich oder verschieden substituiertes Phenyl, $\alpha$-Naphthyl, $\beta$-Naphthyl, Tetrahydronaphthyl oder Indanyl steht, wobei als Substituenten jeweils infrage kommen: Fluor, Chlor, Brom, Iod, jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 8 Kohlenstoffatomen in den jeweiligen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, Cycloalkyl mit 3 bis 7 Kohlenstoffatomen, Phenylalkyl oder Phenoxyalkyl mit jeweils 1 bis 4 Kohlenstoffatomen im geradkettigen oder verzweigten Alkylteil sowie Phenyl oder Phenoxy,

dadurch gekennzeichnet, daß

Thioharnstoff-Derivate der Formel (II)

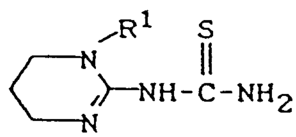

(II)

in welcher

$R^1$ die vorstehende Bedeutung hat ,

mit Acetophenon-Derivaten der Formel (III),

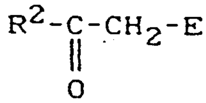

(III)

in welcher

$R^2$ die vorstehende Bedeutung hat

E für Hydroxy oder Halogen steht,

gegebenenfalls in Gegenwart eines Verdünnungsmittels und gegebenenfalls in Gegenwart eines Reaktionshilfsmittels umsetzt und gegebenenfalls anschließend eine Säure addiert.

**Patentansprüche für folgenden Vertragsstaat : GR**

**1.** 2-Aminothiazole der allgemeinen Formel

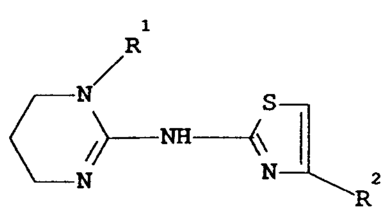

(I)

in welcher

$R^1$ für Wasserstoff oder für geradkettiges oder verzweigtes Alkyl mit 1 bis 4 Kohlenstoffatomen steht und

$R^2$ für einen Rest der Formel

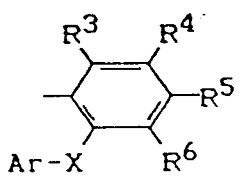

;

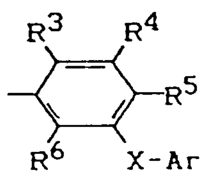

oder

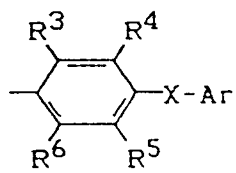

steht,

wobei

$R^3$, $R^4$, $R^5$ und $R^6$ unabhängig voneinander jeweils für Wasserstoff, Fluor, Chlor, Brom, Iod, Nitro, für jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 4 Kohlenstoffatomen in den jeweiligen Alkylteilen oder für jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen -insbesondere Fluor, Chlor, Brom oder Iod-stehen,

X für Sauerstoff, Schwefel, Sulfinyl oder Sulfonyl steht und

Ar für jeweils gegebenenfalls einfach oder mehrfach, gleich oder verschieden substituiertes Phenyl, α-Naphthyl, β-Naphthyl, Tetrahydronaphthyl oder Indanyl steht, wobei als Substituenten jeweils infrage kommen: Fluor, Chlor, Brom, Iod, jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 8 Kohlenstoffatomen in den jeweiligen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, Cycloalkyl mit 3 bis 7 Kohlenstoffatomen, Phenylalkyl oder Phenoxyalkyl mit jeweils 1 bis 4 Kohlenstoffatomen im geradkettigen oder Verzweigten Alkylteil sowie Phenyl oder Phenoxy.

**2.** 2-Aminothiazole gemäß Anspruch 1, bei welchen

$R^1$ für Wasserstoff, Methyl oder Ethyl steht,

$R^2$ für einen Rest der Formel

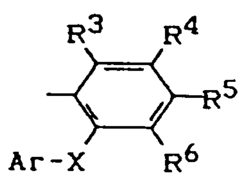 ; 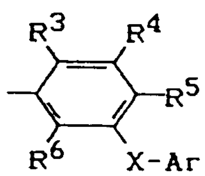 oder 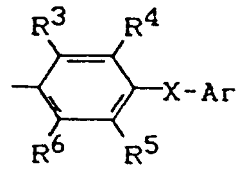

steht,

wobei

$R^3$, $R^4$, $R^5$ und $R^6$ unabhängig voneinander jeweils für Wasserstoff, Fluor, Chlor, Brom, Nitro, Methyl, Ethyl, Methoxy, Ethoxy, Methoxycarbonyl, Ethoxycarbonyl, Dimethylamino, Diethylamino, Methylthio, Ethylthio, Methylsulfinyl, Ethylsulfinyl, Methylsulfonyl oder Ethylsulfonyl oder für Halogenmethyl, Halogenethyl, Halogenmethoxy, Halogenethoxy, Halogenmethylthio, Halogenethylthio, Halogenmethylsulfinyl, Halogenethylsulfinyl, Halogenmethylsulfonyl oder Halogenethylsulfonyl mit jeweils 1 bis 5 gleichen oder verschiedenen Halogenatomen, insbesondere Fluor, Chlor oder Brom stehen,

X für Sauerstoff, Schwefel, Sulfinyl oder Sulfonyl steht und

Ar für jeweils gegebenenfalls ein- bis fünffach gleich oder verschieden substituiertes Phenyl, α-Naphthyl, β-Naphthyl, Tetrahydronaphthyl oder Indanyl steht, wobei als Substituenten jeweils infrage kommen: Fluor, Chlor, Brom, Nitro, jeweils geradkettiges oder verzweigtes Alkyl, Alkoxy, Alkoxycarbonyl oder Dialkylamino mit jeweils 1 bis 6 Kohlenstoffatomen in den jeweiligen Alkylteilen, jeweils geradkettiges oder verzweigtes Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, insbesondere Fluor, Chlor oder Brom, Cyclohexyl mit 3 bis 6 Kohlenstoffatomen, Phenylalkyl oder Phenoxyalkyl mit jeweils 1 bis 3 Kohlenstoffatomen im geradkettigen oder verzweigten Alkylteil sowie Phenyl oder Phenoxy.

**3.** Verfahren zur Herstellung von 2-Aminothiazolen gemäß Ansprüchen 1 und 2, dadurch gekennzeichnet, daß Thioharnstoff-Derivate der Formel (II)

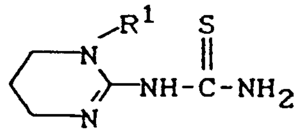

(II)

in welcher

$R^1$ die Bedeutung gemäß Ansprüchen 1 und 2 hat,

mit Acetophenon-Derivaten der Formel (III),

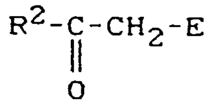

(III)

in welcher

$R^2$ die Bedeutung gemäß Ansprüchen 1 und 2 hat und

E für Hydroxy oder Halogen steht,

gegebenenfalls in Gegenwart eines Verdünnungsmittels und gegebenenfalls in Gegenwart eines Reaktionshilfsmittels umsetzt und gegebenenfalls anschließend eine Säure addiert.

**Claims**

**Claims for the following Contracting States : AT, BE, CH, DE, FR, GB, IT, LI, LU, NL, SE**

**1.** 2-Aminothiazoles of the general formula

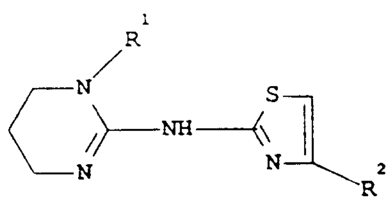

(I)

in which

$R^1$ represents hydrogen or straight-chain or branched alkyl having 1 to 4 carbon atoms and

$R^2$ represents a radical of the formula

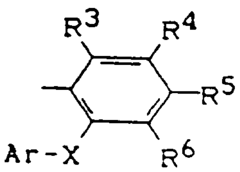 ; 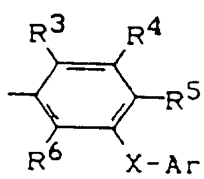 or 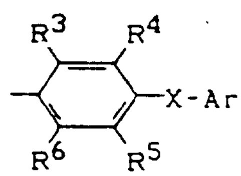

where

$R^3$, $R^4$, $R^5$ and $R^6$ independently of one another in each case represent hydrogen, fluorine, chlorine, bromine, iodine, nitro, in each case straight-chain or branched alkyl, alkoxy, alkoxycarbonyl, dialkylamino, alkylthio, alkylsulphinyl or alkylsulphonyl each having 1 - 4 carbon atoms in the respective alkyl moieties or in each case straight-chain or branched halogenoalkyl, halogenoalkoxy, halogenoalkylthio, halogenoalkylsulphinyl or halogenoalkylsulphonyl each having 1 to 4 carbon atoms and 1 to 9 identical or different halogen atoms, in particular fluorine, chlorine, bromine or iodine,

X represents oxygen, sulphur, sulphinyl or sulphonyl and

Ar represents phenyl, α-naphthyl, β-naphthyl, tetrahydronaphthyl or indanyl which are in each case optionally monosubstituted or polysubstituted by identical or

different substituents, suitable substituents in each case being: fluorine, chlorine, bromine, iodine, in each case straight-chain or branched alkyl, alkoxy, alkoxycarbonyl, dialkylamino, alkylthio, alkylsulphinyl or alkylsulphonyl each having 1 to 8 carbon atoms in the respective alkyl moieties, in each case straight-chain or branched halogenoalkyl, halogenoalkoxy, halogenoalkylthio, halogenoalkylsulphinyl or halogenoalkylsulphonyl each having 1 to 4 carbon atoms and 1 to 9 identical or different halogen atoms, cycloalkyl having 3 to 7 carbon atoms, phenylalkyl or phenoxyalkyl each having 1 to 4 carbon atoms in the straight-chain or branched alkyl moiety and also phenyl or phenoxy.

**2.** 2-Aminothiazoles according to Claim 1, in which
$R^1$ represents hydrogen, methyl or ethyl,
$R^2$ represents a radical of the formula

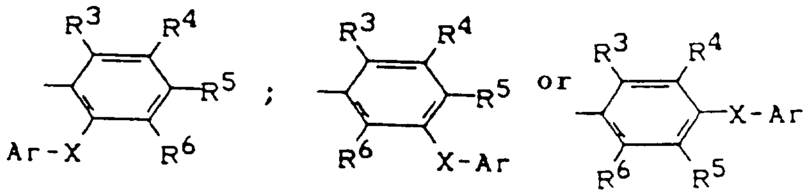

where
$R^3$, $R^4$, $R^5$ and $R^6$ independently of one another in each case represent hydrogen, fluorine, chlorine, bromine, nitro, methyl, ethyl, methoxy, ethoxy, methoxycarbonyl, ethoxycarbonyl, dimethylamino, diethylamino, methylthio, ethylthio, methylsulphinyl, ethylsulphinyl, methylsulphonyl or ethylsulphonyl, or halogenomethyl, halogenoethyl, halogenomethoxy, halogenoethoxy, halogenomethylthio, halogenoethylthio, halogenomethylsulphinyl, halogenoethylsulphinyl, halogenomethylsulphonyl or halogenoethylsulphonyl each having 1 to 5 identical or different halogen atoms, in particular fluorine, chlorine or bromine,
X represents oxygen, sulphur, sulphinyl or sulphonyl and
Ar represents phenyl, α-naphthyl, β-naphthyl, tetrahydronaphthyl or indanyl which are in each case optionally monosubstituted to pentasubstituted by identical or different substituents, suitable substituents in each case being: fluorine, chlorine, bromine, nitro, in each case straight-chain or branched alkyl, alkoxy, alkoxycarbonyl or dialkylamino each having 1 to 6 carbon atoms in the respective alkyl moieties, in each case straight-chain or branched alkylthio, alkylsulphinyl or alkylsulphonyl each having 1 to 3 carbon atoms, in each case straight-chain or branched halogenoalkyl, halogenoalkoxy, halogenoalkylthio, halogenoalkylsulphinyl or halogenoalkylsulphonyl each having 1 to 3 carbon atoms and 1 to 7 identical or different halogen atoms, in particular fluorine, chlorine or bromine, cyclohexyl having 3 to 6 carbon atoms, phenylalkyl or phenoxyalkyl each having 1 to 3 carbon atoms in the straight-chain or branched alkyl moiety and also phenyl or phenoxy.

**3.** Process for the preparation of 2-aminothiazoles according to Claims 1 and 2, characterized in that thiourea derivatives of the formula (II)

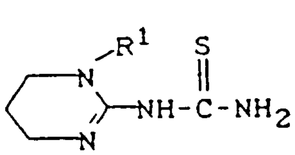

(II)

in which
$R^1$ has the meaning according to Claims 1 and 2,
are reacted with acetophenone derivatives of the formula (III)

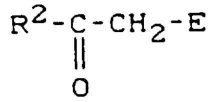 (III)

in which

$R^2$ has the meaning according to Claims 1 and 2 and

E represents hydroxyl or halogen,

if appropriate in the presence of a diluent and if appropriate in the presence of a reaction auxiliary and, if appropriate, an acid is then adducted.

**4.** 2-Aminothiazoles according to Claims 1 and 2 for combating diseases.

**5.** 2-Aminothiazoles according to Claims 1 and 2 for combating mycoses.

**6.** Medicaments containing 2-aminothiazoles according to Claims 1 and 2.

**7.** Use of 2-aminothiazoles according to Claims 1 and 2 in the production of medicaments.

**8.** Use of 2-aminothiazoles according to Claims 1 and 2 in the production of medicaments for combating mycoses.

**Claim for the following Contracting State : ES**

**1.** Process for the preparation of 2-aminothiazoles of the general formula (I) and optionally the physiologically compatible acid addition salts thereof

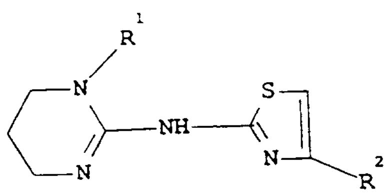 (I)

in which

$R^1$ represents hydrogen or straight-chain or branched alkyl having 1 to 4 carbon atoms and

$R^2$ represents a radical of the formula

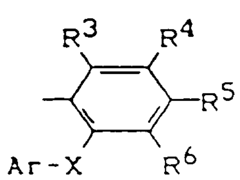 ; 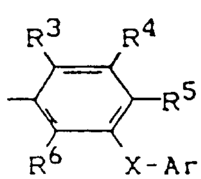 or 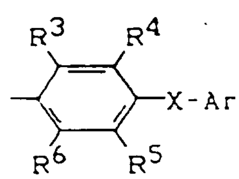

where

$R^3$, $R^4$, $R^5$ and $R^6$ independently of one another in each case represent hydrogen, fluorine, chlorine, bromine, iodine, nitro, in each case straight-chain or branched alkyl, alkoxy, alkoxycarbonyl, dialkylamino, alkylthio, alkylsulphinyl or alkylsulphonyl each having 1 to 4 carbon atoms in the respective alkyl moieties, or in each case straight-chain or branched halogenoalkyl, halogenoalkoxy, halogenoalkylthio, halogenoalkylsulphinyl or halogenoalkylsulphonyl each having 1 to 4 carbon atoms and 1 to 9 identical or different halogen atoms, in particular fluorine, chlorine, bromine or iodine,

X represents oxygen, sulphur, sulphinyl or sulphonyl and

Ar represents phenyl, $\alpha$-naphthyl, $\beta$-naphthyl, tetrahydronaphthyl or indanyl which

are in each case optionally monosubstituted or polysubstituted by identical or different substituents, suitable substituents in each case being: fluorine, chlorine, bromine, iodine, in each case straight-chain or branched alkyl, alkoxy, alkoxycarbonyl, dialkylamino, alkylthio, alkylsulphinyl or alkylsulphonyl each having 1 to 8 carbon atoms in the respective alkyl moieties, in each case straight-chain or branched halogenoalkyl, halogenoalkoxy, halogenoalkylthio, halogenoalkylsulphinyl or halogenoalkylsulphonyl each having 1 to 4 carbon atoms and 1 to 9 identical or different halogen atoms, cycloalkyl having 3 to 7 carbon atoms, phenylalkyl or phenoxyalkyl each having 1 to 4 carbon atoms in the straight-chain or branched alkyl moiety and also phenyl or phenoxy,

characterized in that thiourea derivatives of the formula (II)

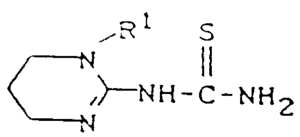

(II)

in which

$R^1$ has the above meaning,

are reacted with acetophenone derivatives of the formula (III)

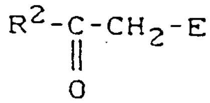

(III)

in which

$R^2$ has the above meaning and

E represents hydroxyl or halogen,

if appropriate in the presence of a diluent and if appropriate in the presence of a reaction auxiliary and, if appropriate, an acid is then adducted.

**Claims for the following Contracting State : GR**

**1.** 2-Aminothiazoles of the general formula

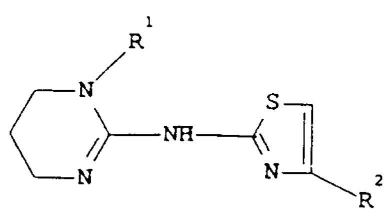

(I)

in which

$R^1$ represents hydrogen or straight-chain or branched alkyl having 1 to 4 carbon atoms and

$R^2$ represents a radical of the formula

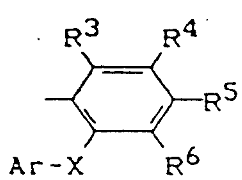 ; 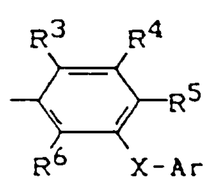 or 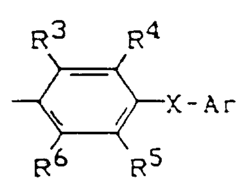

where

| | |
|---|---|
| $R^3$, $R^4$, $R^5$ and $R^6$ | independently of one another in each case represent hydrogen, fluorine, chlorine, bromine, iodine, nitro, in each case straight-chain or branched alkyl, alkoxy, alkoxycarbonyl, dialkylamino, alkylthio, alkylsulphinyl or alkylsulphonyl each having 1 to 4 carbon atoms in the respective alkyl moieties, or in each case straight-chain or branched halogenoalkyl, halogenoalkoxy, halogenoalkylthio, halogenoalkylsulphinyl or halogenoalkylsulphonyl each having 1 to 4 carbon atoms and 1 to 9 identical or different halogen atoms, in particular fluorine, chlorine, bromine or iodine, |
| X | represents oxygen, sulphur, sulphinyl or sulphonyl and |
| Ar | represents phenyl, α-naphthyl, β-naphthyl, tetrahydronaphthyl or indanyl which are in each case optionally monosubstituted or polysubstituted by identical or different substituents, suitable substituents in each case being: fluorine, chlorine, bromine, iodine, in each case straight-chain or branched alkyl, alkoxy, alkoxycarbonyl, dialkylamino, alkylthio, alkylsulphinyl or alkylsulphonyl each having 1 to 8 carbon atoms in the respective alkyl moieties, in each case straight-chain or branched halogenoalkyl, halogenoalkoxy, halogenoalkylthio, halogenoalkylsulphinyl or halogenoalkylsulphonyl each having 1 to 4 carbon atoms and 1 to 9 identical or different halogen atoms, cycloalkyl having 3 to 7 carbon atoms, phenylalkyl or phenoxyalkyl each having 1 to 4 carbon atoms in the straight-chain or branched alkyl moiety and also phenyl or phenoxy. |

**2.** 2-Aminothiazoles according to Claim 1, in which

$R^1$ represents hydrogen, methyl or ethyl,

$R^2$ represents a radical of the formula

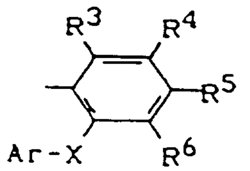 ; 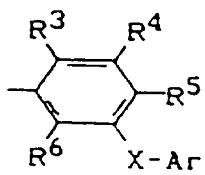 or 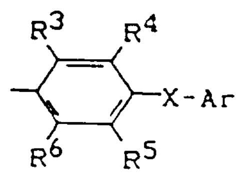

where

| | |
|---|---|
| $R^3$, $R^4$, $R^5$ and $R^6$ | independently of one another in each case represent hydrogen, fluorine, chlorine, bromine, nitro, methyl, ethyl, methoxy, ethoxy, methoxycarbonyl, ethoxycarbonyl, dimethylamino, diethylamino, methylthio, ethylthio, methylsulphinyl, ethylsulphinyl, methylsulphonyl or ethylsulphonyl, or halogenomethyl, halogenoethyl, halogenomethoxy, halogenoethoxy, halogenomethylthio, halogenoethylthio, halogenomethylsulphinyl, halogenoethylsulphinyl, halogenomethylsulphonyl or halogenoethylsulphonyl each having 1 to 5 identical or different halogen atoms, in particular fluorine, chlorine or bromine, |
| X | represents oxygen, sulphur, sulphinyl or sulphonyl and |
| Ar | represents phenyl, α-naphthyl, β-naphthyl, tetrahydronaphthyl or indanyl which are in each case optionally monosubstituted to pentasubstituted by identical or different substituents, suitable substituents in each case being: fluorine, chlorine, bromine, nitro, in each case straight-chain or branched alkyl, alkoxy, alkoxycarbonyl or dialkylamino each having 1 to 6 carbon atoms in the respective alkyl moieties, in each case straight-chain or branched alkylthio, alkylsulphinyl or alkylsulphonyl each having 1 to 3 carbon atoms, in each case straight-chain or branched halogenoalkyl, halogenoalkoxy, halogenoalkylthio, halogenoalkylsulphinyl or halogenoalkylsulphonyl each having 1 to 3 carbon atoms and 1 to 7 identical or different halogen atoms, in particular fluorine, chlorine or bromine, cyclohexyl having 3 to 6 carbon atoms, phenylalkyl or phenoxyalkyl each having 1 to 3 carbon atoms in the straight-chain or branched alkyl moiety and also phenyl or phenoxy. |

**3.** Process for the preparation of 2-aminothiazoles according to Claims 1 and 2, characterized in that thiourea derivatives of the formula (II)

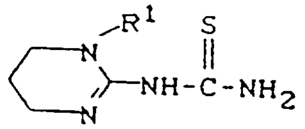

(II)

in which
$R^1$ has the meaning according to Claims 1 and 2,
are reacted with acetophenone derivatives of the formula (III)

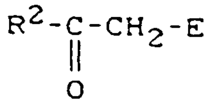

(III)

in which
$R^2$ has the meaning according to Claims 1 and 2 and
E represents hydroxyl or halogen,
if appropriate in the presence of a diluent and if appropriate in the presence of a reaction auxiliary and, if appropriate, an acid is then adducted.

**Revendications**
**Revendications pour les Etats contractants suivants : AT, BE, CH, DE, FR, GB, IT, LI, LU, NL, SE**

**1.** 2-Aminothiazoles de formule générale :

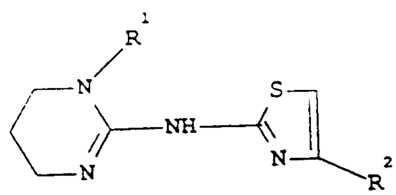

(I)

dans laquelle
$R^1$ représente un atome d'hydrogène ou un groupe alkyle linéaire ou ramifié ayant 1 à 4 atomes de carbone, et
$R^2$ représente un reste de formule

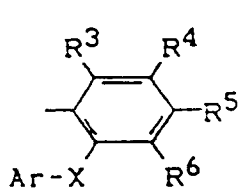

;

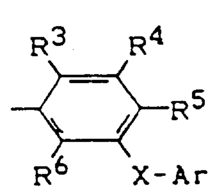

ou

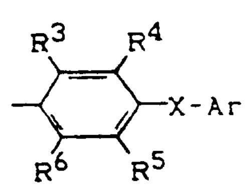

dans lesquelles
$R^3$, $R^4$, $R^5$ et $R^6$ représentent chacun, indépendamment l'un de l'autre, un atome d'hydrogène, de fluor, de chlore, de brome ou d'iode, un groupe nitro, un groupe alkyle, alcoxy, alcoxycarbonyle, dialkylamino, alkylthio, alkylsulfinyle ou alkylsulfonyle, chaque fois linéaire ou ramifié ayant chacun 1 à 4 atomes de carbone dans la partie alkyle respective, ou un groupe halogéno-alkyle, halogéno-alcoxy, halogéno-alkylthio, halogéno-alkylsulfinyle ou halogéno-alkylsulfonyle, linéaire ou ramifié ayant chacun 1 à 4 atomes de carbone et 1 à 9 atomes d'halogène, identiques ou différents, en particulier des atomes de fluor, de chlore, de brome ou d'iode,

X représente un atome d'oxygène, de soufre, un groupe sulfinyle ou sulfonyle, et

Ar représente un groupe phényle, α-naphtyle, β-naphtyle, tétrahydronaphtyle ou indanyle, chacun éventuellement substitué une ou plusieurs fois de façon identique ou différente, et l'on peut citer comme substituant : un atome de fluor, de chlore, de brome, d'iode, un reste alkyle, alcoxy, alcoxycarbonyle, dialkylamino, alkylthio, alkylsulfinyle ou alkylsulfonyle chaque fois linéaire ou ramifié et comportant chacun 1 à 8 atomes de carbone dans les parties alkyles respectives, un groupe halogéno-alkyle, halogéno-alcoxy, halogéno-alkylthio, halogéno-alkylsulfinyle ou halogéno-alkylsulfonyle, chacun linéaire ou ramifié ayant chacun 1 à 4 atomes de carbone et 1 à 9 atomes d'halogène identiques ou différents, un groupe cycloalkyle ayant 3 à 7 atomes de carbone, un groupe phénylalkyle ou phénoxyalkyle ayant chacun 1 à 4 atomes de carbone dans la partie alkyle linéaire ou ramifiée, ainsi qu'un groupe phényle ou phénoxy.

**2.** Aminothiazoles selon la revendication 1, dans lesquels :

$R^1$ représente un atome d'hydrogène, un groupe méthyle ou éthyle,

$R^2$ représente un reste de formule

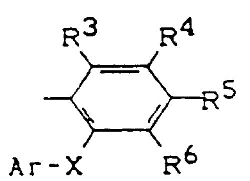
;
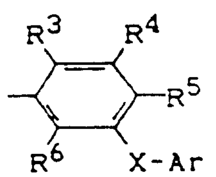
ou
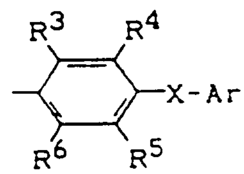

formules dans lesquelles

$R^3$, $R^4$, $R^5$ et $R^6$ représentent chacun, indépendamment l'un de l'autre, un atome d'hydrogène, de fluor, de chlore ou de brome, un groupe nitro, méthyle, éthyle, méthoxy, éthoxy, méthoxycarbonyle, éthoxycarbonyle, diméthylamino, diéthylamino, méthylthio, éthylthio, méthylsulfinyle, éthylsulfinyle, méthylsulfonyle ou éthylsulfonyle ou un groupe halogéno-méthyle, halogéno-éthyle, halogéno-méthoxy, halogéno-éthoxy, halogéno-méthylthio, halogéno-éthylthio, halogéno-méthylsulfinyle, halogéno-éthylsulfinyle, halogéno-méthylsulfonyle ou halogéno-éthylsulfonyle ayant chacun 1 à 5 atomes d'halogène identiques ou différents, notamment le fluor, le chlore ou le brome,

X représente un atome d'oxygène ou de soufre ou un groupe sulfinyle ou sulfonyle, et

Ar représente un groupe phényle, α-naphtyle, β-naphtyle, tétrahydronaphtyle ou indanyle, chacun éventuellement substitués 1 à 5 fois de façon identique ou différente, et l'on peut citer comme substituant : un atome de fluor, de chlore, de brome, un reste nitro, un groupe alkyle, alcoxy, alcoxycarbonyle ou dialkylamino chacun linéaire ou ramifié et comportant chacun 1 à 6 atomes de carbone dans les parties alkyles correspondantes, un groupe alkylthio, alkylsulfinyle ou alkylsulfonyle chacun linéaire ou ramifié ayant chacun 1 à 3 atomes de carbone, un groupe halogéno-alkyle, halogéno-alcoxy, halogéno-alkylthio, halogéno-alkylsulfinyle ou halogéno-alkylsulfonyle, chacun linéaire ou ramifié et ayant chacun 1 à 3 atomes de carbone et 1 à 7 atomes d'halogène identiques ou différents, notamment le fluor, le chlore ou le brome, un groupe cyclohexyle ayant 3 à 6 atomes de carbone, phénylalkyle ou phénoxyalkyle ayant chacun 1 à 3 atomes de carbone dans la partie alkyle linéaire ou ramifiée, ainsi qu'un groupe phényle ou phénoxy.

**3.** Procédé pour préparer des 2-aminothiazoles selon les revendications 1 ou 2, caractérisé en ce qu'on fait réagir des dérivés de la thiourée, de formule (II)

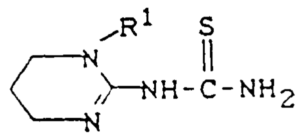 (II)

dans laquelle

R[1] a le sens indiqué aux revendications 1 et 2,

avec des dérivés de l'acétophénone de formule (III)

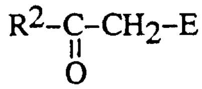 (III)

dans laquelle

R[2] a le sens indiqué dans les revendications 1 et 2, et

E représente un groupe hydroxyle ou un atome d'halogène,

en opérant éventuellement en présence d'un diluant et éventuellement en présence d'un adjuvant de réaction, et l'on ajoute éventuellement ensuite un acide.

**4.** 2-Aminothiazoles selon les revendications 1 et 2, pour combattre des maladies.

**5.** 2-Aminothiazoles selon les revendications 1 et 2, pour combattre des mycoses.

**6.** Médicaments contenant des 2-aminothiazoles selon les revendications 1 et 2.

**7.** Utilisation de 2-aminothiazoles selon les revendications 1 et 2, pour la préparation de médicaments.

**8.** Utilisation de 2-aminothiazoles selon les revendications 1 et 2, pour la préparation de médicaments pour combattre des mycoses.

**Revendication pour l'Etat contractant suivant : ES**

**1.** Procédé pour préparer des 2-aminothiazoles de formule générale (I) et éventuellement leurs sels d'addition d'acide physiologiquement tolérables :

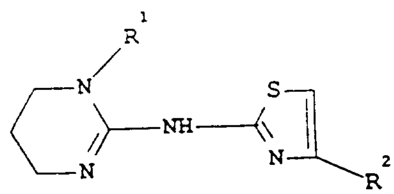 (I)

formule dans laquelle

R[1] représente un atome d'hydrogène ou un groupe alkyle linéaire ou ramifié ayant 1 à 4 atomes de carbone, et

R[2] représente un reste de formule

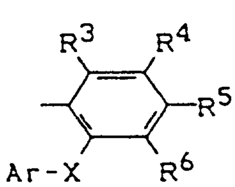 ; 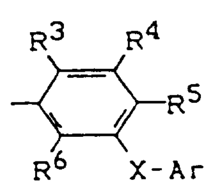 ou 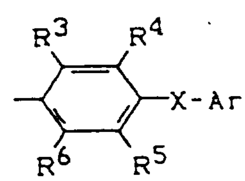

dans lesquelles

$R^3$, $R^4$, $R^5$ et $R^6$ représentent chacun, indépendamment l'un de l'autre, un atome d'hydrogène, de fluor, de chlore, de brome ou d'iode, un groupe nitro, un groupe alkyle, alcoxy, alcoxycarbonyle, dialkylamino, alkylthio, alkylsulfinyle ou alkylsulfonyle, chaque fois linéaire ou ramifié ayant chacun 1 à 4 atomes de carbone dans la partie alkyle respective, ou un groupe halogéno-alkyle, halogéno-alcoxy, halogéno-alkylthio, halogéno-alkylsulfinyle ou halogéno-alkylsulfonyle, linéaire ou ramifié ayant chacun 1 à 4 atomes de carbone et 1 à 9 atomes d'halogène, identiques ou différents, en particulier des atomes de fluor, de chlore, de brome ou d'iode,

X représente un atome d'oxygène, de soufre, un groupe sulfinyle ou sulfonyle, et

Ar représente un groupe phényle, α-naphtyle, β-naphtyle, tétrahydronaphtyle ou indanyle, chacun éventuellement substitué une ou plusieurs fois de façon identique ou différente, et l'on peut citer comme substituant : un atome de fluor, de chlore, de brome, d'iode, un reste alkyle, alcoxy, alcoxycarbonyle, dialkylamino, alkylthio, alkylsulfinyle ou alkylsulfonyle chaque fois linéaire ou ramifié et comportant chacun 1 à 8 atomes de carbone dans les parties alkyles respectives, un groupe halogéno-alkyle, halogéno-alcoxy, halogéno-alkylthio, halogéno-alkylsulfinyle ou halogéno-alkylsulfonyle, chacun linéaire ou ramifié ayant chacun 1 à 4 atomes de carbone et 1 à 9 atomes d'halogène identiques ou différents, un groupe cycloalkyle ayant 3 à 7 atomes de carbone, un groupe phénylalkyle ou phénoxyalkyle ayant chacun 1 à 4 atomes de carbone dans la partie alkyle linéaire ou ramifiée, ainsi qu'un groupe phényle ou phénoxy,

procédé caractérisé en ce que l'on fait réagir des dérivés de la thiourée, de formule (II)

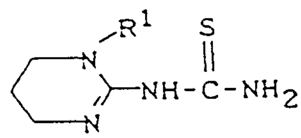

(II)

dans laquelle

$R^1$ a le sens précité,

avec des dérivés de l'acétophénone de formule (III)

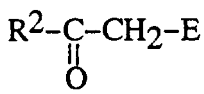

(III)

dans laquelle

$R^2$ a le sens précité, et

E représente un groupe hydroxyle ou un atome d'halogène,

en opérant éventuellement en présence d'un diluant et éventuellement en présence d'un adjuvant de réaction, et l'on fixe éventuellement ensuite, par addition, un acide.

**Revendications pour l'Etat contractant suivant : GR**

**1.** 2-Aminothiazoles de formule générale :

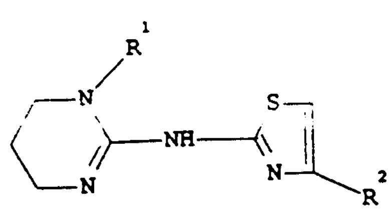

(I)

dans laquelle

$R^1$ représente un atome d'hydrogène ou un groupe alkyle linéaire ou ramifié ayant 1 à 4 atomes de carbone, et

$R^2$ représente un reste de formule

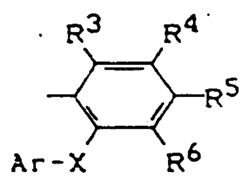 ; 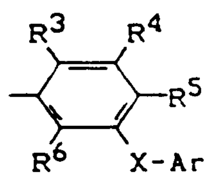 ou 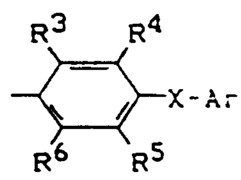

dans lesquelles

$R^3$, $R^4$, $R^5$ et $R^6$ représentent chacun, indépendamment l'un de l'autre, un atome d'hydrogène, de fluor, de chlore, de brome ou d'iode, un groupe nitro, un groupe alkyle, alcoxy, alcoxycarbonyle, dialkylamino, alkylthio, alkylsulfinyle ou alkylsulfonyle, chaque fois linéaire ou ramifié ayant chacun 1 à 4 atomes de carbone dans la partie alkyle respective, ou un groupe halogéno-alkyle, halogéno-alcoxy, halogéno-alkylthio, halogéno-alkylsulfinyle ou halogéno-alkylsulfonyle, linéaire ou ramifié ayant chacun 1 à 4 atomes de carbone et 1 à 9 atomes d'halogène, identiques ou différents, en particulier des atomes de fluor, de chlore, de brome ou d'iode,

X représente un atome d'oxygène, de soufre, un groupe sulfinyle ou sulfonyle, et

Ar représente un groupe phényle, α-naphtyle, β-naphtyle, tétrahydronaphtyle ou indanyle, chacun éventuellement substitué une ou plusieurs fois de façon identique ou différente, et l'on peut citer comme substituant : un atome de fluor, de chlore, de brome, d'iode, un reste alkyle, alcoxy, alcoxycarbonyle, dialkylamino, alkylthio, alkylsulfinyle ou alkylsulfonyle chaque fois linéaire ou ramifié et comportant chacun 1 à 8 atomes de carbone dans les parties alkyles respectives, un groupe halogéno-alkyle, halogéno-alcoxy, halogéno-alkylthio, halogéno-alkylsulfinyle ou halogéno-alkylsulfonyle, chacun linéaire ou ramifié ayant chacun 1 à 4 atomes de carbone et 1 à 9 atomes d'halogène identiques ou différents, un groupe cycloalkyle ayant 3 à 7 atomes de carbone, un groupe phénylalkyle ou phénoxyalkyle ayant chacun 1 à 4 atomes de carbone dans la partie alkyle linéaire ou ramifiée, ainsi qu'un groupe phényle ou phénoxy.

**2.** 2-Aminothiazoles selon la revendication 1, dans lesquels :

$R^1$ représente un atome d'hydrogène, un groupe méthyle ou éthyle,

$R^2$ représente un reste de formule

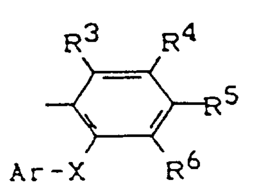 ; 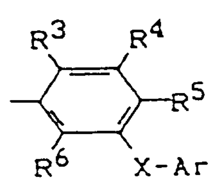 ou 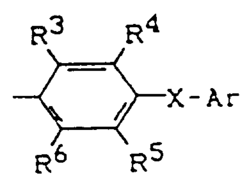

formules dans lesquelles

$R^3$, $R^4$, $R^5$ et $R^6$ représentent chacun, indépendamment l'un de l'autre, un atome d'hydrogène, de fluor, de chlore ou de brome, un groupe nitro, méthyle, éthyle, méthoxy, éthoxy, méthoxycarbonyle, éthoxycarbonyle, diméthylamino, diéthylamino, méthylthio, éthylthio, méthylsulfinyle, éthylsulfinyle, méthylsulfonyle ou éthylsulfonyle ou un groupe halogéno-méthyle, halogéno-éthyle, halogéno-méthoxy, halogéno-éthoxy, halogéno-méthylthio, halogéno-éthylthio, halogéno-méthylsulfinyle, halogéno-éthylsulfinyle, halogéno-méthylsulfonyle ou halogéno-éthylsulfonyle ayant chacun 1 à 5 atomes d'halogène identiques ou différents, notamment le

fluor, le chlore ou le brome,

X représente un atome d'oxygène ou de soufre ou un groupe sulfinyle ou sulfonyle, et

Ar représente un groupe phényle, α-naphtyle, β-naphtyle, tétrahydronaphtyle ou indanyle, chacun éventuellement substitués 1 à 5 fois de façon identique ou différente, et l'on peut citer comme substituant : un atome de fluor, de chlore, de brome, un reste nitro, un groupe alkyle, alcoxy, alcoxycarbonyle ou dialkylamino chacun linéaire ou ramifié et comportant chacun 1 à 6 atomes de carbone dans les parties alkyles correspondantes, un groupe alkylthio, alkylsulfinyle ou alkylsulfonyle chacun linéaire ou ramifié ayant chacun 1 à 3 atomes de carbone, un groupe halogéno-alkyle, halogéno-alcoxy, halogéno-alkylthio, halogéno-alkylsulfinyle ou halogéno-alkylsulfonyle, chacun linéaire ou ramifié et ayant chacun 1 à 3 atomes de carbone et 1 à 7 atomes d'halogène identiques ou différents, notamment le fluor, le chlore ou le brome, un groupe cyclohexyle ayant 3 à 6 atomes de carbone, phénylalkyle ou phénoxyalkyle ayant chacun 1 à 3 atomes de carbone dans la partie alkyle linéaire ou ramifiée, ainsi qu'un groupe phényle ou phénoxy.

**3.** Procédé pour préparer des 2-aminothiazoles selon les revendications 1 et 2, caractérisé en ce qu'on fait réagir des dérivés de la thiourée de formule (II) :

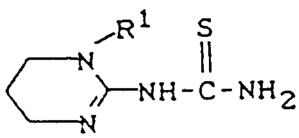

(II)

dans laquelle

$R^1$ a le sens indiqué aux revendications 1 et 2,

avec des dérivés de l'acétophénone de formule (III)

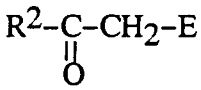

(III)

$R^2$ a le sens indiqué aux revendications 1 et 2, et

E représente un groupe hydroxyle ou un atome d'halogène,

en opérant éventuellement en présence d'un diluant et éventuellement en présence d'un adjuvant de réaction, et l'on fixe éventuellement ensuite, par addition, un acide.